# EUROPEAN PATENT APPLICATION

(11) **EP 4 183 449 A1**
(43) Date of publication of application: **24.05.2023**
(21) Application number: 21208821.5
(22) Date of filing: 17.11.2021
(51) Int. Cl.: A61P 9/00, A61P 25/08, A61P 25/28, A61P 29/00, A61P 31/00, A61P 35/00, A61P 43/00, C07D 401/14, C07D 403/04, C07D 403/14, C07D 405/14, C07D 409/04, C07D 409/14, C07D 417/04, C07D 417/14, A61K 31/426, A61K 31/454, A61K 31/4155

(54) **AUTOPHAGY INDUCING COMPOUNDS AND USES THEREOF**

(71) Applicant: Samsara Therapeutics Inc., Lewes, DE 19958 (US); Institut National de la Santé et de la Recherche Médicale (INSERM), 75013 Paris (FR); Université de Paris, 75006 Paris (FR); Sorbonne Université, 75006 Paris (FR)
(72) Inventor: Hamley, Peter, Oxford, OX3 8SB (GB); Galloway, Warren, 61476 Kronberg im Taunus (DE); Kepp, Oliver, 75270 Paris (FR); Kroemer, Guido, 75270 Paris (FR)
(74) Representative: Krauss, Jan

(57) **Abstract**

The invention relates to pharmaceutical compositions and methods of treating autophagy related diseases and disorders. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection.

## Description

The invention relates to pharmaceutical compositions and methods of treating autophagy related diseases and disorders. The present invention relates to compounds according to Formula (I) or salts, solvates and/or hydrates thereof, wherein said compounds induce and/or stimulate the process of autophagy, as well as uses of the compounds in the treatment and prevention of autophagy related diseases and disorders. Examples are cancer, age-related diseases, and viral infection.

### Background of the invention

Autophagy is the process of removing unnecessary organelles and proteins from cells that are lost or lost function, it helps maintain cell homeostasis and is a cell survival mechanism.

The cell-autonomous antimicrobial defense functions of autophagy, demonstrated initially in the case of streptococci and Mycobacterium tuberculosis have been extended to a wide variety of microbes with a caveat that most highly adapted pathogens have evolved specific protective mechanisms against autophagic elimination of microbes. Other studies have uncovered orderly intersections between autophagy and innate and adaptive immunity, T cell development, differentiation and homeostasis, and inflammatory responses. Thus, autophagy plays a large role in various diseases such as cancer, inflammatory disease, degenerative neurological disease, and immune disease. Autophagy is a cell survival mechanism that is induced in stressed cells

Towers and Thorburn (in: Therapeutic Targeting of Autophagy. EBioMedicine. 2016;14:15-23. doi:10.1016/j.ebiom.2016.10.034) disclose that autophagy is widely accepted as cytoprotective against neurodegenerative diseases and a variety of clinical interventions are moving forward to increase autophagy as a therapeutic intervention. Autophagy has both positive and negative roles in cancer and this has led to controversy over whether or how autophagy manipulation should be attempted in cancer therapy. Nevertheless, cancer is the disease where most current activity in trying to manipulate autophagy for therapy is taking place and dozens of clinical trials are using autophagy inhibition with Chloroquine or Hydroxychloroquine in combination with other drugs for the treatment of multiple neoplasms. They review recent literature implicating autophagy in neurodegenerative diseases and cancer and highlight some of the opportunities, controversies and potential pitfalls of therapeutically targeting autophagy.

Mulcahy Levy and Thorburn (in: Autophagy in cancer: moving from understanding mechanism to improving therapy responses in patients. Cell Death Differ 27, 843-857 (2020). https://doi.org/10.1038/s41418-019-0474-7) disclose that autophagy allows for cellular material to be delivered to lysosomes for degradation resulting in basal or stress-induced turnover of cell components that provide energy and macromolecular precursors. These activities are thought to be particularly important in cancer where both tumor-promoting and tumor-inhibiting functions of autophagy have been described. Autophagy has also been intricately linked to apoptosis and programmed cell death, and understanding these interactions is becoming increasingly important in improving cancer therapy and patient outcomes. In the review, they consider how recent discoveries about how autophagy manipulation elicits its effects on cancer cell behavior can be leveraged to improve therapeutic responses.

Grainger et al. (1995, Nature Medicine 1: 1067-1073) and Reckless et al. (1997, Circulation 95: 1542-1548) have demonstrated that tamoxifen, a potent inducer of autophagy, inhibited atherosclerosis in mice models by suppressing the diet-induced formation of lipid lesions in the aorta by lowering of low-density lipoprotein (LDL) cholesterol.

CN 102516239A discloses aromatic thiazole micro-molecular organic compounds with the structural formula represented by Formula (I), or hydrates thereof or pharmaceutically acceptable salts thereof. The compounds of the invention or compositions containing the compounds can be used for sun protection, anti-ultraviolet and skin damage protection as a cosmetic additive and for inhibition of the cell apoptosis caused by excess ultraviolet irradiation and the expression of cyclooxygenase COX2.

US 2004-0116425A1 discloses related compounds useful in the treatment of diseases associated with prenylation of proteins and pharmaceutically acceptable salts thereof, to pharmaceutical compositions comprising same, and to methods for inhibiting protein prenylation in an organism using the same.

WO 2009-103432A2 extremely broadly relates to molecular probes of the formula (I) L1-R1-L-A-X as defined herein that allow for the observation of the catalytic activity of a selected caspase, cathepsin, MMP and carboxypeptidase in in vitro assays, in cells or in multicellular organisms.

In WO 2010-147653A1 compounds are extremely broadly disclosed for treating ophthalmic conditions related to mislocalization of opsin proteins, the misfolding of mutant opsin proteins and the production of toxic visual cycle products that accumulate in the eye.

WO 2017-216579A1 relates to a heterocyclic compound 1,1'-(((propane-2,2-diylbis(4,1-phenylene))bis(oxy))bis(ethane-2,1-diyl))dipyrrolidine and its medical uses, for example as an autophagy inducer.

The development of more selective autophagy inducers is needed if they are to become medicinally useful in the treatment and/or prevention of diseases where autophagy plays a role. It is an object of the present invention to provide effective agents that can be used for the prevention and treatment of conditions and diseases that can be treated/prevented by inducing and/or stimulating of autophagy, in particular cancer, age-related diseases, and infections. Other objects and advantages will become apparent to the person of skill when studying the present description of the present invention.

In a first aspect of the present invention, the above object is solved by a compound according to Formula (I), 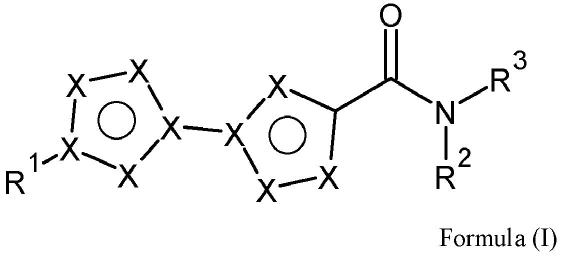 wherein
X is independently selected from chemically possible combinations of C, N, O, and S, and is optionally substituted with -CH₃, -CH₂-CH₃ or COOH;
R¹ is selected from cyclopentyl, cyclohexyl, optionally including chemically possible N, O, and/or S, and optionally mono- or bi-substituted with -CH3, -NH₂, -COOH, C₁ to C₄ alkoxy, halo, trifluoromethyl, trifluoromethoxy;
R² is selected from H, CH₃, straight or branched C₂ to C₆ alkyl, optionally including chemically possible N, O, and/or S, and optionally substituted with -CH₃, -NH₂, -OH, -COOH, cyclopentyl, cyclohexyl, bicyclo[2.2.1]heptane, bicyclo[3.1.1]heptane, bicyclo[2.2.2]octane, optionally including chemically possible N, O, and/or S, and optionally substituted with -CH₃, -CH₂-CH₃, -OH, - isopropyl, -COOH, -COOCH₃, -CH₂-cyclohexyl, -NH₂;
R³ is selected from H, straight or branched C₁ to C₆ alkyl, optionally substituted with -CH₃, -OH, - CH₂-CH₃, -NH₂, -CH₂-NH₂, -CH₂-NH-CH₃, -COOH, optionally including chemically possible one or more N, O, and/or S; C₁ to C₄ alkoxy, optionally including chemically possible one or more N, O, and/or S, and optionally mono- or bi substituted with, -NH₂, -OH, -COOH; cyclopropyl, cyclobutyl, optionally including chemically possible one or more N, O, and/or S, and optionally mono- or bi substituted with -CH₃, -CH₂-CH₃, -NH₂, -CH₂-NH₂, -CH₂-NH-CH₃, -OH, -COOH;
with the provisio that R² and R³ are not both H or CH₃;
R² and R³ may be connected to form a cyclobutyl, cyclopentyl, cyclohexyl, cycloheptyl ring, a bicyclic ring structure of cyclopentyl or cyclohexyl fused with cyclopentyl or cyclohexyl (i.e. cyclopentyl/cyclopentyl, cyclopentyl/cyclohexyl, and cyclohexyl/cyclohexyl), bicyclo[3.1.1]heptane, optionally including chemically possible one or more N, O, and/or S, and optionally being mono- or bi-substituted with straight or branched C₁ to C₆ alkyl, optionally substituted with -CH₃, -OH, -CH₂-CH₃, -NH₂, -NH-CH₃, -CH₂-NH₂, -CH₂-NH-CH₃, -OH, -COOH, -COOCH₃, =O, isopropyl, sulfonamide, and optionally including chemically possible one or more N, O, and/or S;
a physiologically acceptable salt, a solvate, a hydrate, an enantiomer or a polymorph thereof.

The compounds described herein were shown surprisingly to be highly effective autophagy inducers/stimulators. The present inventors thus invented compounds for the induction and/or stimulation of autophagy in disease- and undesired conditions-related cells of a patient or subject. In the case of a compound represented by the general Formula according to the present invention, the autophagy is effectively induced and/or stimulated.

It was surprisingly found that the compounds according to Formula I, as an autophagy inducer, were effective and exhibits many advantages compared to other autophagy-related treatment options. In comparison with prior art compounds as tested (including data not shown), an improvement of the present invention lies in the unexpected observation that the compounds described herein are highly effective autophagy inducers (see Examples below). Based on these results this is evidence that these compounds are active in autophagy-related diseases as disclosed herein. Furthermore, examples for this activity are shown in the Examples below which also relate to specific activities in models of viral infections and diseases involving protein misfolding. By way of an exemplar medical application, the compounds according to Formula 170/171 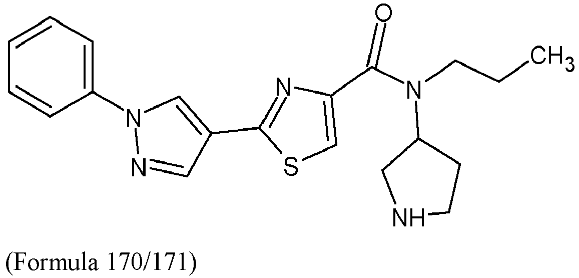 were shown to be surprisingly efficacious (see Examples below).

Preferred is a compound according to the present invention according to the following formula II, 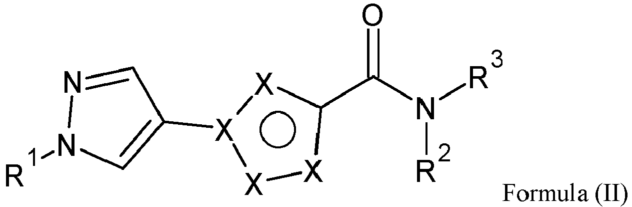 wherein X, R¹, R² and R³ are as above, a physiologically acceptable salt, a solvate, a hydrate, an enantiomer or a polymorph thereof..

Preferred is a compound according to the present invention according to the following formula III, 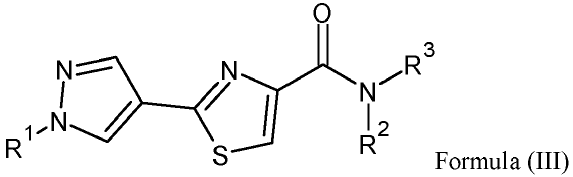 wherein R¹, R² and R³ are as above, a physiologically acceptable salt, a solvate, a hydrate, an enantiomer or a polymorph thereof.

Preferred is a compound according to the present invention, wherein R¹ is selected from 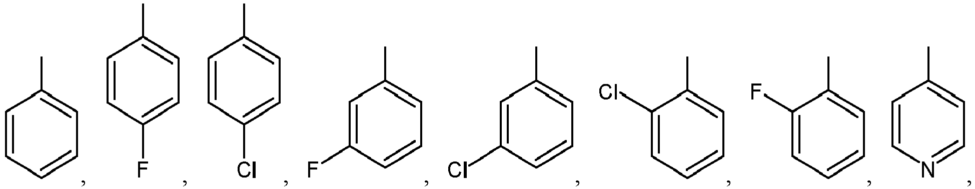 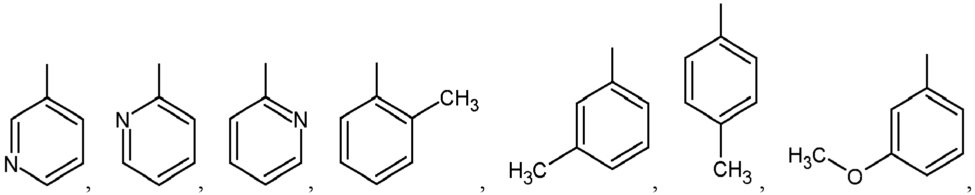 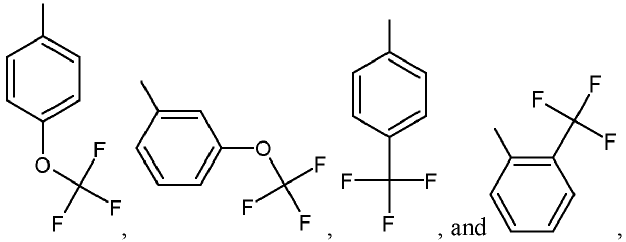 a physiologically acceptable salt, a solvate, a hydrate, an enantiomer or a polymorph thereof.

Preferred is a compound according to the present invention, wherein R¹ and X are as above, R² is selected from H, -CH₃, cyclohexyl, 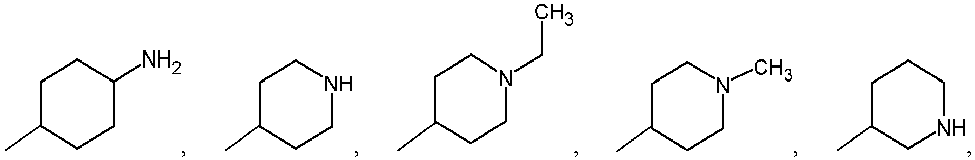 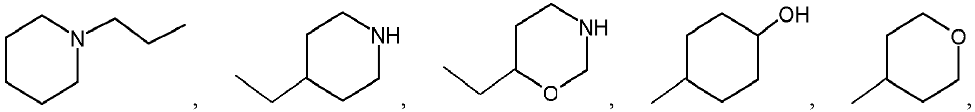 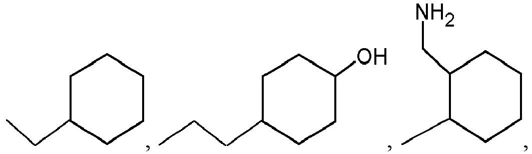 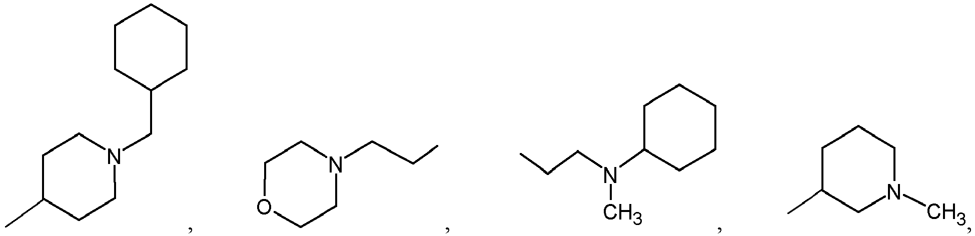 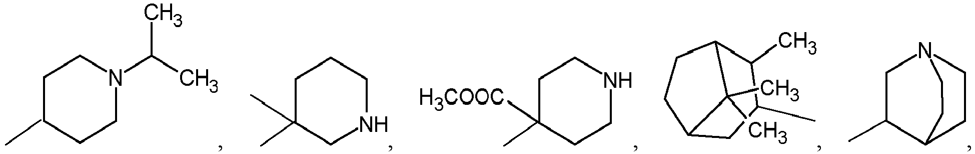 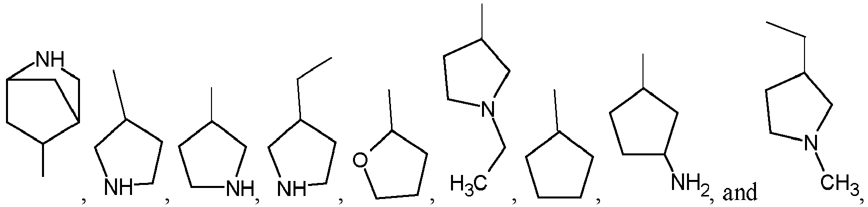 R³ is selected from H, -CH₃, -CH₂-CH₃, propyl, isopropyl, -CH₂-CH₂-OH, -CH₂-CH₂-NH₂, - CH₂-CH₂-CH₂-NH₂, -CH₂-CH₂-CH₂-CH₂-CH₂-NH₂, -CH₂-CH₂-O-CH₂-CH₂- NH₂, -CH₂-CH(CH₃)₂, -CH₂-CH(NH₂)-CH₃, -CH(-CH₃)-CH₂-NH₂, -CH₂- CH₂-N(CH₃)₂, 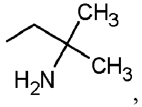 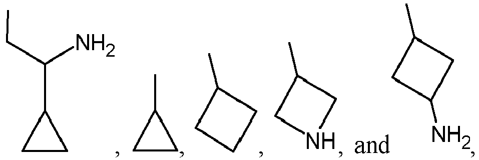 and R² and R³ may be connected to form a ring selected from 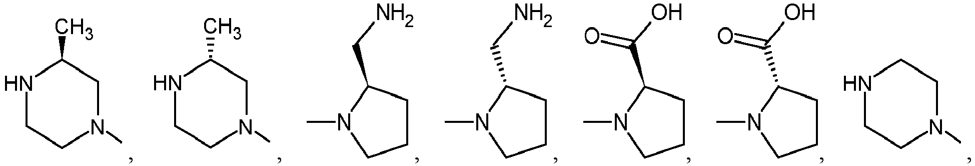 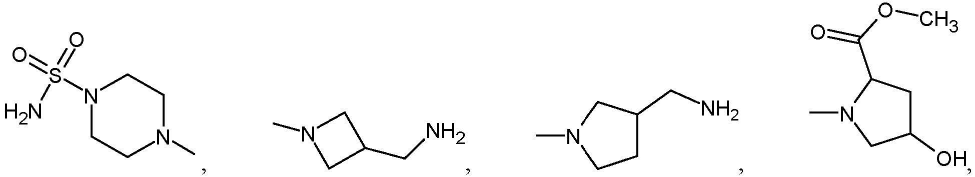 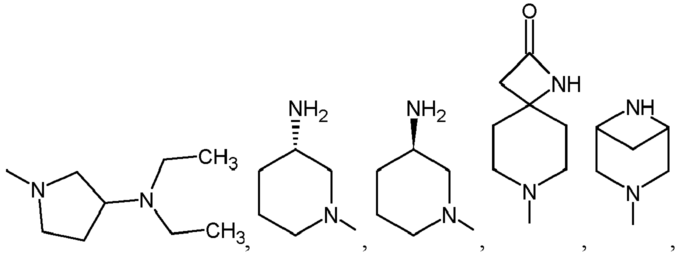 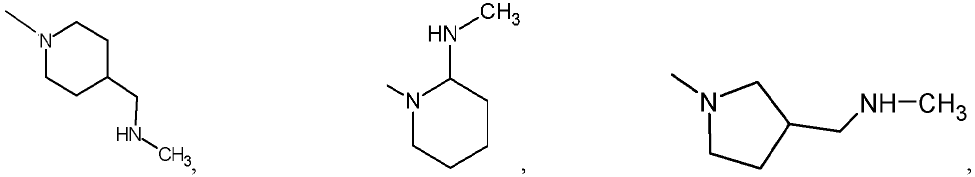 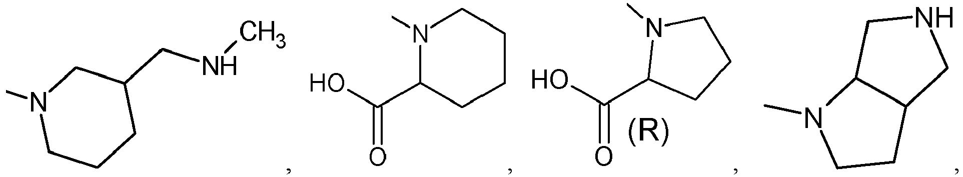 and 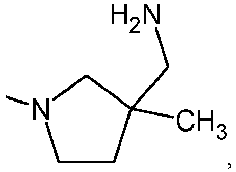 a physiologically acceptable salt, a solvate, a hydrate, an enantiomer or a polymorph thereof.

Preferred is a compound according to the present invention according to the following formula IV, 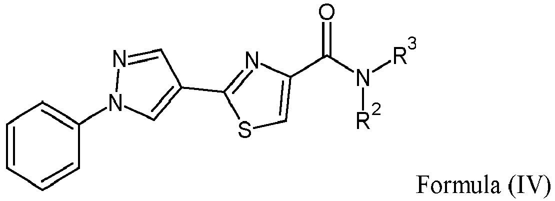 wherein R² is selected from 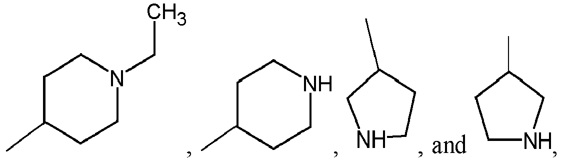 R³ is selected from H, CH₃, -CH₂-CH₂-CH₃, and isopropyl, or R² and R³ form a ring selected from 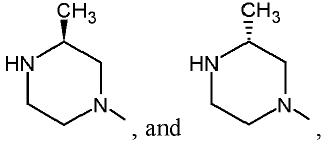 a physiologically acceptable salt, a solvate, a hydrate, an enantiomer or a polymorph thereof.

Preferred is a compound according to the present invention selected from the following:
N-(piperidin-4-yl)-N-(propan-2-yl)-2-{1-[2- (trifluoromethyl)phenyl]- 1H-pyrazol-4-yl}-1,3-thiazole-4-carboxamide (Formula 47);
N-propyl-N-[(3S)-pyrrolidin-3-yl]-2-{1-[3- (trifluoromethyl)phenyl]- 1H-pyrazol-4-yl}-1,3- thiazole-4-carboxamide (Formula 48);
1-[(2R)-1-{2-[1-(3-methoxyphenyl)-1H- pyrazol-4-yl]-1,3-thiazole-4 carbonyl}pyrrolidin-2-yl]methanamine (Formula 50);
(2S)-1-[2-(1-phenyl-1H-pyrazol-4-yl)-1,3-thiazole-4 carbonyl]pyrrolidine-2- carboxylic acid (Formula 60);
2-[1-(2-fluorophenyl)-1H- pyrazol-4-yl]-N-propyl-N- [(3S)-pyrrolidin-3-yl]-1,3- thiazole-4-carboxamide (Formula 92);
5-(1-phenyl-1H-pyrazol-4-yl)-N-propyl-N-[(3S)-pyrrolidin-3-yl]-1H-pyrrole- 2-carboxamide (Formula 93);
5-(1-phenyl-1H-pyrazol-4-yl)-N-propyl-N-[(3R)-pyrrolidin-3-yl]-1H-pyrrole- 2-carboxamide (Formula 94);
5-(1-phenyl-1H-pyrazol-4- yl)-N-propyl-N-[(3S)- pyrrolidin-3-yl]furan-2- carboxamide (Formula 98);
5-(I-phenyl-1H-pyrazol-4- yl)-N-propyl-N-[(3R)- pyrrolidin-3-yl]furan-2- carboxamide (Formula 99);
4-[2-(1-phenyl-1H-pyrazol-4-yl)-1,3-thiazole-4- carbonyl]piperazine-1-sulfonamide (Formula 105); N-[2-(2-aminoethoxy)ethyl]-4-(1-phenyl-1H-pyrazol-4- yl)thiophene-2- carboxamide (Formula 110);
2-(2-phenyl-1,3-oxazol-5- yl)-N-propyl-N-(pyrrolidin- 3-yl)-1,3-thiazole-4- carboxamide (Formula 116);
5-(1-phenyl-1H-pyrazol-4- yl)-N-propyl-N-(pyrrolidin- 3-yl)-1H-pyrrole-2- carboxamide (Formula 120);
2-(5-phenylthiophen-2-yl)-N-propyl-N-(pyrrolidin-3-yl)-1,3-thiazole-4-carboxamide (Formula 122); 4-(1-phenyl-1H-pyrazol-4-yl)-N-propyl-N-(pyrrolidin-3-yl)-1H-pyrrole-2-carboxamide (Formula 123);
1-[2-(1-phenyl-1H-pyrazol-4-yl)-1,3-thiazole-4- carbonyl]azetidin-3-amine (Formula 124); 4-(1-phenyl-1H-pyrazol-4- yl)-N-propyl-N-(pyrrolidin- 3-yl)thiophene-2- carboxamide (Formula 141);
1-[2-(1-phenyl-1H-pyrazol-4-yl)-1,3-thiazole-4-carbonyl]pyrrolidine-2- carboxylic acid (Formula 144);
1-[(2R)-1-[2-(1-phenyl-1H-pyrazol-4-yl)-1,3-thiazole-4 carbonyl]pyrrolidin-2- yl]methanamine (Formula 156);
(3S)-3-methyl-1-[2-(1-phenyl-1H-pyrazol-4-yl)-1,3-thiazole-4- carbonyl]piperazine (Formula 157);
2-(1-phenyl-1H-pyrazol-4- yl)-N-(piperidin-4-yl)-N-(propan-2-yl)-1,3-thiazole- 4-carboxamide (Formula 163);
2-(1-phenyl-1H-pyrazol-4- yl)-N-propyl-N-[(3S)-pyrrolidin-3-yl]-1,3- thiazole-4-carboxamide (Formula 170);
2-(1-phenyl-1H-pyrazol-4-yl)-N-propyl-N-[(3R)-pyrrolidin-3-yl]-1,3- thiazole-4-carboxamide (Formula 171);
N-(1-ethylpiperidin-4-yl)-2-(1-phenyl-1H-pyrazol-4-yl)-1,3-thiazole-4-carboxamide (Formula 185); N-(1-ethylpiperidin-4-yl)-N-methyl-2-(1-phenyl-1H-pyrazol-4-yl)-1,3-thiazole-4 carboxamide (Formula 196); and
a physiologically acceptable salt, a solvate, a hydrate, an enantiomer or a polymorph thereof.

Further preferred is a compound according to the present invention selected from the following:
5-(1-phenyl-1H-pyrazol-4-yl)-N-propyl-N-[(3S)-pyrrolidin-3-yl]-1H-pyrrole- 2-carboxamide (Formula 93);
5-(1-phenyl-1H-pyrazol-4-yl)-N-propyl-N-[(3R)-pyrrolidin-3-yl]-1H-pyrrole- 2-carboxamide (Formula 94);
2-(5-phenylthiophen-2-yl)-N-propyl-N-(pyrrolidin-3-yl)-1,3-thiazole-4-carboxamide (Formula 122); 4-(1-phenyl-1H-pyrazol-4-yl)-N-propyl-N-(pyrrolidin-3-yl)-1H-pyrrole-2-carboxamide (Formula 123);
(3S)-3-methyl-1-[2-(1-phenyl-1H-pyrazol-4-yl)-1,3-thiazole-4- carbonyl]piperazine (Formula 157); 2-(1-phenyl-1H-pyrazol-4- yl)-N-(piperidin-4-yl)-N-(propan-2-yl)-1,3-thiazole- 4-carboxamide (Formula 163);
2-(1-phenyl-1H-pyrazol-4- yl)-N-propyl-N-[(3S)-pyrrolidin-3-yl]-1,3- thiazole-4-carboxamide (Formula 170);
2-(1-phenyl-1H-pyrazol-4-yl)-N-propyl-N-[(3R)-pyrrolidin-3-yl]-1,3 - thiazole-4-carboxamide (Formula 171);
N-(1-ethylpiperidin-4-yl)-2- (1-phenyl-1H-pyrazol-4-yl)- 1,3-thiazole-4-carboxamide (Formula 185); and
N-(1-ethylpiperidin-4-yl)-N-methyl-2-(1-phenyl-1H-pyrazol-4-yl)-1,3-thiazole-4 carboxamide (Formula 196);
a physiologically acceptable salt, a solvate, a hydrate, an enantiomer or a polymorph thereof.

The term "pharmaceutically acceptable salt" refers to a pharmaceutically acceptable organic or inorganic salt of the compound of the invention. This may include addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Exemplary salts also include oxalate, chloride, bromide, iodide, bisulphate, acid phosphate, isonicotinate, salicylate, acid citrate, oleate, tannate, pantothenate, bitartrate, ascorbate, gentisinate, gluconate, glucuronate, saccharate, formate, benzoate, glutamate, ethanesulfonate, and benzenesulfonate salts. For other examples of pharmaceutically acceptable salts, reference can be made to Gould (1986, Int J Pharm 33: 201-217).

According to a further aspect of the invention, there is a provided a method for producing a compound according to the present invention, comprising the steps according to any one of general experimental procedures 1 to 4 as disclosed herein.

According to a further aspect of the invention, there is a provided a pharmaceutical composition comprising a pharmaceutically effective amount of the compound according to the present invention, and a pharmaceutically or therapeutically acceptable excipient or carrier.

The term "pharmaceutically or therapeutically acceptable excipient or carrier" refers to a solid or liquid filler, diluent or encapsulating substance which does not interfere with the effectiveness or the biological activity of the active ingredients and which is not toxic to the host, which may be either humans or animals, to which it is administered. Depending upon the particular route of administration, a variety of pharmaceutically-acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water. Pharmaceutically acceptable carriers or excipients also include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal Si0₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for the compounds according to the present invention, and will readily be able to choose suitable pharmaceutically acceptable carriers or excipients, depending, e.g., on the formulation and administration route of the pharmaceutical composition.

All suitable modes of administration are contemplated according to the invention. For example, administration of the medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, buccal, sublingual, transmucosal, inhalation, intra- atricular, intranasal, rectal or ocular routes, abuse deterrent and abuse resistant formulations, sterile solutions suspensions and depots for parenteral use, and the like, administered as immediate release, sustained release, delayed release, controlled release, extended release and the like. The medicament may be formulated in discrete dosage units and can be prepared by any of the methods well known in the art of pharmacy. The pharmaceutical composition of the present invention can be formulated using methods known in the art to provide rapid, sustained or delayed release of the active ingredient after administration to a mammal. In general, at least one compound according to the present invention is admixed with at least one pharmaceutically acceptable carrier and/or excipient.

In addition to the aforementioned compounds of the invention, the pharmaceutical composition can contain two or more compounds according to the present invention and also other therapeutically active substances.

The dosage of the pharmaceutical composition according to the present invention can be appropriately selected according to the route of administration, the subject to be administered, the target disease and its severity, age, sex weight, individual differences and disease state. Dosage may be repeated several times a day.

According to the present invention, a mammalian subject can be preferably selected from a mouse, rat, cat, dog, rabbit, goat, sheep, horse, camel, lama, cow, monkey, a farm animal, a sport animal, and a pet, and a human.

Further provided is a compound of the invention as defined herein for use in the prevention and/or treatment of conditions and/or diseases in a mammalian subject, such as a human. A condition and/or disease suitable for treatment according to the relevant aspects of the invention is one which is characterized by defective or insufficient autophagy or which would benefit from modulation such as induction of autophagy.

The invention further encompasses the use of a compound of the invention as an autophagy inducer. The use may be a cosmetic use and/or in vitro, for example in an in vitro assay.

US 9,138,400, for example, relates to a cosmetic process for detoxifying the skin and/or for combating cutaneous aging, comprising the topical application on the skin of a composition that comprises at least one activator of the autophagy of cells of the skin. Eckhart L, Tschachler E, and Gruber F (in: Autophagic Control of Skin Aging. Front Cell Dev Biol. 2019;7:143. Published 2019 Jul 30. doi:10.3389/fcell.2019.00143) review the evidence for cell type-specific roles of autophagy in the skin and their differential contributions to aging.

Modified or altered autophagy has been shown to be relevant in neurodegenerative disease, as demonstrated by the accumulation of protein aggregates, for example in Alzheimer disease, Parkinson's disease, polyglutamine diseases, muscle diseases, and amyotrophic lateral sclerosis. Modified autophagy has also been implicated in other neurological diseases including epilepsies, neurometabolic and neurodevelopmental disorders such as schizophrenia. Autophagy inhibition plays a key role in the pathogenesis of inherited autophagic vacuolar myopathies (including Danon disease, X- linked myopathy with excessive autophagy, and infantile autophagic vacuolar myopathy), all of which are characterized by lysosomal defects and an accumulation of autophagic vacuoles. Autophagic vacuolar myopathies and cardiomyopathies can also be secondary to treatment with autophagy-inhibiting drugs (chloroquine, hydroxychloroquine and colchicine), which are used experimentally to interrogate autophagic flux and clinically to treat malaria, rheumatological diseases, and gout.

Autophagy impairment has also been implicated in the pathogenesis of inclusion body myositis, an age- associated inflammatory myopathy that is currently refractory to any form of treatment, along with other muscular dystrophies such as tibial muscular dystrophy.

Modified basal autophagy levels are seen in rheumatoid arthritis and osteoarthritis. Other aspects of the immune response associated with dysfunctional autophagy are seen in neutrophils from patients with familial Mediterranean fever and in monocytes from patients with TNF receptor-associated periodic syndrome, both of which are autoinflammatory disorders. Moreover, autophagy regulates an important neutrophil function, the generation of neutrophil extracellular traps (NETs). The important role of autophagy in the induction of NET formation has been studied in several neutrophil-associated disorders such as gout, sepsis, and lung fibrosis. Furthermore, there is a relationship between autophagy and the secretory pathway in mammalian macrophages for the release of IL1B, demonstrating a possible alternative role of autophagy for protein trafficking. This role has also been implied in neutrophils through exposure of protein epitopes on NETs by acidified LC3-positive vacuoles in sepsis and anti-neutrophil cytoplasmic antibody associated vasculitis. Patients with chronic kidney disease also have impaired autophagy activation in leukocytes, which is closely related to their cardiac abnormalities. There is also evidence for altered autophagy in pancreatic beta cells and in adipocytes of patients with type 2 diabetes.

A crucial role for therapy-induced autophagy in cancer cells has recently emerged, in modulating the interface of cancer cells and the immune system; primarily, by affecting the nature of danger signaling (i.e., the signaling cascade that facilitates the exposure and/or release of danger signals) associated with immunogenic cell death (ICD).

Therefore, compounds according to the present invention are for use in the prevention and/or treatment of an autophagy-related disease or condition in a mammalian subject, such as a human.

By "treatment" or "treating" is meant any treatment of a disease or disorder, in a mammal, including: preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require complete recovery or complete prevention of a stress.

Preferred is a compound for use according to the present invention, wherein said autophagy-related disease or condition is selected from the group consisting of neurodegenerative diseases, Huntington's disease, Alzheimer's disease, Parkinson's disease, systemic lupus erythematosus, epilepsy, cancer, liver diseases, a1 antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKU1, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, stroke, coronary artery diseases, myocardial infarction, unstable angina pectoris, atherosclerosis or vasculitis, Behcet's syndrome, giant cell arteritis, polymyalgia rheumatica, Wegener's granulomatosis, Churg-Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, ALS, antigen induced recall response, immune response suppression, muscle degeneration and atrophy, frailty in aging, spinal cord injury, and diseases and conditions involving misfolded and/or nonfolded proteins as well as age-related forms of the diseases and conditions (such as neurodegenerative disease, cancer, and metabolic syndrome). Particularly preferred are ALS, Alzheimer's disease, Parkinson's disease, and Charcot Marie Tooth syndrome.

Cheon SY, Kim H, Rubinsztein DC, and Lee JE. (in: Autophagy, Cellular Aging and Age-related Human Diseases. Exp Neurobiol 2019;28:643-657. https://doi.org/10.5607/en.2019.28.6.643) disclose that during aging, cellular factors suggested as the cause of aging have been reported to be associated with progressively compromised autophagy. Dysfunctional autophagy may contribute to age-related diseases, such as neurodegenerative disease, cancer, and metabolic syndrome, in the elderly. Therefore, restoration of impaired autophagy to normal may help to prevent age-related disease and extend lifespan and longevity. They provide an overview of the mechanisms of autophagy underlying cellular aging and the consequent disease.

Similarly, Leidal, A.M., Levine, B. & Debnath, J. (in: Autophagy and the cell biology of age-related disease. Nat Cell Biol 20, 1338-1348 (2018). https://doi.org/10.1038/s41556-018-0235-8) review that autophagy declines with age and that impaired autophagy predisposes individuals to age-related diseases, whereas interventions that stimulate autophagy often promote longevity.

Also, Vaccaro Maria Ines, De Tata Vincenzo, and Gonzalez Claudio Daniel (in: Editorial: Autophagy in Endocrine-Metabolic Diseases Associated With Aging; Frontiers in Endocrinology, 11, 2020, pp 572, doi=10.3389/fendo.2020.00572) present a special issue containing a collection of 12 articles covering a broad range of key topics on the interplay of the different types of autophagy alterations with aging, endocrine-metabolic, and degenerative diseases.

Further preferred is the compound for use according to the present invention, wherein said prevention and/or treatment comprises a combination of at least two compounds for use according to the present invention, and/or a combination with at least one additional pharmaceutically active substance for said autophagy-related disease or condition.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical composition of the present invention are employed in co-therapy approaches, i.e. in co-administration with other medicaments or drugs and/or any other therapeutic agent which might be beneficial in the context of the methods of the present invention. Nevertheless, the other medicaments or drugs and/or any other therapeutic agent can be administered separately from the compound for use, if required, as long as they act in combination (i.e. directly and/or indirectly, preferably synergistically) with the present compound(s) (for use).

In another aspect of the compound for use according to the present invention the prevention and/or treatment further comprises detecting and/or monitoring in said subject a response of at least one autophagy-biomarker. The biomarker is preferably selected from the group consisting of BECN1, ATG8/LC3 family, including LC3A, LC3B, LC3C), LC3-II, ULK1, p62, NBR1, ATG5 and ATG7.

This monitoring usually is performed on a biologically sample taken from the mammalian subject, and comprises commonly known tests, for example antibody based, PCR based, and the like. The tests are repeated over time, and can be compared to control samples and/or samples taken earlier from the mammalian subject. The results help the attending physician to maintain or modify the course of a treatment, usually based on the severity of the clinical symptoms of the autophagy-related disease and/or condition as treated.

In another aspect thereof, the present invention provides methods for preventing and/or treating an autophagy-related disease and/or condition in a mammalian subject, such as a human, comprising administering to said mammal an effective amount of a compound or a pharmaceutical composition according to the present invention.

Preferred is the method according to the present invention, wherein said autophagy-related disease or condition is selected from the group consisting of neurodegenerative diseases, Huntington's disease, Alzheimer's disease, Parkinson's disease, systemic lupus erythematosus, epilepsy, cancer, liver diseases, a1 antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKU1, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, stroke, coronary artery diseases, myocardial infarction, unstable angina pectoris, atherosclerosis or vasculitis, Behcet's syndrome, giant cell arteritis, polymyalgia rheumatica, Wegener's granulomatosis, Churg-Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, ALS, antigen induced recall response, immune response suppression, muscle degeneration and atrophy, frailty in aging, spinal cord injury, and diseases and conditions involving misfolded and/or nonfolded proteins. Particularly preferred are ALS, Alzheimer's disease, Parkinson's disease, and Charcot Marie Tooth syndrome.

The dosage of the pharmaceutical composition to be administered according to the present invention can be appropriately selected according to the route of administration, the subject to be administered, the target disease and its severity, age, sex weight, individual differences and disease state. Dosage may be repeated several times a day.

According to the present invention, a mammalian subject can be preferably selected from a mouse, rat, cat, dog, rabbit, goat, sheep, horse, camel, lama, cow, monkey, a farm animal, a sport animal, and a pet, and a human.

In addition to the aforementioned compounds of the invention, the pharmaceutical composition as administered can contain two or more compounds according to the present invention and also other therapeutically active substances. In the method, the compound for use can be provided and/or is administered as a suitable pharmaceutical composition as discussed above. The compounds can be administered alone or in combination with other active compounds - for example with medicaments already known for the treatment of the aforementioned conditions and/or diseases, whereby in the latter case a favorable additive, amplifying or preferably synergistically effect is noticed.

In another aspect of the method according to the present invention the prevention and/or treatment further comprises detecting and/or monitoring in said subject a response of at least one autophagy-biomarker. The biomarker is preferably selected from the group of BECN1, and the ATG8/LC3 family, including LC3A, LC3B, LC3C), LC3-11, ULK1, p62, NBR1, ATG5 and ATG7.

This monitoring usually is performed on a biologically sample taken from the mammalian subject, and comprises commonly known tests, for example antibody based, PCR based, and the like. The tests are repeated over time, and can be compared to control samples and/or samples taken earlier from the mammalian subject. The results help the attending physician to maintain or modify the course of a treatment, usually based on the severity of the clinical symptoms of the autophagy-related disease and/or condition as treated.

It was surprisingly found that the compounds according to Formula I, as an autophagy inducer, were effective and exhibits many advantages compared to other autophagy-related treatment options, particularly as a combination treatment. In comparison with prior art compounds as tested (including data not shown), an improvement of the present invention lies in the unexpected observation that the compounds described herein are highly effective autophagy inducers (see Examples below). By way of an exemplar medical application, the compounds according to Formula 170/171 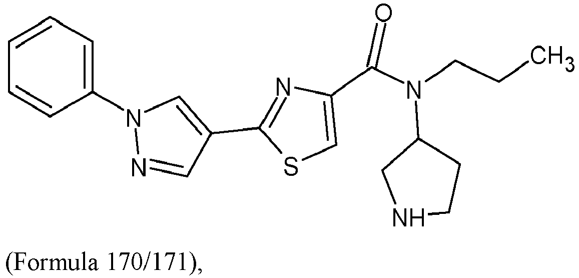 were shown to be surprisingly efficacious (see Examples below).

The present invention will now be described further in the following examples, nevertheless, without being limited thereto. For the purposes of the present invention, all references as cited herein are incorporated by reference in their entireties.

### EXAMPLES

The following examples have been performed in part using preferred compounds as disclosed. Nevertheless, it should be understood that the present invention is nor in any way limited thereto, and the person of skill is readily able to adjust the conditions as described to other compounds according to the present invention.

Compounds of the present invention were synthesized from commercially available starting materials, in preferably by following the relevant general experimental procedures as given below (general experimental procedures 1-4).

### Abbreviations used:

- Boc: tertiary-butoxycarbonyl
- *t*Bu: tertiary butyl
- DCM: dichloromethane
- DIPEA: N,N-Diisopropylethylamine
- DMF: dimethylformamide
- DMSO: dimethylsulfoxide
- dppf: 1, 1'-bis(diphenylphosphino)ferrocene
- eq: equivalents
- ESI: electrospray ionisation
- Hz: hertz
- H: hours
- HATU: 1-[Bis(dimethylamino)methylene]-1H-1,2,3 -triazolo[4,5-b]pyridinium 3-oxide hexafluorophosphate
- HPLC: high performance liquid chromatography
- *J*: coupling constant in Hz
- LCMS: liquid chromatography-mass spectrometry
- min: minutes
- NMR: nuclear magnetic resonance
- N: normal (equivalents per litre)
- Ph: phenyl
- RT: retention time
- rt: room temperature
- SFC: supercritical fluid chromatography
- THF: tetrahydrofuran
- TLC: thin-layer chromatography
- vol: volumes
- X-Phos: 2-Dicyclohexylphosphin-2',4',6'-triisopropylbiphenyl
- X-Phos Pd G2 chloride: Second generation XPhos Precatalyst (X-Phos aminobiphenyl palladium precatalyst); Chloro(2-dicyclohexylphosphino-2',4',6'-triisopropyl-1,1'-biphenyl)[2-(2'-amino-1,1'-biphenyl)]palladium(II)

### General experimental procedure 1

Schematic overview over synthesis:

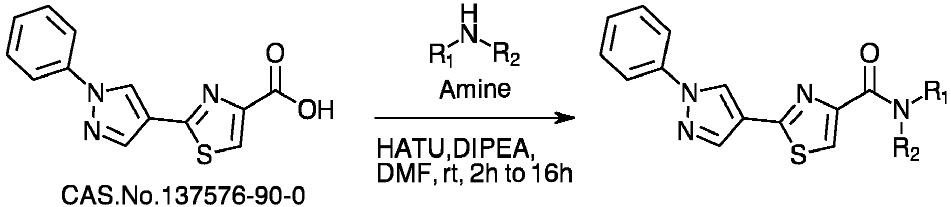

To a solution of 2-(1-phenyl-1H-pyrazol-4-yl)thiazole-4-carboxylic acid (CAS.No.137576-90-0 , 1.0 eq) in DMF (10 vol) at room temperature, HATU (1.5 eq) and DIPEA (3.0 eq) were added. The reaction mixture was stirred at rt for 15-30 min, then the amine derivative (1.0 eq) was added to reaction mixture at room temperature. The reaction mixture was stirred at rt for 2 h to 16 h (the progress of the reaction was monitored by TLC and LCMS analysis). After completion of the reaction, the reaction mixture was poured into cold water (10 vol) and extracted with ethyl acetate (3 × 5 vol). The combined organic fractions were washed with cold water 3-4 times, dried over sodium sulfate and concentrated under reduced pressure. The crude material was purified by flash column chromatography (CombiFlash^{®} system) to provide the target compound.

### General experimental procedure 2

Schematic overview over synthesis:

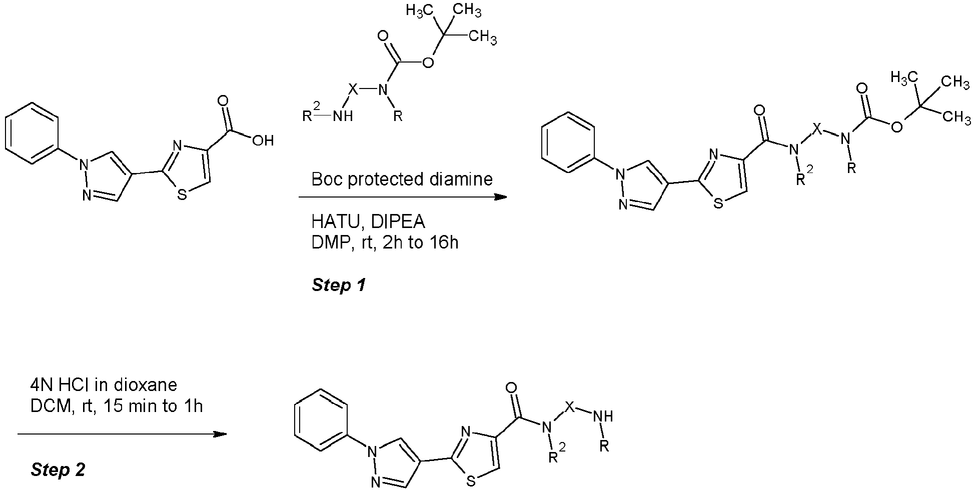

### Step 1 - Synthesis of Boc-protected intermediate

To a solution of 2-(1-phenyl-1H-pyrazol-4-yl) thiazole-4-carboxylic acid (CAS. No. 137576-90-0, 1.0 eq) in DMF (10 vol) at rt, HATU (1.5 eq) and DIPEA (3.0 eq) were added. The reaction mixture was stirred for 15-20 min, then the Boc-protected amine derivative (1.0 eq) was added to reaction mixture at rt. The reaction mixture was stirred at rt for 2 h to 16 h (the progress of the reaction was monitored by TLC and LCMS analysis). After completion of the reaction, the reaction mixture was poured into cold water (10 vol) and extracted with ethyl acetate (3 × 5 vol). The combined organic fractions were washed with cold water 3-4 times, dried over sodium sulfate and concentrated under reduced pressure. The crude material was purified by flash column chromatography (CombiFlash^{®} system) to provide the Boc-protected intermediate.

### Step 2 - Deprotection

To a stirred solution of the Boc-protected intermediate (1.0eq) in DCM (10 vol) at 0°C or rt, 4N HCl in dioxane was added. The resulting reaction mixture was stirred at rt for 15min to 1 h (the progress of the reaction was monitored by TLC and LCMS analysis). After completion of reaction, the reaction mixture was concentrated under reduced pressure and triturated (typically with diethyl ether or DCM) to provide the target compound.

### General experimental procedure 3

Schematic overview over synthesis (X = unspecified heteroatom):

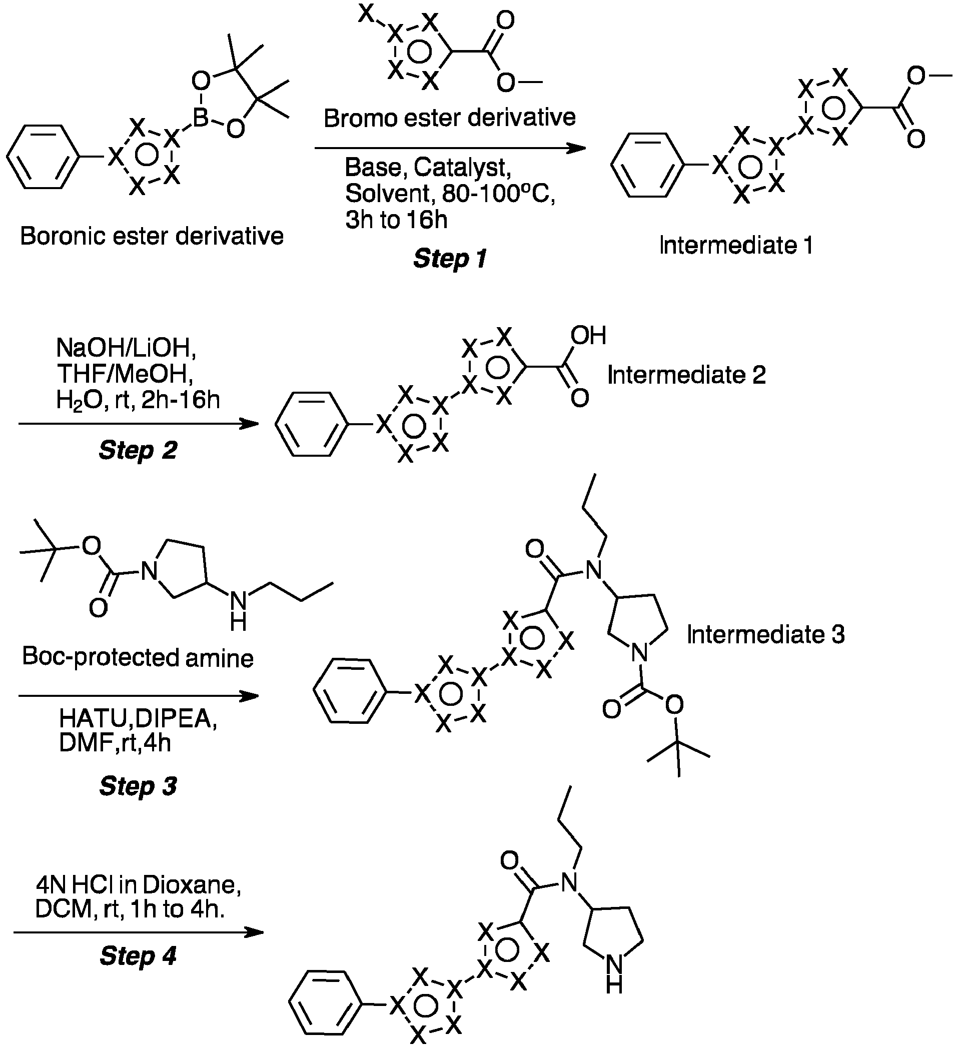

### Step 1 - Cross-coupling

To a stirred solution of the Boronic ester derivative (1.0 eq) and the Bromo ester derivative (1.2 eq) in a suitable solvent (DMF or 1,4-dioxane:water (9:1, 10 vol) at rt, a suitable base (potassium phosphate or potassium carbonate or sodium carbonate; 2.0 to 3.0 eq) was added. The reaction mixture was degassed with nitrogen gas for 10 min. A suitable palladium catalyst (Pd(PPh₃)₄ or PdCl₂(dppf).DCM or X-Phos Pd G2 or (t-Bu₃P)₂Pd; 0.1 eq) was added. The reaction mixture was again degassed with nitrogen gas for 10 min and then heated at 80 °C-100 °C for 3 h to 16 h (the progress of the reaction was monitored by TLC and LCMS analysis). After completion of the reaction, the reaction mixture was diluted with water (10 vol) and extracted with ethyl acetate (3 × 5 vol). The combined organic fractions were dried over anhydrous sodium sulfate and concentrated under reduced pressure. The crude material was purified by flash column chromatography (CombiFlash^{®} system) to provide Intermediate 1.

### Step 2 - Ester hydrolysis

To a stirred solution of Intermediate 1 (1.0eq) in a suitable solvent (typically THF (10 vol) or MeOH (10 vol) or EtOH (10 vol)) at rt, a suitable base (typically LiOH (8.0 eq) in water (10 vol) or NaOH (5.0eq) in water (10 vol)) was added. The reaction mixture was stirred at rt for 2 h to 16 h (the progress of the reaction was monitored by TLC and LCMS analysis). After completion of reaction, the reaction mixture was distilled out and extracted with ethyl acetate (2 X 5 vol). The aqueous layer was acidified (typically with 1N HCl or a saturated solution of citric acid) and extracted with ethyl acetate (3 × 10 vol). The combined organic fractions were dried over sodium sulfate and concentrated under reduced pressure to provide Intermediate 2.

### Step 3- Formation of Boc-protected amide

To a stirred solution of Intermediate 2 in DMF (10 vol) at room temperature, HATU (1.5 eq) and DIPEA (3.0 eq) were added. The reaction mixture was stirred at rt for 15-30 min, then the Boc-protected amine (1.0 eq) was added into reaction mixture. The reaction mixture was stirred at rt (the progress of the reaction was monitored by TLC and LCMS analysis). After completion of reaction (typically ~4h), the reaction mixture was poured into cold water (10 vol) and extracted with ethyl acetate (3 × 10 vol). The combined organic fractions were washed with cold water 3-4 times, dried over sodium sulfate and concentrated under reduced pressure. The crude material was purified by flash column chromatography (CombiFlash^{®} system) to provide Intermediate 3.

### Step 4 - deprotection

To a stirred solution of Intermediate 3 (1.0eq) in DCM (10 vol) at 0°C, 4N HCl in Dioxane (5 vol) was added. The reaction mixture was stirred at rt for 1 h to 4 h (the progress of the reaction was monitored by TLC and LCMS analysis). After completion of reaction, the reaction mixture was concentrated under reduced pressure to provide crude material, which was triturated with diethyl ether to provide the target compound.

### General experimental procedure 4

Schematic overview over synthesis (X = unspecified heteroatom):

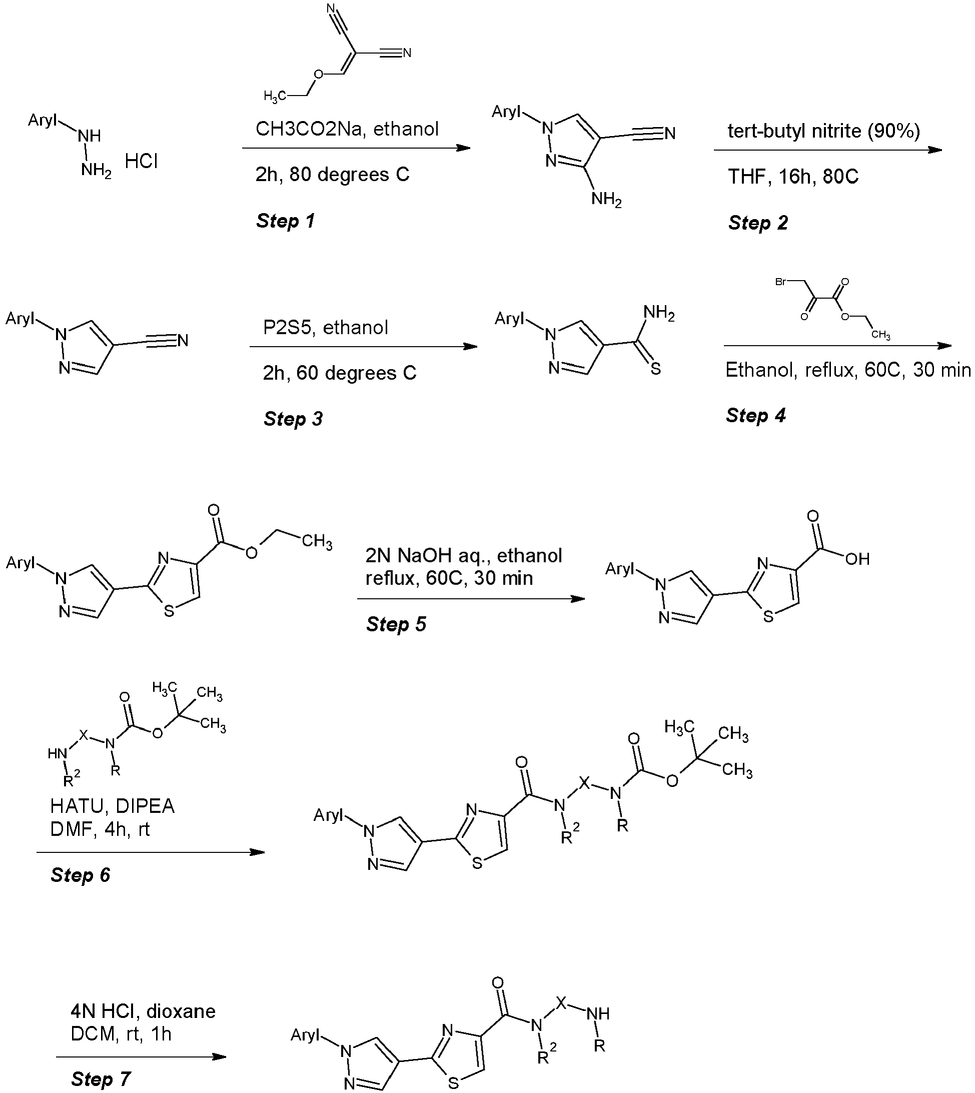

### Step-1: Synthesis of 5-amino-1H-pyrazole-4-carbonitrile intermediate

To a stirred solution of the appropriate hydrazine hydrochloride (20.69mmol) in ethanol (50mL) at rt, sodium acetate (3.39 g, 41.39mmol) and 2-(ethoxymethylene)malononitrile (2.52 g, 20.69mmol) were added. The reaction mixture was refluxed at 80°C for 2h (the progress of reaction was monitored by TLC and LCMS analysis). After completion of the reaction, the reaction mixture was concentrated under reduced pressure. The residual material was washed with cold water (50 mL) and extracted with ethyl acetate (3 × 100 mL). The combined organic fractions were dried over anhydrous sodium sulfate and concentrated under reduced pressure to provide the crude 5-amino-1-aryl-1H-pyrazole-4-carbonitrile.

### Step-2: Synthesis of 1H-pyrazole-4-carbonitrile intermediate:

To a stirred solution of the crude 5-amino-1-aryl-1H-pyrazole-4-carbonitrile (18.24 mmol) in THF (50 mL) at rt, tert-butyl nitrite(90%)(4.13g,36.49mmol) was added. The reaction mixture was refluxed at 80 °C for 16 h (the progress of the reaction was monitored by TLC and LCMS analysis). After completion of reaction, the reaction mixture was concentrated under reduced pressure. The residual material was purified by flash column chromatography to provide the 1H-pyrazole-4-carbonitrile.

### Step-3: Synthesis of 1H-pyrazole-4-carbothioamide intermediate

To a stirred solution of the 1H-pyrazole-4-carbonitrile (15.5 mmol) in ethanol (37 mL) at rt, phosphorus pentasulfide (6.93 g, 31.1 mmol) was added. The reaction mixture was refluxed at 60 °C for 2 h (the progress of the reaction was monitored by TLC and LCMS analysis). After completion of the reaction, the reaction mixture was distilled under vacuum. The crude reaction mixture was washed with sodium bicarbonate solution (100 mL) until neutralization of pH and extracted with ethyl acetate (3 × 150 mL). The combined organic fractions were dried over sodium sulfate, concentrated under reduced pressure and triturated with diethyl ether to afford the crude 1H-pyrazole-4-carbothioamide.

### Step-4: Synthesis of ethyl 2-aryl-1H-pyrazol-4-yl)thiazole-4-carboxylate:

To a stirred solution of the 1H-pyrazole-4-carbothioamide (14.70 mmol) in ethanol (50 mL) at rt, ethyl 3-bromo-2-oxopropanoate (3.14 g, 16.17 mmol) was added. The reaction mixture was refluxed at 60 °C for 30 min (the progress of the reaction was monitored by TLC and LCMS analysis). After completion of the reaction, the reaction mixture was distilled under vacuum to provide the ethyl 2-aryl-1H-pyrazol-4-yl)thiazole-4-carboxylate.

### Step-5: Synthesis of 2-aryl-1H-pyrazol-4-yl)thiazole-4-carboxylic acid:

To a stirred solution of the ethyl 2-aryl-1H-pyrazol-4-yl)thiazole-4-carboxylate (14.4 mmol) in ethanol (60 mL) at rt, 2 N NaOH solution (21.62 mL) was added. The reaction mixture was heated at 60 °C for 30 minutes (the progress of the reaction was monitored by TLC and LCMS analysis). After completion of the reaction, the reaction mixture was poured into water (50 mL) and extracted with ethyl acetate (3 × 50 mL). The aqueous layer was separated out and acidified with 2 M HCl solution to generated a precipitatwhich was isolated by filtration and dried under reduced pressure to provide the 2-aryl-1H-pyrazol-4-yl)thiazole-4-carboxylic acid.

### Step-6: Synthesis of final thiazole-4-carboxamide compound or N-Boc protected thiazole-4-carboxamide intermediate

To a stirred solution of 2-aryl-1H-pyrazol-4-yl)thiazole-4-carboxylic acid (0.58 mmol) in DMF (4 mL) at rt, HATU (0.33g, 0.87 mmol) and DIPEA (0.21 g, 1.7 mmol) were added. The reaction mixture was stirred for 30 min and then the required amine (0.58mmol) was added at rt. The reaction mixture was stirred at rt for 4h (the progress of the reaction was monitored by TLC and LCMS analysis). After completion of the reaction, the reaction mixture was poured into cold water (15 mL) and extracted with ethyl acetate (3 × 50 mL). The combined organic fractions were washed with cold water 3-4 times, dried over sodium sulfate and concentrated under reduced pressure. The crude material was purified by flash column chromatography) to provide the product as a solid.

### Step-7: deprotection of thiazole-4-carboxamide to obtain final product

To a stirred solution of the N-Boc protected intermediate from Step 6 above (0.35 mmol) in DCM (2 mL, 10V) at 0°C, 4M HCl in Dioxane (1mL, 5V) was added. The reaction mixture was stirred at room temperature for 1h (the progress of the reaction was monitored by TLC and LCMS analysis). After completion of reaction, the reaction mixture was concentrated under reduced pressure, and the crude material triturated with diethyl ether to provide the target thiazole-4-carboxamide hydrochloride as a solid.

### Full synthetic details for a representative set of compounds

### 1. Synthesis of N-(1-ethylpiperidin-4-yl)-N-methyl-2-(1-phenyl-1H-pyrazol-4-yl)thiazole-4-carboxamide (Compound 196):

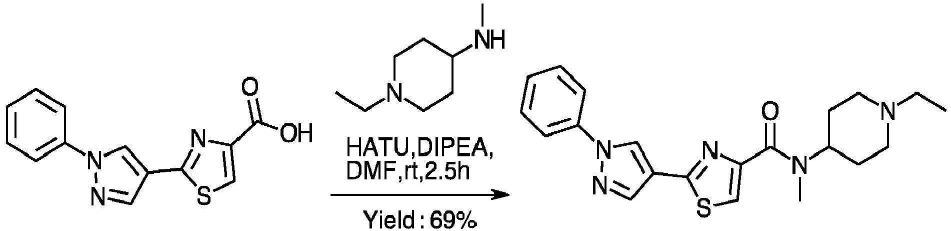

Prepared according to General experimental procedure 1 using 2-(1-phenyl-1H-pyrazol-4-yl)thiazole-4-carboxylic acid (0.050g, 0.18mmol), HATU (0.105g, 0.27mmol) and DIPEA (0.1mL, 0.55mmol) in DMF (1.8 mL), stirred for 30 min prior to the addition of 1-ethyl-N-methylpiperidin-4-amine (0.026g, 0.18mmol). The reaction mixture was stirred at rt for 2.5 h. The crude material was purified by flash column chromatography (CombiFlash^{®} system; 4-5% MeOH/DCM) to provide N-(1-ethylpiperidin-4-yl)-N-methyl-2-(1-phenyl-1H-pyrazol-4-yl)thiazole-4-carboxamide **(Compound 196:** 0.043g, 69% yield) as an pale yellow solid.
**LCMS [ESI, M+1]:** 396.12 (RT: 1.280 min, Purity: 100%),
**HPLC:** RT: 6.816 min, Purity: 100%
¹H **NMR (400 MHz, *d*₆-DMSO)** δ 9.12 (s, 1H), 8.17 (d, *J* = 22.9 Hz, 1H), 7.91 - 7.84 (m, 3H), 7.47 (t, *J* = 7.8 Hz, 2H), 7.30 (t, *J* = 7.2 Hz, 1H), 4.26 (m, 1H), 3.73 (m, 1H), 2.97 (m, 2H), 2.88 (s, 3H), 1.72 (m, 7H), 0.89 (t, *J* = 18 Hz, 3H).

### 2. Synthesis of 4-(2-(1-phenyl-1H-pyrazol-4-yl) thiazole-4-carbonyl)piperazine-1-sulfonamide (Compound 105):

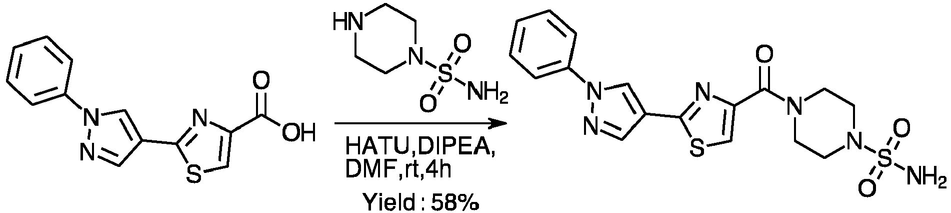

Prepared according to general procedure 1 using 2-(1-phenyl-1H-pyrazol-4-yl) thiazole-4-carboxylic acid (0.100g, 0.36mmol), HATU (0.210g, 0.55mmol) and DIPEA (0.143g, 7.74mmol) in DMF (1mL), stirred for 15 min prior to the addition of piperazine-1-sulfonamide (0.480g, 1.10mmol. The reaction mixture was stirred at rt for 4h. The following modification to the general procedure were used: after completion of the reaction, the reaction mixture was poured into ice cold water to obtain a solid precipitate. The solid material was isolated by filtration and washed with ice cold water (20mL) and hexane(30mL). The solid material was triturated with diethyl ether to provide 4-(2-(1-phenyl-1H-pyrazol-4-yl)thiazole-4-carbonyl)piperazine-1-sulfonamide (**Compound 105:** 0.090g, 58% yield) as a light brown solid.
**LCMS [ESI, M+1]:** 418.9 (RT: 1.576 min, Purity: 89.38%),
**HPLC:** RT: 6.139 min, Purity: 95.57%,
**¹H NMR (400 MHz, *d*₆-DMSO)** : δ 9.21 (s, 1H), 8.30 (s, 1H), 8.10 (s, 1H), 7.94 (d, J = 7.8 Hz, 2H), 7.55 (t, J = 7.9 Hz, 2H), 7.38 (t, J = 7.4 Hz, 1H), 6.89 (s, 2H), 3.81 (m, 4H), 3.04 (m, 4H).

### 3. Synthesis of N-isopropyl-2-(1-phenyl-1H-pyrazol-4-yl)-N-(piperidin-4-yl)thiazole-4-carboxamide hydrochloride (Compound 163):

### Overall synthetic scheme

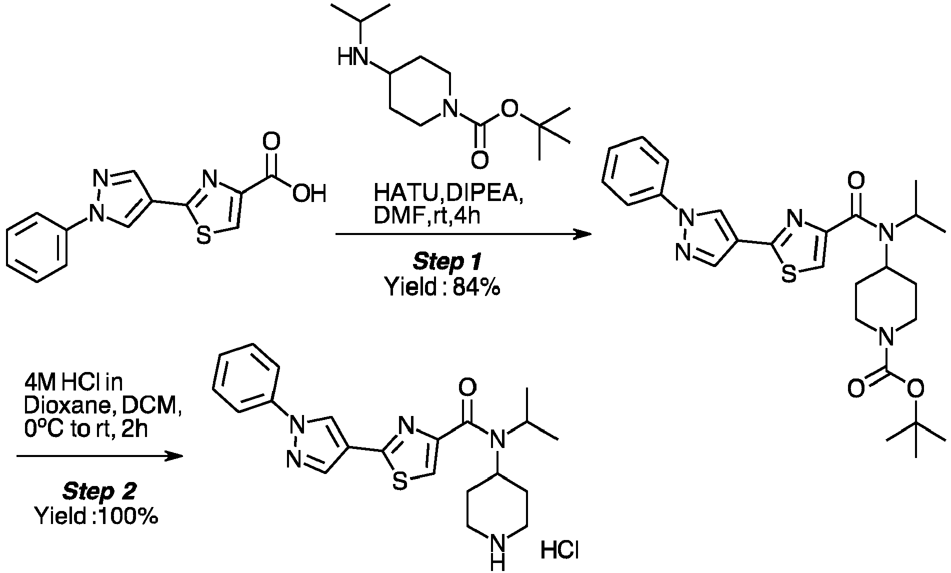

Synthetic procedure - Prepared according to general experimental procedure 2.

In **step 1** 2-(1-phenyl-1H-pyrazol-4-yl)thiazole-4-carboxylic acid (1.5g, 5.52mmol) and 4-(isopropylamino)piperidine-1-carboxylate (1.6g, 6.63mmol) were used. The solution of 2-(1-phenyl-1H-pyrazol-4-yl)thiazole-4-carboxylic acid (1.5g, 5.52mmol), HATU (3.1g, 8.29mmol) and DIPEA (2.1g, 16.58mmol) in in DMF (15mL) was stirred at rt for 15 min prior to the addition of 4-(isopropylamino)piperidine-1-carboxylate (1.6g, 6.63mmol). The reaction mixture was stirred at rt for 4h. The crude material was purified by flash column chromatography (CombiFlash^{®} system; 40-45% ethyl acetate/hexane) to provide tert-butyl 4-(N-isopropyl-2-(1-phenyl-1H-pyrazol-4-yl)thiazole-4-carboxamido)piperidine-1-carboxylate (2.3g, 84% yield) as an off-white solid.
**LCMS [ESI, M+1]:** 440.1[M-56] (RT: 2.400 min, Purity: 95.09%),
**¹H NMR (400 MHz, *d*₆-DMSO)** : δ 9.18 (s, 1H), 8.25 (s, 1H), 7.95 (d, J = 7.8 Hz, 2H), 7.84 (d, J = 9.3 Hz, 1H), 7.55 (t, J = 7.9 Hz, 2H), 7.38 (t, J = 7.4 Hz, 1H), 4.08 - 3.95 (m, 1H), 3.87 (s, 2H), 2.78 (d, J = 25.5 Hz, 2H), 2.72 - 2.64 (m, 2H), 1.89 - 1.58 (m, 2H), 1.48 (d, J = 14.9 Hz, 1H), 1.39 (d, J = 13.9 Hz, 9H), 1.30 - 1.10 (m, 6H).

In **step 2,** 4-(N-isopropyl-2-(1-phenyl-1H-pyrazol-4-yl)thiazole-4-carboxamido)piperidine-1-carboxylate (2.3g, 4.64mmol) in DCM (23mL) was used. The solution was cooled to 0 °C prior to the addition of 4M HCl in Dioxane (11.5mL, 5V). The reaction mixture was stirred for 2h. The crude material was triturated with diethyl ether to provide N-isopropyl-2-(1-phenyl-1H-pyrazol-4-yl)-N-(piperidin-4-yl)thiazole-4-carboxamide hydrochloride (Compound 163: 2.0g, 100% yield) as a white solid.
**LCMS [ESI, M+1]:** 396.2 (RT: 1.399 min, Purity: 95.09%),
**HPLC Purity:** RT: 4.799 min, Purity: 95.39%,
**¹H NMR (400 MHz, D₂O)** : δ 8.55 (s, 1H), 8.09 (s, 1H), 7.58 (d, J = 7.1 Hz, 3H), 7.45 (t, J = 7.7 Hz, 2H), 7.34 (t, J = 7.5 Hz, 1H), 3.97 (m, 1H), 3.57 (m, 1H), 3.47 (d, J = 11.5 Hz, 2H), 3.05 (d, J = 11.9 Hz, 1H), 2.87 (d, J = 10.9 Hz, 2H), 1.92 (d, J = 12.5 Hz, 2H), 1.41 (s, 1H), 1.16 (d, J = 6.2 Hz, 6H).

### 4. Synthesis of N-(1-ethylpiperidin-4-yl)-2-(1-phenyl-1H-pyrazol-4-yl)thiazole-4-carboxamide (Compound 185):

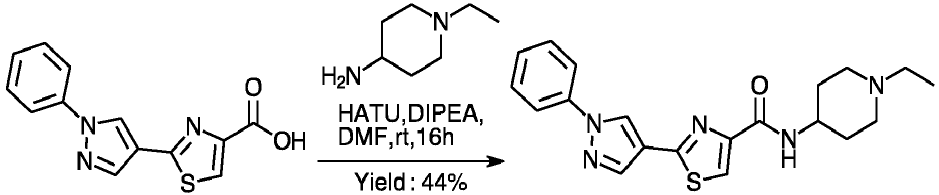

Prepared by general procedure 1 using 2-(1-phenyl-1H-pyrazol-4-yl)thiazole-4-carboxylic acid (0.050g, 0.184mmol) HATU (0.105g, 0.276mmol) and DIPEA (0.1 mL, 0.552mmol) in DMF (1.8mL, 36V), stirred for 20 min prior to the addition of 1-ethylpiperidin-4-amine (0.023g, 0.184mmol). The reaction mixture was stirred at rt for 16h. The crude material was purified by flash column chromatography (CombiFlash^{®} system; 80% ethyl acetate/hexane) to provide N-(1-ethylpiperidin-4-yl)-2-(1-phenyl-1H-pyrazol-4-yl)thiazole-4-carboxamide (Compound 185: 0.031g, 44.% yield) as a white solid.
**LCMS [ESI, M+1]:** 382.1 (RT: 1.29 min, Purity: 99.52%),
**HPLC:** RT: 7.137 min, Purity: 99.29%
**¹H NMR (400 MHz, *d*₆-DMSO):** δ 9.14 (s, 1H), 8.89 (s, 1H), 8.21 (d, *J* = 35.6 Hz, 2H), 7.86 (d*, J* = 7.9 Hz, 2H), 7.47 (t, *J* = 7.6 Hz, 2H), 7.31 (d, *J* = 7.2 Hz, 1H), 4.06 (m, 1H), 3.51 (d, *J* = 46.9 Hz, 2H), 3.01 (m, 4H), 1.95 (m, 2H), 1.80 (m, 2H), 1.14 (t, *J* = 6.4 Hz, 3H).

### 5. Synthesis of (S)-(3-methylpiperazin-1-yl)(2-(1-phenyl-1H-pyrazol-4-yl)thiazol-4-yl)methanone hydrochloride (Compound 157)

### Overall synthetic scheme

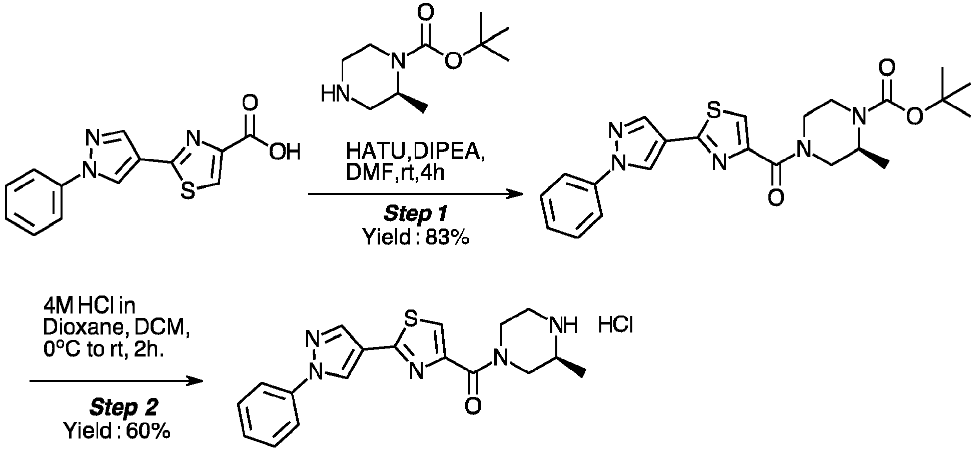

### Synthetic procedure

In **step 1** 2-(1-phenyl-1H-pyrazol-4-yl)thiazole-4-carboxylic acid (0.050g, 0.18mmol) and (S)-2-methylpiperazine-1-carboxylate (0.039g, 0.18mmol) were used. The solution of -(1-phenyl-1H-pyrazol-4-yl)thiazole-4-carboxylic acid (0.050g, 0.18mmol), HATU (0.105g, 0.27mmol) and DIPEA (0.071g, 0.55mmol) in in DMF (0.5mL) was stirred at rt for 15 min prior to the addition of (S)-2-methylpiperazine-1-carboxylate (0.039g, 0.18mmol). The reaction mixture was stirred at rt for 4h. The crude material was purified by flash column chromatography (CombiFlash^{®} system; 0-45% ethyl acetate/hexane) to provide tert-butyl (S)-2-methyl-4-(2-(1-phenyl-1H-pyrazol-4-yl)thiazole-4-carbonyl)piperazine-1-carboxylate (0.069g, 83% yield) as an off-white solid.
**LCMS [ESI, M+1]:** 398.1[M-56] (RT: 2.232 min, Purity: 97.00%),
**¹H NMR (400 MHz, *d*₆-DMSO)** : 9.23 (s, 1H), 8.32 (s, 1H), 8.08 (d, J = 40.6 Hz, 1H), 7.98 (d, J = 7.9 Hz, 2H), 7.58 (t, J = 7.8 Hz, 2H), 7.41 (t, J = 7.3 Hz, 1H), 4.45 - 4.15 (m, 4H), 3.82 (s, 1H), 3.14 (d, J = 28.7 Hz, 2H), 1.44 (s, 9H), 1.12 (s, 3H).

In **step 2** tert-butyl (S)-2-methyl-4-(2-(1-phenyl-1H-pyrazol-4-yl)thiazole-4-carbonyl)piperazine-1-carboxylate (0.07g, 0.20mmol) in DCM (0.7mL) was used. The solution was cooled to 0°C prior to the addition of 4M HCl in Dioxane (0.7mL, 5V). The reaction mixture was stirred at rt for 2h. The crude material was triturated with diethyl ether to provide (S)-(3-methylpiperazin-1-yl)(2-(I-phenyl-1H-pyrazol-4-yl)thiazol-4-yl)methanone hydrochloride **(Compound 157:** 0.032g, 60% yield) as an off-white solid.
**LCMS [ESI, M+1]:** 354.1 (RT: 1.497 min, Purity: 98.55%),
**HPLC Purity:** RT: 4.373 min, Purity: 99.24%,
**¹H NMR (400 MHz, *d*₆-DMSO)** : δ 9.22 (s, 1H), 8.30 (s, 1H), 8.17 (s, 1H), 7.94 (d, J = 8.0 Hz, 2H), 7.52 (dd, J = 37.0, 29.2 Hz, 2H), 7.38 (t, J = 7.6 Hz, 1H), 4.47 (d, J = 11.6 Hz, 2H), 3.38 (d, J = 6.9 Hz, 3H), 3.14 (d, J = 21.2 Hz, 2H), 1.28 (d, J = 16.8 Hz,3H).

### 6. Synthesis of (R)-5-(1-phenyl-1H-pyrazol-4-yl)-N-propyl-N-(pyrrolidin-3-yl)-1H-pyrrole-2-carboxamide hydrochloride (Compound 94)

### Overall synthetic scheme

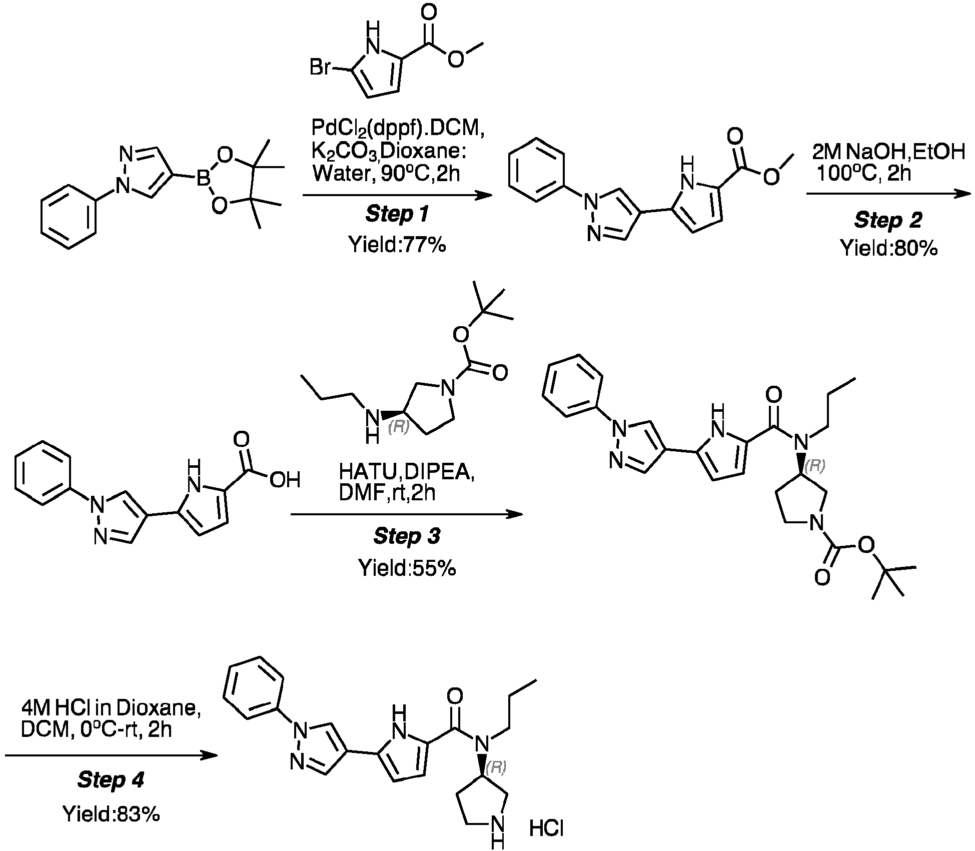

Synthetic procedure - Prepared by general experimental procedure 3.

In **step 1,** 1-phenyl-4-(4,4,5,5-tetramethyl-1,3,2-dioxaborolan-2-yl)-1H-pyrazole (3.1g, 11.81mmol) and K₂CO₃ (3.39g, 24.5mmol) in water (20mL) were added to a stirred solution of methyl 5-bromo-1H-pyrrole-2-carboxylate (2.0g, 9.80mmol) in dioxane (15mL) at room temperature. PdCl₂(dppf)DCM complex (0.8g, 0.98mmol) was used as the palladium catalyst. The reaction mixture was stirred at 90°C for 2h. The crude material was purified by flash column chromatography (CombiFlash^{®} system; 20-30% ethyl acetate/hexane] to provide methyl 5-(1-phenyl-1H-pyrazol-4-yl)-1H-pyrrole-2-carboxylate (2.0g, 77%) as a beige solid.
**LCMS [ESI, M+1]:** 267.9 (RT: 1.896 min, Purity: 83.25%),

In **step 2** 2M NaOH (20mL) was added to a stirred solution of methyl 5-(1-phenyl-1H-pyrazol-4-yl)-1H-pyrrole-2-carboxylate (2.0g, 7.48mmol) in EtOH (20mL) at room temperature. The reaction mixture was heated at 100 °C for 2h. The following purification procedure was used: after completion of reaction, the reaction mixture was concentrated under reduced pressure. The residue was dissolved in water (50mL) and extracted with diethyl ether (3 X 100mL). The aqueous layer was slowly acidified with a saturated solution of citric acid up to pH ~4-5. A solid was precipitate out, which was isolated by filtration and dried under reduced pressure to provide 5-(1-phenyl-1H-pyrazol-4-yl)-1H-pyrrole-2-carboxylic acid (1.5g, 80%) as an off-white solid.
**LCMS [ESI, M-18]:** 254.2 (RT: 1.641 min, Purity: 88.07%),
**HPLC Purity:** RT: 6.453 min, Purity: 88.13%
¹**H NMR (400 MHz, *d*₆-DMSO):** δ 12.25 (s, 1H), 11.93 (d, J = 8.0 Hz, 1H), 8.95 (s, 1H), 8.25 (s, 1H), 7.80 (d, J = 7.7 Hz, 2H), 7.54 (t, J = 8.0 Hz, 2H), 7.34 (t, J = 7.4 Hz, 1H), 6.79 (dd, J = 3.6, 2.4 Hz, 1H), 6.46 (dd, J = 3.6, 2.5 Hz, 1H).

In **step 3** the mixture of 5-(1-phenyl-1H-pyrazol-4-yl)-1H-pyrrole-2-carboxylic acid (1.0g, 3.95mmol), HATU (2.25g, 5.92mmol) and DIPEA (1.55g, 11.85mmol) in DMF (15mL) was stirred at rt for 30 min. After addition of tert-butyl *(R)*-3-(propylamino)pyrrolidine-1-carboxylate (0.90g, 3.95mmol) the reaction mixture was stirred at rt for 2h. The crude material was purified by flash column chromatography (CombiFlash^{®} system; 0-10% ethyl acetate/hexane) to provide tert-butyl *(R)*-3-(5-(1-phenyl-1H-pyrazol-4-yl)-*N*-propyl-1*H*-pyrrole-2-carboxamido)pyrrolidine-1-carboxylate (1.0g, 55 %) as a pale yellow oil.
**LCMS [ESI, M-56]:** 408.1 (RT: 2.369 min, Purity: 94.70%).

In **step 4** tert-butyl *(R)*-3-(5-(1-phenyl-1*H*-pyrazol-4-yl)-*N*-propyl-1*H*-pyrrole-2-carboxamido) pyrrolidine-1-carboxylate (1.0g, 2.15mmol) was used. The reaction mixture was stirred at rt for 3h. The crude material, was triturated with diethyl ether to provide *(R)-*5-(1-phenyl-1*H*-pyrazol-4-yl)-*N-*propyl-*N*-(pyrrolidin-3-yl)-1*H*-pyrrole-2-carboxamide hydrochloride (**Compound 94:** 0.650g, 83%) as a light greenish solid.
**LCMS [ESI, M+1]:** 364.4 (RT: 1.460 min, Purity: 98.31%),
**HPLC Purity:** RT: 5.085 min, Purity: 97.74%
**Chiral HPLC Purity:** RT: 11.97 min, Purity: 98.79%
**¹H NMR (400 MHz, D₂O**): δ 8.17 (s, 1H), 7.88 (s, 1H), 7.54 - 7.41 (m, 4H), 7.32 (t, J = 7.3 Hz, 1H), 6.45 (d, J = 3.6 Hz, 1H), 6.27 (d, J = 3.6 Hz, 1H), 4.35 (m, 1H), 3.70 (t, J = 9.0 Hz, 1H), 3.44 (dt, J = 22.0, 13.3 Hz, 4H), 3.19 (dd, J = 20.6, 10.3 Hz, 1H), 2.46 (d, J = 8.8 Hz, 1H), 2.19 (m, 1H), 1.71 - 1.47 (m, 2H), 0.84 (t, J = 7.3 Hz, 3H).

### 7. Synthesis of (2S)-1-[2-(1-phenyl-1H-pyrazol-4-yl)-1,3-thiazole-4-carbonyl]pyrrolidine-2-carboxylic acid (Compound 60)

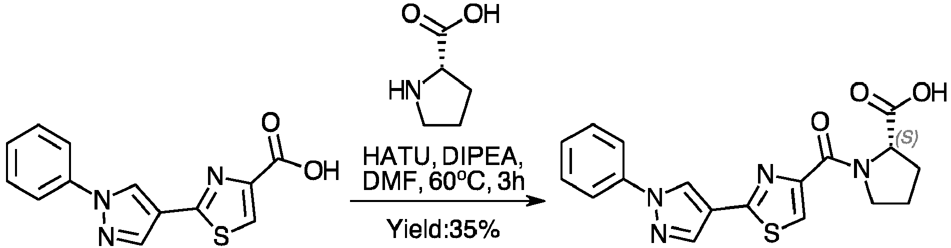

Prepared according to general experimental procedure 1 using L-proline (0.169g, 1.47mmol). The reaction mixture was stirred at 60°C for 3h. A modified purification procedure was used: the crude material was purified by preparative HPLC (0.05% HCl in water/ACN as the mobile phase) to provide (2S)-1-[2-(1-phenyl-1H-pyrazol-4-yl)-1,3-thiazole-4-carbonyl]pyrrolidine-2-carboxylic acid **(Compound 60:** 0.190g, 35% yield) as a white solid.
**LCMS [ESI, M+1]:** 369.0 (RT: 1.617 min, Purity: 99.36%),
**HPLC:** RT: 6.319 min, Purity: 98.81%
**¹H NMR (400 MHz, CD₃OD)**: δ 8.83 (s, 1H), 8.25 (s, 1H), 8.20 (s, 1H), 7.84 (d, *J* = 8.8 Hz, 2H), 7.53 (t, *J* = 8.0 Hz, 2H), 7.40 (dd, *J* = 10.7, 4.2 Hz, 1H), 5.36 (dd, *J* = 8.8, 2.7 Hz, 1H), 3.86 - 3.76 (m, 2H), 2.36-2.26 (m, 2H), 2.14 - 1.92 (m, 2H).

### 8. Synthesis of (R)-2-(1-phenyl-1H-pyrazol-4-yl)-N-propyl-N-(pyrrolidin-3-yl)thiazole-4-carboxamide hydrochloride (Compound 171) and (S)-2-(1-phenyl-1H-pyrazol-4-yl)-N-propyl-N-(pyrrolidin-3-yl)thiazole-4-carboxamide hydrochloride (Compound 170)

### Overall synthetic scheme

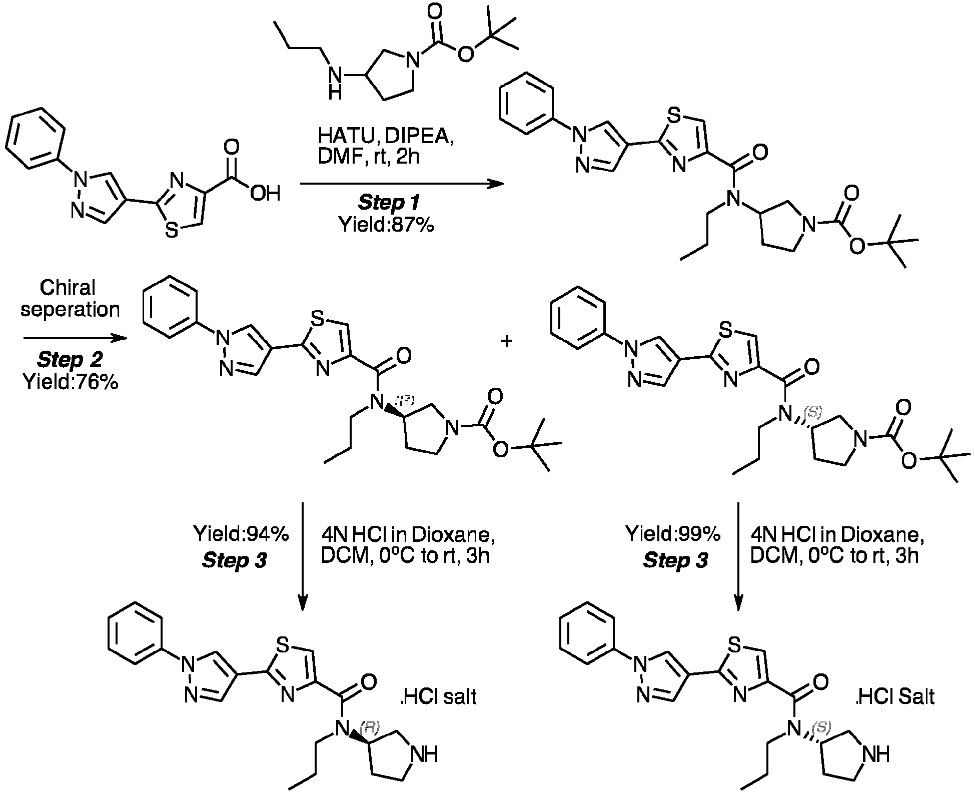

### Step 1 - Synthesis of tert-butyl 3-(2-(1-phenyl-1H-pyrazol-4-yl)-N-propylthiazole-4-carboxamid o)pyrrolidine-1-carboxylate

To a stirred solution of acid (4.0g, 14.74mmol) in DMF (4mL, 10 vol) at rt,. HATU (8.4g, 22.11mmol) and DIPEA (5.7g, 44.23mmol) were added. The reaction mixture was stirred for 5 min. Then tert-butyl 3-(propylamino)pyrrolidine-1-carboxylate (3.36g, 14.74mmol) was added to the reaction mixture at rt. The reaction mixture was stirred at rt for 2h (the progress of the reaction was monitored by TLC and LCMS analysis). After completion of the reaction, the reaction mixture was poured into cold water (80 ml) and extracted with ethyl acetate (3 × 40 ml). The combined organic fractions were washed with cold water 3-4 times, dried over sodium sulphate and concentrated under reduced pressure. The crude material which was purified by column chromatography (0-20% ethyl acetate/Hexane) to provide tert-butyl 3-(2-(1-phenyl-1H-pyrazol-4-yl)-N-propylthiazole-4-carboxamido)pyrrolidine-1-carboxylate(6.2g, 87% yield) as an off-white solid.
**LCMS [ESI, M+1]:** 426.1[M-56] (RT: 2.349 min, Purity: 96.95%),
**Chiral HPLC: R-Isomer-** RT: 6.91min, Purity: 49.30%,
**S-Isomer-** RT: 8.70 min, Purity: 49.21%,
**¹H NMR (400 MHz, *d*₆-DMSO):** δ 9.19 (s, 1H), 8.25 (d, J = 4.5 Hz, 1H), 8.03 (s, 1H), 7.94 (d, J = 7.7 Hz, 2H), 7.54 (t, J = 7.8 Hz, 2H), 7.38 (t, J = 7.4 Hz, 1H), 4.57 (s, 1H), 3.64 (s, 1H), 3.26 (d, J = 11.8 Hz, 3H), 2.07 (s, 2H), 1.56 (s, 3H), 1.39 (s, 9H), 0.88 (s, 2H), 0.78 (d, J = 7.5 Hz, 2H).

### Step 2 - Synthesis of tert-butyl (R)-3-(2-(1-phenyl-1H-pyrazol-4-yl)-N-propylthiazole-4-carboxa mido)pyrrolidine-1-carboxylate and tert-butyl (S)-3-(2-(1-phenyl-1H-pyrazol-4-yl)-N-propylthiazole-4-carboxamido)pyrrolidine-1-carboxylate

Racemic tert-butyl 3-(2-(1-phenyl-1H-pyrazol-4-yl)-N-propylthiazole-4-carboxamido)pyrrolidine -1-carboxylate was purified by chiral SFC purification to isolate tert-butyl(*R*)-3-(2-(1-phenyl-1H-pyrazol-4-yl)-N-propylthiazole-4-carboxamido)pyrrolidine-1-carboxylate (2.4g) and tert-butyl (S)-3-(2-(1-phenyl-1H-pyrazol-4-yl)-N-propylthiazole-4-carboxa mido)pyrrolidine-1-carboxylate (2.3g).

Data for tert-butyl(*R*)-3-(2-(1-phenyl-1H-pyrazol-4-yl)-N-propylthiazole-4-carboxamido)pyrrolidine-1-carboxylate:
**LCMS [ESI, M+1]:** 382.1[M-100] (RT: 2.402min, Purity: 100%),
**HPLC Purity: R-Isomer-** RT: 8.97min, Purity: 100%,
**Chiral HPLC Purity: R-Isomer-**RT:6.87 min, 100%,
**¹H NMR (400 MHz, CD₃OD)** : δ 8.86 (s, 1H), 8.21 (s, 1H), 7.92 (s, 1H), 7.86 (d, J = 7.1 Hz, 2H), 7.54 (t, J = 7.9 Hz, 2H), 7.40 (t, J = 7.4 Hz, 1H), 4.74 (s, 1H), 3.56 (dd, J = 97.5, 62.6 Hz, 6H), 2.22 (s, 2H), 1.86 - 1.66 (m, 2H), 1.48 (s, 9H), 0.96 (s, 3H).

Data for tert-butyl (*S*)-3-(2-(1-phenyl-1H-pyrazol-4-yl)-N-propyl thiazole-4-carboxa mido)pyrrolidine-1-carboxylate:
**LCMS [ESI, M+1]:** 425.1[M-56] (RT: 2.402 min, Purity: 100%),
**HPLC Purity: S-Isomer-** RT: 8.974min, Purity: 98.02%,
**Chiral HPLC Purity: S-Isomer-**RT:8.69 min, 97.46%,
**¹H NMR (400 MHz, CD₃OD)** : δ 8.86 (s, 1H), 8.21 (s, 1H), 7.91 (d, J = 7.5 Hz, 1H), 7.86 (d, J = 7.2 Hz, 2H), 7.54 (t, J = 7.9 Hz, 2H), 7.40 (t, J = 7.3 Hz, 1H), 4.74 (s, 1H), 3.57 (dd, J = 88.5, 57.6 Hz, 6H), 2.22 (s, 2H), 1.74 (d, J = 7.1 Hz, 2H), 1.48 (s, 9H), 0.95 (s, 3H).

### Step 3a - Synthesis of (R)-2-(1-phenyl-1H-pyrazol-4-yl)N-propyl-N-(pyrrolidin-3-yl)thiazole-4-carbox amide hydrochloride (Compound 171)

To a stirred solution of tert-butyl (R)-3-(2-(1-phenyl-1H-pyrazol-4-yl)-N-propylthiazole-4-carboxamido)pyrrolidine-1-carboxylate (2.4 g, 4.98mmol) in DCM (24 ml, 10 vol) at 0°C, 4N HCl in Dioxane (4.8 ml, 2 vol) was added to the reaction mixture. The reaction mixture was stirred at rt for 3h (the progress of the reaction was monitored by TLC and LCMS analysis). After completion of the reaction, the reaction mixture was concentrated under reduced pressure to provide crude material which was purified by trituration with diethyl ether to provide (*R*)-2-(1-phenyl-1H-pyrazol-4-yl)-N-propyl-N-(pyrrolidin-3-yl)thiazole-4-carboxamide hydrochloride (**Compound 171:** 1.8g, 94% yield) as an off-white solid.
**LCMS [ESI, M+1]:** 382.1 (RT: 1.417min, Purity: 99.86%),
**HPLC:** RT: 4.726 min, Purity: 99.77%,
**Chiral HPLC:** RT: 6.00min, Purity: 100%,
**¹H NMR (400 MHz, CD₃OD)** : δ 8.88 (s, 1H), 8.20 (s, 1H), 8.02 (s, 1H), 7.86 (d, J = 8.0 Hz, 2H), 7.55 (t, J = 7.9 Hz, 2H), 7.41 (t, J = 7.4 Hz, 1H), 4.36 (s, 1H), 3.77 (d, J = 25.4 Hz, 1H), 3.67 (s, 4H), 3.57 - 3.44 (m, 1H), 3.29 - 3.19 (m, 1H), 2.49 (d, J = 57.8 Hz, 2H), 1.82 (dd, J = 14.8, 7.3 Hz, 2H), 0.93 (t, J = 6.4 Hz, 3H).

### Step 3b -_Synthesis of (S)-2-(1-phenyl-1H-pyrazol-4-yl)-N-propyl-N-(pyrrolidin-3-yl)thiazole-4-carboxamide hydrochloride (Compound 170)

To a stirred solution of tert-butyl (R)-3-(2-(1-phenyl-1H-pyrazol-4-yl)-N-propylthiazole-4-carboxamido)pyrrolidine-1-carboxylate (2.3g, 4.77mmol) in DCM (23ml, 10 vol) at 0°C, 4N HCl in dioxane (4.6ml, 2 vol) was added. The reaction mixture was stirred at rt for 3h (the progress of the reaction was monitored by TLC and LCMS analysis). After completion of the reaction, the reaction mixture was concentrated under reduced pressure to provide crude material which was purified by trituration with diethyl ether to provide (*S*)-2-(1-phenyl-1H-pyrazol-4-yl)-N-propyl-N-(pyrrolidin-3-yl)thiazole-4-carboxamide hydrochloride (**Compound 170:** 1.8g, 99% yield) as an off-white solid.
**LCMS [ESI, M+1]:** 382.1 (RT: 1.421 min, Purity: 98.92%),
**HPLC:** RT: 4.720 min, Purity: 98.21%,
**Chiral HPLC:** RT: 6.80min, Purity: 99.45%,
**¹H NMR (400 MHz, CD₃OD)** : δ 8.87 (s, 1H), 8.20 (s, 1H), 8.02 (s, 1H), 7.86 (d, J = 7.8 Hz, 2H), 7.55 (t, J = 7.9 Hz, 2H), 7.41 (t, J = 7.4 Hz, 1H), 4.36 (s, 1H), 3.77 (d, J = 24.0 Hz, 1H), 3.67 (s, 3H), 3.59 - 3.46 (m, 1H), 3.28 - 3.19 (m, 1H), 2.56 (s, 1H), 2.42 (s, 1H), 1.82 (dd, J = 14.9, 7.4 Hz, 2H), 0.93 (s, 3H).

### 9. Synthesis of N-isopropyl-N-(piperidin-4-yl)-2-(1-(2-(trifluoromethyl)phenyl)-1H-pyrazol -4-yl)thiazole -4-carboxamide hydrochloride (Compound 47)

### Overall synthetic scheme

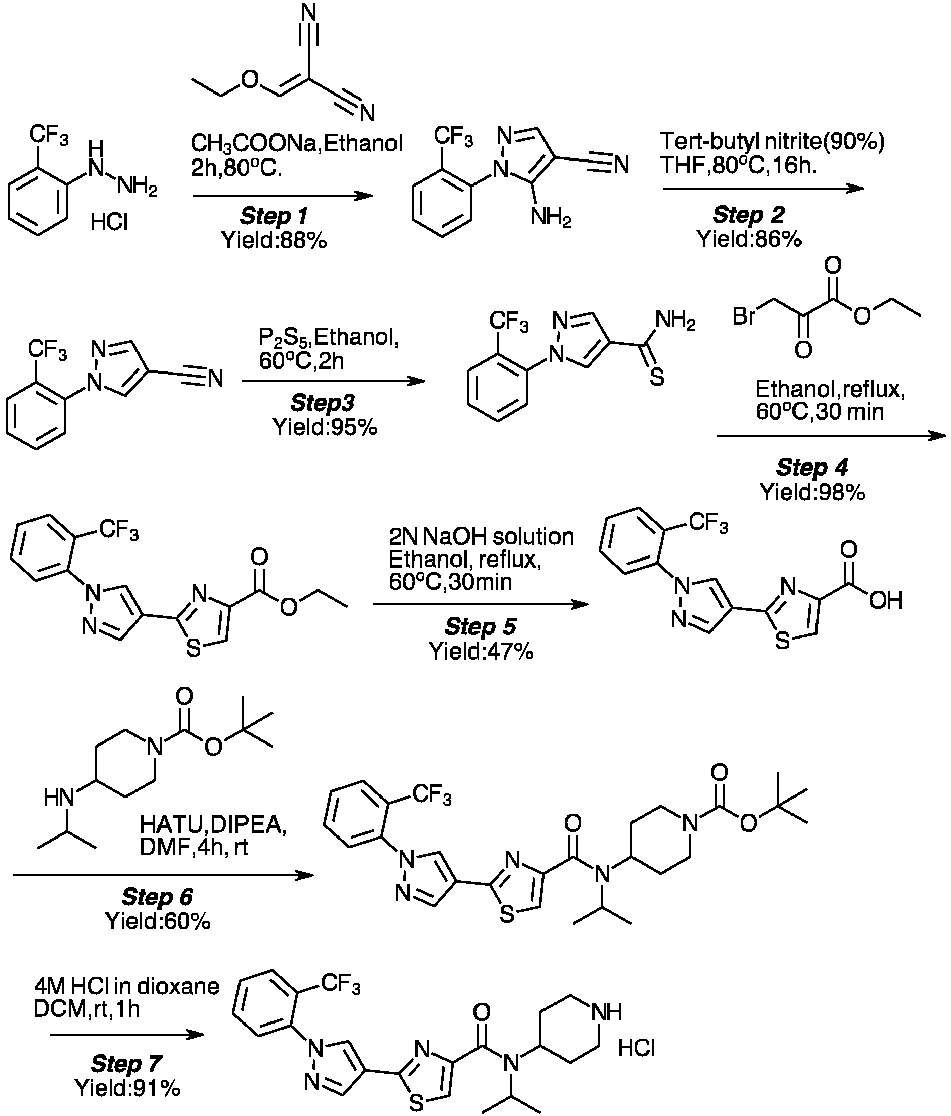

### Step-1: Synthesis of 5-amino-1-(2-(trifluoromethyl)phenyl)-1H-pyrazole-4-carbonitrile:

To a stirred solution of (2-(trifluoromethyl)phenyl)hydrazine hydrochloride (4.4 g, 20.69mmol) in ethanol (50mL) at rt, sodium acetate (3.39 g, 41.39mmol) and 2-(ethoxymethylene)malononitrile (2.52 g, 20.69mmol) were added. The reaction mixture was refluxed at 80 °C for 2h (the progress of reaction was monitored by TLC and LCMS analysis). After completion of the reaction, the reaction mixture was concentrated under reduced pressure. The residual material was washed with cold water (50 mL) and extracted with ethyl acetate (3 × 100 mL). The combined organic fractions were dried over anhydrous sodium sulfate and concentrated under reduced pressure to provide 5-amino-1-(2-(trifluoromethyl)phenyl)-1H-pyrazole-4-carbonitrile (5.4 g, 88% yield) as a yellow solid.
**LCMS [ESI, M+1]:** 253.30 (RT: 1.454min, Purity: 73.75%)
¹**H NMR (400 MHz, *d*₆-DMSO):** δ 7.85 (d, J = 7.7 Hz, 1H), 7.81 - 7.72 (m, 1H), 7.72 - 7.64 (m, 2H), 7.46 (d, J = 7.7 Hz, 1H), 6.59 (s, 2H).

### Step-2: Synthesis of 1-(2-(trifluoromethyl)phenyl)-1H-pyrazole-4-carbonitrile:

To a stirred solution of 5-amino-1-(2-(trifluoromethyl)phenyl)-1H-pyrazole-4-carbonitrile (4.6 g,18.24 mmol) in THF (50 mL) at rt, tert-butyl nitrite(90%)(4.13g,36.49mmol) was added. The reaction mixture was refluxed at 80 °C for 16 h (the progress of the reaction was monitored by TLC and LCMS analysis). After completion of reaction, the reaction mixture was concentrated under reduced pressure. The residual material was purified by flash column chromatography (CombiFlash^{®} system; 0-5% ethyl acetate in hexane) to provide 1-(2-(trifluoromethyl)phenyl)-1H-pyrazole-4-carbonitrile (3.7 g, 86% yield) as a yellow solid.
**LCMS [ESI, M-1]:**236.2(RT: 1.826min, Purity: 89.90%)
**¹H NMR (400 MHz, *d*₆-DMSO):** δ 8.93 (s, 1H), 8.30 (s, 1H), 7.92 (d, J = 7.8 Hz, 1H), 7.83 (t, J = 7.4 Hz, 1H), 7.74 (t, J = 7.7 Hz, 1H), 7.65 (d, J = 7.8 Hz, 1H).

### Step-3: Synthesis of 1-(2-(trifluoromethyl)phenyl)-1H-pyrazole-4-carbothioamide

To a stirred solution of (1-(2-(trifluoromethyl)phenyl)-1H-pyrazole-4-carbonitrile (3.7 g,15.5 mmol) in ethanol (37 mL) at rt, phosphorus pentasulfide (6.93 g, 31.1 mmol) was added. The reaction mixture was refluxed at 60 °C for 2 h (the progress of the reaction was monitored by TLC and LCMS analysis). After completion of the reaction, the reaction mixture was distilled under vacuum. The crude reaction mixture was washed with sodium bicarbonate solution (100 mL) until neutralization of pH and extracted with ethyl acetate (3 × 150 mL). The combined organic fractions were dried over sodium sulfate, concentrated under reduced pressure and triturated with diethyl ether to afford 1-(2-(trifluoromethyl)phenyl)-1H-pyrazole-4-carbothioamide (4.0g, 95% yield) as a yellow solid.
**LCMS [ESI, M+1]:** 272.29(RT: 1.606min, Purity: 80.03%)
**¹H NMR (400 MHz, *d*₆-DMSO):** δ 9.45 (s, 1H), 9.21 (s, 1H), 8.41 (s, 1H), 8.10 (s, 1H), 7.90 (t, J = 8.3 Hz, 1H), 7.79 (t, J = 7.7 Hz, 1H), 7.69 (t, J = 7.8 Hz, 1H), 7.65 - 7.57 (m, 1H).

### Step-4: Synthesis of ethyl 2-(1-(2-(trifluoromethyl)phenyl)-1H-pyrazol-4-yl)thiazole-4-carboxylate:

To a stirred solution of 1-(2-(trifluoromethyl)phenyl)-1H-pyrazole-4-carbothioamide (4.0 g, 14.70 mmol) in ethanol (50 mL) at rt, ethyl 3-bromo-2-oxopropanoate (3.14 g, 16.17 mmol) was added. The reaction mixture was refluxed at 60°C for 30 min (the progress of the reaction was monitored by TLC and LCMS analysis). After completion of the reaction, the reaction mixture was distilled under vacuum and triturated with diethyl ether to provide ethyl 2-(1-(2-(trifluoromethyl)phenyl)-1H-pyrazol-4-yl)thiazole-4-carboxylate (5.3 g, 98% yield) as a yellow solid.
**LCMS [ESI, M+1]:** 367.9(RT: 2.015min, Purity: 72.00%)
**¹H NMR (400 MHz, *d*₆-DMSO):** δ 8.75 (s, 1H), 8.39 (d, J = 13.9 Hz, 1H), 8.23 (s, 1H), 7.91 (d, J = 7.8 Hz, 1H), 7.85 - 7.75 (m, 1H), 7.72 (d, J = 7.3 Hz, 1H), 7.67 (d, J = 7.8 Hz, 1H), 4.24 (dd, J = 14.2, 7.1 Hz, 2H), 1.28 - 1.19 (t, J = 7.2 Hz, 3H).

### Step-5: Synthesis of 2-(1-(2-(trifluoromethyl)phenyl)-1H-pyrazol-4-yl)thiazole-4-carboxylic acid:

To a stirred solution of ethyl 2-(1-(2-(trifluoromethyl)phenyl)-1H-pyrazol-4-yl)thiazole-4-carboxylate (5.3 g, 14.4 mmol) in ethanol (60 mL) at rt, 2 N NaOH solution (21.62 mL) was added. The reaction mixture was heated at 60 °C for 30 minutes (the progress of the reaction was monitored by TLC and LCMS analysis). After completion of the reaction, the reaction mixture was poured into water (50 mL) and extracted with ethyl acetate (3 × 50 mL). The aqueous layer was separated out and acidified with 2 M HCl solution to generated a precipitate, which was isolated by filtration and dried under reduced pressure to provide 2-(1-(2-(trifluoromethyl)phenyl)-1H-pyrazol-4-yl)thiazole-4-carboxylic acid (2.3 g, 47% yield) as a brown solid.
**LCMS [ESI, M+1]:** 339.85 (RT: 1.613min, Purity: 99.13%)
**HPLC:** 6.280min, 99.63%
**¹H NMR (400 MHz, *d*₆-DMSO):** δ 13.01 (s, 1H), 8.69 (d, J = 21.4 Hz, 1H), 8.44 - 8.28 (m, 1H), 8.18 (d, J = 21.6 Hz, 1H), 7.91 (d, J = 7.7 Hz, 1H), 7.81 (t, J = 7.5 Hz, 1H), 7.67 (dt, J = 30.1, 15.0 Hz, 2H).

### Step-6: Synthesis of tert-butyl-4-(N-isopropyl-2-(1-(2-(trifluoromethyl)phenyl)-1H-pyrazol-4-yl) thiazole-4-carboxamido)piperidine-1 -carboxylate:

To a stirred solution of 2-(1-(2-(trifluoromethyl)phenyl)-1H-pyrazol-4-yl)thiazole-4-carboxylic acid (0.20 g, 0.58 mmol) in DMF (4 mL) at rt, HATU (0.33g, 0.87 mmol) and DIPEA (0.21 g, 1.7 mmol) were added. The reaction mixture was stirred for 30 min and then tert-butyl 4-(isopropylamino)piperidine-1-carboxylate (0.142g, 0.58mmol) was added at rt. The reaction mixture was stirred at rt for 4h (the progress of the reaction was monitored by TLC and LCMS analysis). After completion of the reaction, the reaction mixture was poured into cold water (15 mL) and extracted with ethyl acetate (3 × 50 mL). The combined organic fractions were washed with cold water 3-4 times, dried over sodium sulfate and concentrated under reduced pressure. The crude material was purified by flash column chromatography (CombiFlash^{®} system; 18-22% ethyl acetate in Hexane) to provie tert-butyl4-(N-isopropyl-2-(1-(2-(trifluoromethyl)phenyl)-1H-pyrazol-4-yl)thiazole-4-carboxamido)piperidine-1-carboxylate (0.2g,60% yield) as a white solid.
**LCMS [ESI, M-56]:** 507.98(RT: 2.388min, Purity: 97.26%)

### Step-7: Synthesis of N-isopropyl-N-(piperidin-4-yl)-2-(1-(2-(trifluoromethyl)phenyl)-1H-pyrazol -4-yl)thiazole -4-carboxamide hydrochloride (Compound 47)

To a stirred solution of tert-butyl4-(N-isopropyl-2-(1-(2-(trifluoromethyl)phenyl)-1H-pyrazol-4-yl)thiazole-4-carboxamid o)piperidine-1-carboxylate (0.20 g, 0.35 mmol) in DCM (2 mL, 10V) at 0°C, 4M HCl in Dioxane (1mL, 5V) was added. The reaction mixture was stirred at room temperature for 1h (the progress of the reaction was monitored by TLC and LCMS analysis). After completion of reaction, the reaction mixture was concentrated under reduced pressure, and the crude material triturated with diethyl ether to provide (N-isopropyl-N-(piperidin-4-yl)-2-(1-(2-(trifluoromethyl)phenyl)-1H-pyrazol-4-yl)thiazole-4-carboxamide hydro chloride (**Compound 47:** 0.150g, 91% yield), as an off-white sticky solid.
**LCMS [ESI, M+1]:** 464.07(RT: 1.411min, Purity: 98.37%)
**HPLC:** RT: 4.758min, Purity: 97.90%
**¹H NMR (400 MHz, CD₃OD):** δ 8.53 (s, 1H), 8.25 (s, 1H), 7.96 (d, J = 7.8 Hz, 1H), 7.91 - 7.73 (m, 3H), 7.68 (d, J = 7.8 Hz, 1H), 4.26 (s, 1H), 3.66 (d, J = 17.7 Hz, 1H), 3.51 (d, J = 10.5 Hz, 2H), 3.24 - 2.99 (m, 3H), 1.96 (s, 2H), 1.55 (s, 1H), 1.25 (d, J = 74.4 Hz, 6H).

**Table 1: Compounds 1-210 with chemical name, LCMS data and experimental procedure**

| **Comp. No.** | **Chemical Name** | **MW** | **Observed MW in LCMS** | **Salt Form** | **Exp. Proc. No.** |
|---|---|---|---|---|---|
| 1 | N-(1-ethylpiperidin-4-yl)-N- methyl-5-(1-phenyl-1H-pyrazol-4-yl)-1H-pyrrole-2-carboxamide | 377 | 378 | Free base | 3 |
| 2 | (3S)-3-methyl-1-[4-(5-phenyl- 1,3,4-thiadiazol-2-yl)thiophene-2-carbonyl]piperazine | 370 | 371 | HCl salt | 3 |
| 3 | (3S)-3-methyl-1-[4-(1-phenyl- 1H-pyrazol-4-yl)-1H-pyrrole-2-carbonyl]piperazine | 335 | 336 | HCl salt | 3 |
| 4 | (3S)-3-methyl-1-[5-(1-phenyl-1H-pyrazol-4-yl)thiophene-3 - carbonyl]piperazine | 352 | 353 | HCl salt | 3 |
| 5 | 5-(1-phenyl-1H-pyrazol-4-yl)-N-(piperidin-4-yl)-N-(propan-2-yl)-1H-pyrrole-2-carboxamide | 377 | 378 | HCl salt | 3 |
| 6 | 2-[1-(4-fluorophenyl)-1H-pyrazol-4-yl]-N-(piperidin-4-yl)-N-(propan-2-yl)- 1, 3 -thiazole-4-carboxamide | 413 | 414 | HCl salt | |
| 7 | (3S)-3-methyl-1-[2-(5-phenylthiophen-2-yl)-1,3- thiazole-4-carbonyl]piperazine | 369 | 370 | HCl salt | 3 |
| 8 | N-propyl-2-[1-(pyridin-3-yl)-1H-pyrazol-4-yl]-N-[(3R)-pyrrolidin-3-yl]-1,3-thiazole-4- carboxamide | 382 | 383 | HCl salt | 4 |
| 9 | N-propyl-2-[1-(pyridin-4-yl)-1H-pyrazol-4-yl]-N-[(3 S)-pyrrolidin-3-yl]-1,3-thiazole-4- carboxamide | 382 | 383 | HCl salt | 4 |
| 10 | N-methyl-N-(oxan-4-yl)-5-(1-phenyl-1H-pyrazol-4-yl)furan-2-carboxamide | 351 | 352 | Free base | 3 |
| 11 | 1-[(2R)-1-[5-(1-phenyl-1H-pyrazol-4-yl)furan-2-carbonyl]pyrrolidin-2-yl]methanamine | 336 | 337 | HCl salt | 3 |
| 12 | N-(2-aminoethyl)-2-(2-phenyl-1,3-oxazol-5-yl)-1,3-thiazole-4-carboxamide | 314 | 315 | HCl Salt | 3 |
| 13 | (3 S)-3 -methyl- 1 -[2-(2-phenyl-1,3-oxazol-5 -yl)- 1, 3 -thiazole-4-carbonyl]piperazine | 354 | 355 | HCl Salt | 3 |
| 14 | 1-[(2R)-1-[2-(2-phenyl-1,3-oxazol-5-yl)-1, 3 -thiazole-4-carbonyl]pyrrolidin-2- yl]methanamine | 354 | 355 | HCl Salt | 3 |
| 15 | N-methyl-N-(oxan-4-yl)-4-(1- phenyl-1H-pyrazol-4-yl)thiophene- 2-carboxamide | 367 | 368 | Free base | 3 |
| 16 | 4-(1-phenyl-1H-pyrazol-4-yl)-N-{[(3R)-pyrrolidin-3-yl]methyl}thiophene-2- carboxamide | 352 | 353 | HCl Salt | 3 |
| 17 | N-(5-aminopentyl)-4-(1-phenyl-1H-pyrazol-4-yl)thiophene-2-carboxamide | 354 | 355 | HCl Salt | 3 |
| 18 | N-(2-aminoethyl)-4-(1-phenyl-1H - pyrazol-4- yl)thiophene- 2-carboxamide | 312 | 313 | HCl Salt | 3 |
| 19 | N-methyl-N-(oxan-4-yl)-5-(1- phenyl-1H-pyrazol-4-yl)-1H-pyrrole-2-carboxamide | 350 | 351 | Free base | 3 |
| 20 | N-(2-aminoethyl)-5-(1-phenyl- 1H-pyrazol-4-yl)-1H-pyrrole-2-carboxamide | 295 | 296 | HCL salt | 3 |
| 21 | N-(5-aminopentyl)-5-(1-phenyl- 1H-pyrazol-4-yl)-1H-pyrrole-2-carboxamide | 337 | 338 | HCL salt | 3 |
| 22 | N-propyl-2-[1-(pyridin-3-yl)-1H-pyrazol-4-yl]-N-[(3S)-pyrrolidin-3-yl]-1,3-thiazole-4- carboxamide | 382 | 383 | HCl salt | 4 |
| 23 | N-propyl-2-[1-(pyridin-4-yl)-1H-pyrazol-4-yl]-N-[(3R)-pyrrolidin- 3-yl]-1,3-thiazole-4- carboxamide | 382 | 383 | HCl salt | 4 |
| 24 | (3S)-3-methyl-1-(2-{1-[3-(trifluoromethoxy)phenyl]-1H- pyrazol-4-yl}-1,3-thiazole-4-carbonyl)piperazine | 437 | 438 | HCl salt | 4 |
| 25 | N-propyl-N-[(3S)-pyrrolidin-3- yl]-2-{ 1-[3-(trifluoromethoxy)phenyl]-1H-pyrazol-4-yl}-1,3-thiazole-4-carboxamide | 465 | 1.71 | HCl salt | 4 |
| 26 | N-propyl-N-[(3R)-pyrrolidin-3- yl]-2-{ 1-[3-(trifluoromethoxy)phenyl]-1H- pyrazol-4-yl}-1,3-thiazole-4-carboxamide | 465 | 1.72 | HCl salt | 4 |
| 27 | (3S)-3-methyl-1-{2-[1-(3-methylphenyl)-1H-pyrazol-4-yl]- 1,3-thiazole-4- carbonyl}piperazine | 367 | 368 | HCl salt | 4 |
| 28 | 2-[1-(3-methylphenyl)-1H-pyrazol-4-yl]-N-propyl-N-[(3 S)-pyrrolidin-3 -yl] -1,3 -thiazole-4-carboxamide | 395 | 396 | HCl Salt | 4 |
| 29 | 2-[1-(3-methylphenyl)-1H-pyrazol-4-yl]-N-propyl-N-[(3R)-pyrrolidin-3-yl]-1,3-thiazole-4-carboxamide | 395 | 396 | HCl Salt | 4 |
| 30 | (3S)-1-{2-[1-(3-chlorophenyl)-1H-pyrazol-4-yl] -1,3 -thiazole-4-carbonyl}-3-methylpiperazine | 387 | 388 | HCl salt | 4 |
| 31 | 2-[1-(3-chlorophenyl)-1H-pyrazol-4-yl]-N-propyl-N-[(3 S)-pyrrolidin-3 -yl] -1,3 -thiazole-4-carboxamide | 415 | 416 | HCl salt | 4 |
| 32 | pyrazol-4-yl]-N-propyl-N-[(3R)-pyrrolidin-3 -yl] -1,3 -thiazole-4-carboxamide | 415 | 416 | HCl salt | 4 |
| 33 | (3S)-3-methyl-1-{2-[1-(pyridin-2-yl)-1H-pyrazol-4-yl]-1,3- thiazole-4-carbonyl }piperazine | 354 | 355 | HCl salt | 4 |
| 34 | (3S)-1-{2-[1-(4-fluorophenyl)-1H-pyrazol-4-yl]-1,3-thiazole-4-carbonyl}-3-methylpiperazine | 371 | 372 | HCl salt | 4 |
| 35 | 2-(2-phenyl-1,3-oxazol-5-yl)-N-propyl-N-[(3S)-pyrrolidin-3-yl]- 1,3-thiazole-4-carboxamide | 382 | 383 | HCl salt | 3 |
| 36 | 5-(1-phenyl-1H-pyrazol-4-yl)-N-propyl-N-[(3R)-pyrrolidin-3-yl]thiophene- 3 -carboxamide | 380 | 381 | HCl Salt | 3 |
| 37 | (3S)-3-methyl-1-(2-{1-[4-(trifluoromethoxy)phenyl]-1H- pyrazol-4-yl}-1,3-thiazole-4-carbonyl)piperazine | 437 | 438 | HCl salt | 4 |
| 38 | (3S)-3-methyl-1-(2-{1-[3-(trifluoromethyl)phenyl]-1H- pyrazol-4-yl}-1,3-thiazole-4- carbonyl)piperazine | 421 | 422 | HCl salt | 4 |
| 39 | (3S)-1-{2-[1-(4-chlorophenyl)- 1H-pyrazol-4-yl]-1,3-thiazole-4-carbonyl}-3-methylpiperazine | 388 | 388 | HCl salt | 4 |
| 40 | 2-[1-(4-chlorophenyl)-1H-pyrazol-4-yl]-N-propyl-N-[(3 S)-pyrrolidin-3 -yl] -1,3 -thiazole-4-carboxamide | 416 | 416 | HCl salt | 4 |
| 41 | 2-[1-(4-chlorophenyl)-1H-pyrazol-4-yl]-N-propyl-N-[(3R)-pyrrolidin-3 -yl] -1,3 -thiazole-4-carboxamide | 416 | 416 | HCl salt | 4 |
| 42 | 5-(1-phenyl-1H-pyrazol-4-yl)-N-propyl-N-[(3S)-pyrrolidin-3-yl]thiophene- 3 -carboxamide | 380 | 381 | HCl Salt | 3 |
| 43 | N-propyl-2-[1-(pyridin-2-yl)-1H-pyrazol-4-yl]-N-[(3S)-pyrrolidin-3-yl]-1,3-thiazole-4- carboxamide | 382 | 383 | HCl salt | 4 |
| 44 | N-propyl-2-[1-(pyridin-2-yl)-1H-pyrazol-4-yl]-N-[(3R)-pyrrolidin- 3-yl]-1,3-thiazole-4-carboxamide | 382 | 383 | HCl salt | 4 |
| 45 | N-propyl-N-[(3S)-pyrrolidin-3- yl]-2-{1-[4-(trifluoromethoxy)phenyl]-1H- pyrazol-4-yl}-1,3-thiazole-4-carboxamide | 466 | 466 | HCl salt | 4 |
| 46 | N-propyl-N-[(3R)-pyrrolidin-3- yl]-2-{1-[4-(trifluoromethoxy)phenyl]-1H- pyrazol-4-yl}-1,3-thiazole-4-carboxamide | 466 | 466 | HCl salt | 4 |
| 47 | N-(piperidin-4-yl)-N-(propan-2- yl)-2-{1-[2-(trifluoromethyl)phenyl]-1H-pyrazol-4-yl}-1,3-thiazole-4-carboxamide | 464 | 464 | HCl salt | see full experim ental |
| 48 | N-propyl-N-[(3S)-pyrrolidin-3- yl]-2-{1-[3-(trifluoromethyl)phenyl]-1H- pyrazol-4-yl}-1,3-thiazole-4-carboxamide | 450 | 450 | HCl Salt | 4 |
| 49 | N-propyl-N-[(3R)-pyrrolidin-3- yl]-2-{1-[3-(trifluoromethyl)phenyl]-1H- pyrazol-4-yl}-1,3-thiazole-4-carboxamide | 450 | 450 | HCl Salt | 4 |
| 50 | 1-[(2R)-1-{ 2-[1-(3-methoxyphenyl)-1H-pyrazol-4- yl]-1,3-thiazole-4-carbonyl}pyrrolidin-2-yl]methanamine | 383 | 384 | HCl salt | 4 |
| 51 | 2-[1-(3-methoxyphenyl)-1H-pyrazol-4-yl]-N-propyl-N-[(3 S)-pyrrolidin-3 -yl] -1,3 -thiazole-4-carboxamide | 412 | 412 | HCl salt | 4 |
| 52 | 2-[1-(3-fluorophenyl)-1H-pyrazol-4-yl]-N-propyl-N-[(3 S)-pyrrolidin-3 -yl] -1,3 -thiazole-4-carboxamide | 399 | 400 | HCl salt | 4 |
| 53 | 2-[1 -(3 -fluorophenyl)-1H-pyrazol-4-yl]-N-propyl-N-[(3R)-pyrrolidin-3 -yl] -1,3 -thiazole-4-carboxamide | 399 | 400 | HCl salt | 4 |
| 54 | 2-[1-(4-fluorophenyl)-1H-pyrazol-4-yl]-N-propyl-N-[(3R)-pyrrolidin-3 -yl] -1,3 -thiazole-4-carboxamide | 399 | 400 | HCl salt | 4 |
| 55 | (2R)-1-[2-(1-phenyl-1H-pyrazol- 4-yl)-1,3 -thiazole-4- carbonyl]pyrrolidine-2-carboxylic acid | 367 | 369 | Free Base | see full experim ental |
| 56 | 2-[1-(3-methoxyphenyl)-1H-pyrazol-4-yl]-N-propyl-N-[(3R)-pyrrolidin-3 -yl] -1,3 -thiazole-4-carboxamide | 412 | 412 | HCl salt | 4 |
| 57 | 2-[1-(4-methylphenyl)-1H-pyrazol-4-yl]-N-(piperidin-4-yl)-N-(propan-2-yl)-1, 3 -thiazole-4-carboxamide | 410 | 410 | HCl Salt | 4 |
| 58 | 2-[1-(4-fluorophenyl)-1H-pyrazol-4-yl]-N-propyl-N-[(3 S)-pyrrolidin-3 -yl] -1,3 -thiazole-4-carboxamide | 399 | 400 | HCl salt | 4 |
| 59 | 4-(1-phenyl-1H-pyrazol-4-yl)-N-propyl-N-[(3 S)-pyrrolidin-3-yl]thiophene- 2-carboxamide | 380 | 381 | HCl Salt | 3 |
| 60 | (2S)-1-[2-(1-phenyl-1H-pyrazol- 4-yl)-1,3-thiazole-4- carbonyl]pyrrolidine-2-carboxylic acid | 368 | 369 | Free Base | see full experim ental |
| 61 | 2-[1-(3-methoxyphenyl)-1H-pyrazol-4-yl]-N-(piperidin-4-yl)-N-(propan-2-yl)-1,3-thiazole-4-carboxamide | 426 | 426 | HCl Salt | 4 |
| 62 | (3S)-1-{2-[1-(3-methoxyphenyl)-1H-pyrazol-4-yl]-1,3-thiazole-4- carbonyl}-3 -methylpiperazine | 383 | 384 | HCl Salt | 4 |
| 63 | (3S)-3-methyl-1-{2-[1-(4-methylphenyl)-1H-pyrazol-4-yl]- 1,3-thiazole-4-carbonyl}piperazine | 367 | 368 | HCl Salt | 4 |
| 64 | 2-[1-(4-methylphenyl)-1H-pyrazol-4-yl]-N-propyl-N-[(3 S)-pyrrolidin-3 -yl] -1,3 -thiazole-4-carboxamide | 396 | 396 | HCl salt | 4 |
| 65 | 2-[1-(4-methylphenyl)-1H-pyrazol-4-yl]-N-propyl-N-[(3R)-pyrrolidin-3 -yl] -1,3 -thiazole-4-carboxamide | 396 | 396 | HCl salt | 4 |
| 66 | 2-[1-(2-chlorophenyl)-1H-pyrazol-4-yl]-N-(piperidin-4-yl)-N-(propan-2-yl)-1,3 -thiazole-4-carboxamide | 430 | 430 | HCl salt | 4 |
| 67 | (3S)-3-methyl-1-[5-(1-phenyl-1H-pyrazol-4-yl)-1H-pyrrole-2-carbonyl]piperazine | 335 | 336 | HCl salt | 3 |
| 68 | 1-[(2R)-1-[5-(1-phenyl-1H-pyrazol-4-yl)-1H-pyrrole-2-carbonyl]pyrrolidin-2- yl]methanamine | 335 | 336 | HCl salt | 3 |
| 69 | 2-(5-phenylthiophen-2-yl)-N-propyl-N-[(3S)-pyrrolidin-3-yl]- 1,3-thiazole-4-carboxamide | 398 | 398 | HCl Salt | 3 |
| 70 | 2-(5-phenylthiophen-2-yl)-N- propyl-N-[(3R)-pyrrolidin-3-yl]-1,3 -thiazole-4-carboxamide | 398 | 398 | HCl Salt | 3 |
| 71 | 2-(1-phenyl-1H-pyrazol-4-yl)-N-(propan-2-yl)-N-(pyrrolidin-3-yl)-1,3-thiazole-4-carboxamide | 382 | 382 | HCl Salt | 2 |
| 72 | (3S)-3-methyl-1-(2-{1-[2-(trifluoromethyl)phenyl]-1H- pyrazol-4-yl}-1,3-thiazole-4- carbonyl)piperazine | 421 | 422 | HCl salt | 4 |
| 73 | 1-[(2R)-1-(2-{1-[2-(trifluoromethyl)phenyl]-1H- pyrazol-4-yl}-1,3-thiazole-4- carbonyl)pyrrolidin-2- yl]methanamine | 421 | 422 | HCl salt | 4 |
| 74 | N-(piperidin-4-yl)-N-(propan-2- yl)-2-{1-[4-(trifluoromethyl)phenyl]-1H- pyrazol-4-yl}-1,3-thiazole-4-carboxamide | 464 | 464 | HCl Salt | 4 |
| 75 | (3S)-3-methyl-1-(2-{1-[4-(trifluoromethyl)phenyl]-1H- pyrazol-4-yl}-1,3-thiazole-4- carbonyl)piperazine | 421 | 422 | HCl Salt | 4 |
| 76 | 1-[(2R)-1-(2-{1-[4-(trifluoromethyl)phenyl]-1H- pyrazol-4-yl}-1,3-thiazole-4- carbonyl)pyrrolidin-2- yl]methanamine | 421 | 422 | HCl Salt | 4 |
| 77 | N-propyl-N-[(3S)-pyrrolidin-3- yl]-2-{1-[4-(trifluoromethyl)phenyl]-1H- pyrazol-4-yl}-1,3-thiazole-4-carboxamide | 450 | 450 | HCl Salt | 4 |
| 78 | N-propyl-N-[(3R)-pyrrolidin-3- yl]-2-{1-[4-(trifluoromethyl)phenyl]-1H-pyrazol-4-yl}-1,3-thiazole-4-carboxamide | 450 | 450 | HCl Salt | 4 |
| 79 | (3S)-1-{2-[1-(2-chlorophenyl)- 1H-pyrazol-4-yl]-1,3-thiazole-4-carbonyl}-3-methylpiperazine | 388 | 388 | HCl salt | 4 |
| 80 | 1-[(2R)-1-{2-[1-(2-chlorophenyl)-1H-pyrazol-4-yl]- 1,3-thiazole-4- carbonyl } pyrrolidin-2-yl]methanamine | 388 | 388 | HCl salt | 4 |
| 81 | 4-(1-phenyl-1H-pyrazol-4-yl)-N-propyl-N-[(3R)-pyrrolidin-3-yl]thiophene- 2-carboxamide | 380 | 381 | HCl Salt | 3 |
| 82 | N-propyl-N-[(3S)-pyrrolidin-3- yl]-2-{1-[2-(trifluoromethyl)phenyl]-1H-pyrazol-4-yl}-1,3-thiazole-4-carboxamide | 450 | 450 | HCl salt | 4 |
| 83 | N-propyl-N-[(3R)-pyrrolidin-3- yl]-2-{1-[2-(trifluoromethyl)phenyl]-1H-pyrazol-4-yl}-1,3-thiazole-4-carboxamide | 450 | 450 | HCl salt | 4 |
| 84 | 2-[1-(2-methylphenyl)-1H-pyrazol-4-yl]-N-(piperidin-4-yl)-N-(propan-2-yl)-1,3-thiazole-4-carboxamide | 410 | 410 | HCl Salt | 4 |
| 85 | (3S)-3-methyl-1-{2-[1-(2-methylphenyl)-1H-pyrazol-4-yl]- 1,3-thiazole-4- carbonyl }piperazine | 367 | 368 | HCl Salt | 4 |
| 86 | 1-[(2R)-1-{2-[1-(2-methylphenyl)-1H-pyrazol-4-yl]- 1,3-thiazole-4- carbonyl } pyrrolidin-2-yl]methanamine | 367 | 368 | HCl Salt | 4 |
| 87 | 2-[1-(2-methylphenyl)-1H-pyrazol-4-yl]-N-propyl-N-[(3 S)-pyrrolidin-3 -yl] -1,3 -thiazole-4-carboxamide | 396 | 396 | HCl Salt | 4 |
| 88 | 2-[1-(2-methylphenyl)-1H-pyrazol-4-yl]-N-propyl-N-[(3R)-pyrrolidin-3 -yl] -1,3 -thiazole-4-carboxamide | 396 | 396 | HCl Salt | 4 |
| 89 | 2-[1-(2-chlorophenyl)-1H-pyrazol-4-yl]-N-propyl-N-[(3 S)-pyrrolidin-3-yl] -1,3 -thiazole-4-carboxamide | 416 | 416 | salt | 4 |
| 90 | 2-[1-(2-chlorophenyl)-1H-pyrazol-4-yl]-N-propyl-N-[(3R)-pyrrolidin-3 -yl] -1,3 -thiazole-4-carboxamide | 416 | 416 | HCl salt | 4 |
| 91 | 1-[(2R)-1-{2-[1-(2-fluorophenyl)-1H-pyrazol-4-yl]- 1,3-thiazole-4-carbonyl}pyrrolidin-2-yl]methanamine | 371 | 372 | HCl salt | 4 |
| 92 | 2-[1-(2-fluorophenyl)-1H-pyrazol-4-yl]-N-propyl-N-[(3 S)-pyrrolidin-3 -yl] -1,3 -thiazole-4-carboxamide | 399 | 400 | HCl salt | 4 |
| 93 | 5-(1-phenyl-1H-pyrazol-4-yl)-N-propyl-N-[(3S)-pyrrolidin-3-yl]- 1H-pyrrole-2-carboxamide | 363 | 364 | HCl salt | 3 |
| 94 | 5-(1-phenyl-1H-pyrazol-4-yl)-N-propyl-N-[(3R)-pyrrolidin-3-yl]- 1H-pyrrole-2-carboxamide | 363 | 364 | HCl salt | see full experim ental |
| 95 | 2-[1-(2-fluorophenyl)-1H-pyrazol-4-yl]-N-(piperidin-4-yl)-N-(propan-2-yl)- 1, 3 -thiazole-4-carboxamide | 414 | 414 | HCl salt | 4 |
| 96 | (3S)-1-{2-[1-(2-fluorophenyl)-1H-pyrazol-4-yl]-1,3-thiazole-4-carbonyl}-3-methylpiperazine | 371 | 372 | HCl salt | 4 |
| 97 | 2-[1-(2-fluorophenyl)-1H-pyrazol-4-yl]-N-propyl-N-[(3R)-pyrrolidin-3 -yl] -1,3 -thiazole-4-carboxamide | 399 | 400 | HCl salt | 4 |
| 98 | 5-(1-phenyl-1H-pyrazol-4-yl)-N-propyl-N-[(3S)-pyrrolidin-3- yl]furan-2-carboxamide | 364 | 365 | HCl Salt | 3 |
| 99 | 5-(1-phenyl-1H-pyrazol-4-yl)-N-propyl-N-[(3R)-pyrrolidin-3- yl]furan-2-carboxamide | 364 | 365 | HCl Salt | 3 |
| 100 | 5-(1-phenyl-1H-pyrazol-4-yl)-N-[(piperidin-4-yl)methyl]furan-2-carboxamide | 350 | 351 | HCl Salt | 3 |
| 101 | N-[2-(2-aminoethoxy)ethyl]-5-(1 phenyl-1H-pyrazol-4-yl)furan-2-carboxamide | 340 | 341 | HCl Salt | 3 |
| 102 | (3S)-3-methyl-1-[5-(1-phenyl- 1H-pyrazol-4-yl)furan-2-carbonyl]piperazine | 336 | 337 | HCl Salt | 3 |
| 103 | N-(1-ethylpiperidin-4-yl)-4-(1-phenyl-1H-pyrazol-4- yl)thiophene-2-carboxamide | 381 | 381 | HCl Salt | 3 |
| 104 | 1-[2-(1-phenyl-1H-pyrazol-4-yl)-1,3-thiazole-4-carbonyl]piperazine | 339 | 340 | HCl Salt | 2 |
| 105 | 4-[2-(1-phenyl-1H-pyrazol-4-yl)- 1,3-thiazole-4- carbonyl]piperazine-1-sulfonamide | 418 | 419 | Free Base | see full experim ental |
| 106 | N-{[(2R)-morpholin-2-yl]methyl}-2-(1-phenyl-1H- pyrazol-4-yl)-1,3-thiazole-4- carboxamide | 369 | 370 | HCl salt | 2 |
| 107 | N-{[(2S)-morpholin-2-yl]methyl}-2-(1-phenyl-1H- pyrazol-4-yl)-1,3-thiazole-4- carboxamide | 369 | 370 | HCl salt | 2 |
| 108 | 2-(1-phenyl-1H-pyrazol-4-y1)-N-{[(3 S)-pyrrolidin-3-yl]methyl}- 1,3-thiazole-4-carboxamide | 353 | 354 | HCl salt | 2 |
| 109 | N-(1-ethylpiperidin-4-yl)-5-(1- phenyl-1H-pyrazol-4-yl)furan-2-carboxamide | 364 | 365 | HCl Salt | 3 |
| 110 | N-[2-(2-aminoethoxy)ethyl]-4-(1 phenyl-1H-pyrazol-4-yl)thiophene-2-carboxamide | 356 | 357 | HCl Salt | 3 |
| 111 | 4-(1-phenyl-1H-pyrazol-4-y1)-N-[(piperidin-4- yl)methyl]thiophene-2-carboxamide | 366 | 367 | HCl Salt | 3 |
| 112 | 2-(1-phenyl-1H-pyrazol-4-y1)-N-{[(3R)-pyrrolidin-3-yl]methyl}- 1,3-thiazole-4-carboxamide | 353 | 354 | HCl salt | 2 |
| 113 | (3S)-3-methyl-1-[4-(1-phenyl- 1H-pyrazol-4-yl)thiophene-2-carbonyl]piperazine | 352 | 353 | HCl Salt | 3 |
| 114 | 1-[(2R)-1-[4-(1-phenyl-1H-pyrazol-4-yl)thiophene-2-carbonyl]pyrrolidin-2- yl]methanamine | 352 | 353 | HCl Salt | 3 |
| 115 | 4-(5-phenyl-1,3,4-thiadiazol-2- yl)-N-propyl-N-(pyrrolidin-3-yl)thiophene-2-carboxamide | 398 | 399 | HCl Salt | 3 |
| 116 | 2-(2-phenyl-1,3-oxazol-5-yl)-N- propyl-N-(pyrrolidin-3-yl)-1,3-thiazole-4-carboxamide | 382 | 383 | HCl Salt | 3 |
| 117 | N-[(1R,3R)-3-aminocyclopentyl] 2-(1-phenyl-1H-pyrazol-4-yl)- 1,3-thiazole-4-carboxamide | 353 | 354 | HCl Salt | 2 |
| 118 | (3R)-3-methyl-1-[2-(1-phenyl- 1H-pyrazol-4-yl)-1,3-thiazole-4-carbonyl]piperazine | 353 | 354 | HCl Salt | 2 |
| 119 | 1-{1-[2-(1-phenyl-1H-pyrazol-4- yl)-1,3-thiazole-4- carbonyl]azetidin-3-yl}methanamine | 339 | 340 | Metha n sulfon ic acid salt | 2 |
| 120 | 5-(1-phenyl-1H-pyrazol-4-yl)-N-propyl-N-(pyrrolidin-3-yl)-1H-pyrrole-2-carboxamide | 363 | 364 | HCl Salt | 3 |
| 121 | 1-{1-[2-(1-phenyl-1H-pyrazol-4- yl)-1,3-thiazole-4- carbonyl]pyrrolidin-3-yl}methanamine | 353 | 354 | HCl Salt | 2 |
| 122 | 2-(5-phenylthiophen-2-yl)-N- propyl-N-(pyrrolidin-3-yl)-1,3-thiazole-4-carboxamide | 398 | 398 | HCl Salt | 3 |
| 123 | 4-(1-phenyl-1H-pyrazol-4-yl)-N-propyl-N-(pyrrolidin-3-yl)-1H-pyrrole-2-carboxamide | 363 | 364 | HCl Salt | 3 |
| 124 | 1-[2-(1-phenyl-1H-pyrazol-4-yl)- 1,3-thiazole-4-carbonyl]azetidin-3-amine | 325 | 326 | HCl Salt | 2 |
| 125 | methyl 4-hydroxy-1-[2-(1- phenyl-1H-pyrazol-4-yl)-1,3-thiazole-4-carbonyl]pyrrolidine-2-carboxylate | 398 | 399 | Free base | 1 |
| 126 | 4-(5-phenyl-1,3,4-oxadiazol-2- yl)-N-propyl-N-(pyrrolidin-3-yl)thiophene-2-carboxamide | 382 | 383 | HCl salt | 3 |
| 127 | 4-(1-phenyl-1H-pyrazol-4-y1)-N-propyl-N-(pyrrolidin-3-yl)-1,3-thiazole- 2-carboxamide | 382 | 382 | HCl Salt | 2 |
| 128 | N-[(2R)-2-aminopropyl]-2-(1-phenyl-1H-pyrazol-4-yl)-1,3- thiazole-4-carboxamide | 327 | 328 | HCl Salt | 2 |
| 129 | N-[(2R)-1-aminopropan-2-yl]-2-(1-phenyl-1H-pyrazol-4-yl)-1,3-thiazole-4-carboxamide | 327 | 328 | HCl Salt | 2 |
| 130 | 5-(1-phenyl-1H-pyrazol-4-yl)-N-propyl-N-(pyrrolidin-3- yl)thiophene-3-carboxamide | 380 | 381 | HCl Salt | 3 |
| 131 | 1-[(2S)-1-[2-(1-phenyl-1H-pyrazol-4-yl)- 1, 3 -thiazole-4-carbonyl]pyrrolidin-2- yl]methanamine | 353 | 354 | HCl Salt | 2 |
| 132 | 2-(1-phenyl-1H-pyrazol-4-y1)-N-(piperidin-3 -yl)- 1, 3 -thiazole-4-carboxamide | 353 | 354 | HCl Salt | 2 |
| 133 | N-cyclobutyl-2-(1-phenyl-1H-pyrazol-4-yl)-N-(piperidin-3-yl)-1,3-thiazole-4-carboxamide | 408 | 408 | HCl Salt | 2 |
| 134 | N,N-diethyl-1-[2-(1-phenyl-1H-pyrazol-4-yl)-1,3-thiazole-4-carbonyl]pyrrolidin-3-amine | 396 | 396 | Free Base | 1 |
| 135 | 2-(1-phenyl-1H-pyrazol-4-yl)-N-propyl-N-(pyrrolidin-3-yl)-1,3-oxazole-4-carboxamide | 365 | 366 | HCl Salt | 3 |
| 136 | 4-(1-phenyl-1H-pyrazol-4-yl)-N-propyl-N-(pyrrolidin-3-yl)furan-2-carboxamide | 364 | 365 | HCl Salt | 3 |
| 137 | 1-[(3S)-1-[2-(1-phenyl-1H-pyrazol-4-yl)-1,3-thiazole-4-carbonyl]pyrrolidin-3- yl]methanamine | 353 | 354 | HCl Salt | 2 |
| 138 | N-(1-methylpiperidin-4-yl)-2-(1-phenyl-1H-pyrazol-4-yl)-1,3-thiazole-4-carboxamide | 367 | 368 | Free Base | 1 |
| 139 | N-methyl-2-(1-phenyl-1H-pyrazol-4-yl)-N-(piperidin-3-yl)- 1,3-thiazole-4-carboxamide | 367 | 368 | HCl Salt | 2 |
| 140 | 5-(1-phenyl-1H-pyrazol-4-yl)-N-propyl-N-(pyrrolidin-3-yl)furan- 2-carboxamide | 364 | 365 | HCl Salt | 3 |
| 141 | 4-(1-phenyl-1H-pyrazol-4-yl)-N-propyl-N-(pyrrolidin-3-yl)thiophene-2-carboxamide | 380 | 381 | HCl Salt | 3 |
| 142 | (3S)-1-[2-(1-phenyl-1H-pyrazol-4-yl)-1,3-thiazole-4-carbonyl]piperidin-3-amine | 353 | 354 | HCl Salt | 2 |
| 143 | (3R)-1-[2-(1-phenyl-1H-pyrazol- 4-yl)-1,3-thiazole-4-carbonyl]piperidin-3-amine | 353 | 354 | HCl Salt | 2 |
| 144 | 1-[2-(1-phenyl-1H-pyrazol-4-yl)- 1,3-thiazole-4- carbonyl]pyrrolidine-2-carboxylic acid | 367 | 369 | Free Base | 1 |
| 145 | N-(2-amino-2-methylpropyl)-2- (1-phenyl-1H-pyrazol-4-yl)-1,3-thiazole-4-carboxamide | 341 | 342 | HCl Salt | commer cially availabl e |
| 146 | 2-(1-phenyl-1H-pyrazol-4-y1)-N-[(1r,3r)-3-aminocyclobutyl]-1,3-thiazole-4-carboxamide | 339 | 340 | HCl Salt | 2 |
| 147 | 2-(1-phenyl-1H-pyrazol-4-y1)-N-[(1r,4r)-4-hydroxycyclohexyl]- 1,3-thiazole-4-carboxamide | 368 | 369 | Free Base | 1 |
| 148 | 2-(1-phenyl-1H-pyrazol-4-yl)-N-{2,6,6-trimethylbicyclo[3.1.1]heptan-3-yl}-1,3-thiazole-4-carboxamide | 407 | 407 | Free Base | 1 |
| 149 | N-ethyl-2-(1-phenyl-1H-pyrazol- 4-yl)-N-(pip eridin-4-yl)-1, 3 -thiazole-4-carboxamide | 382 | 382 | HCl Salt | 2 |
| 150 | N-ethyl-2-(1-phenyl-1H-pyrazol- 4-yl)-N-(piperidin-3 -yl)-1,3 -thiazole-4-carboxamide | 382 | 382 | HCl Salt | 2 |
| 151 | N-cyclohexyl-2-(1-phenyl-1H- pyrazol-4-yl)- N-propyl-1 , 3 -thiazole-4-carboxamide | 395 | 395 | Free Base | 1 |
| 152 | methyl({1-[2-(1-phenyl-1H-pyrazol-4-yl)-1,3-thiazole-4- carbonyl]pyrrolidin-2-yl}methyl)amine | 367 | 368 | Free Base | 2 |
| 153 | 1-{3-methyl-1-[2-(1-phenyl-1H-pyrazol-4-yl)- 1, 3 -thiazole-4-carbonyl]piperidin-2-yl}methanamine | 382 | 382 | HCl Salt | 2 |
| 154 | 1-[2-(1-phenyl-1H-pyrazol-4-yl)- 1,3-thiazole-4-carbonyl]-1,4-diazepane | 353 | 354 | HCl Salt | 2 |
| 155 | N-cyclohexyl-N-[2-(dimethylamino)ethyl]-2-(1- phenyl-1H-pyrazol-4-yl)-1,3-thiazole-4-carboxamide | 424 | 424 | Free Base | 1 |
| 156 | 1-[(2R)-1-[2-(1-phenyl-1H-pyrazol-4-yl)-1,3-thiazole-4-carbonyl]pyrrolidin-2- yl]methanamine | 353 | 354 | HCl Salt | see full experim ental |
| 157 | (3S)-3-methyl-1-[2-(1-phenyl-1H-pyrazol-4-yl)-1,3-thiazole-4-carbonyl]piperazine | 353 | 354 | HCl Salt | 2 |
| 158 | {1-[2-(1-phenyl-1H-pyrazol-4-yl)-1,3-thiazole-4-carbonyl]piperazin-2-yl} methanol | 369 | 370 | Free Base | 2 |
| 159 | N-(3-aminopropyl)-2-(1-phenyl-1H-pyrazol-4-yl)-1,3-thiazole-4-carboxamide | 327 | 328 | HCl Salt | 2 |
| 160 | N-(3-methylpiperidin-3-yl)-2-(1-phenyl-1H-pyrazol-4-yl)-1,3-thiazole-4-carboxamide | 367 | 368 | HCl Salt | 2 |
| 161 | N-(5-aminopentyl)-2-(1-phenyl-1H-pyrazol-4-yl)-1,3-thiazole-4-carboxamide | 355 | 356 | Free Base | 2 |
| 162 | N-(2-aminoethyl)-2-(1-phenyl- 1H-pyrazol-4-yl)-1,3-thiazole-4-carboxamide | 313 | 314 | HCl Salt | 2 |
| 163 | 2-(1-phenyl-1H-pyrazol-4-yl)-N-(piperidin-4-yl)-N-(propan-2-yl)- 1,3-thiazole-4-carboxamide | 396 | 396 | HCl Salt | see full experim ental |
| 164 | N-methyl-1-[2-(1-phenyl-1H- pyrazol-4-yl)-1,3-thiazole-4- carbonyl]-N-(propan-2-yl)piperidin-4-amine | 410 | 410 | Free Base | 1 |
| 165 | 1-{1-[2-(1-phenyl-1H-pyrazol-4- yl)-1,3-thiazole-4- carbonyl]piperidin-4-yl}ethan-1-amine | 382 | 382 | HCl Salt | 2 |
| 166 | 7-[2-(1-phenyl-1H-pyrazol-4-yl)- 1,3-thiazole-4-carbonyl]-1,7-diazaspiro[3.5]nonan-2-one | 393 | 394 | Free Base | 1 |
| 167 | 1-[2-(1-phenyl-1H-pyrazol-4-yl)- 1,3-thiazole-4-carbonyl]piperidin-3-amine | 353 | 354 | HCl Salt | 2 |
| 168 | 2-methyl-4-[2-(1-phenyl-1H- pyrazol-4-yl)-1,3-thiazole-4-carbonyl]morpholine | 354 | 355 | Free Base | 1 |
| 169 | N-methyl-N-[(oxolan-2-yl)methyl]-2-(1-phenyl-1H- pyrazol-4-yl)-1,3-thiazole-4- carboxamide | 368 | 369 | Free Base | 1 |
| 170 | 2-(1-phenyl-1H-pyrazol-4-y1)-N-propyl-N-[(3S)-pyrrolidin-3-yl]- 1,3-thiazole-4-carboxamide | 381 | 382 | HCl Salt | see full experim ental |
| 171 | 2-(1-phenyl-1H-pyrazol-4-y1)-N-propyl-N-[(3R)-pyrrolidin-3-yl]- 1,3-thiazole-4-carboxamide | 381 | 382 | HCl Salt | see full experim ental |
| 172 | N-methyl-N-(oxan-4-yl)-2-(1-phenyl-1H-pyrazol-4-yl)-1,3-thiazole-4-carboxamide | 368 | 369 | Free Base | 1 |
| 173 | N-ethyl-N-methyl-2-(1-phenyl-1H-pyrazol-4-yl)-1,3-thiazole-4-carboxamide | 312 | 313 | Free Base | 1 |
| 174 | N-(1-benzylpyrrolidin-3-yl)-2-(1-phenyl-1H-pyrazol-4-yl)-1,3-thiazole-4-carboxamide | 430 | 430 | Free Base | 1 |
| 175 | N-[2-(2-aminoethoxy)ethyl]-2-(1 phenyl-1H-pyrazol-4-yl)-1,3- thiazole-4-carboxamide | 357 | 358 | HCl Salt | 2 |
| 176 | N-[2-(4-hydroxyphenyl)ethyl]-2- (1-phenyl-1H-pyrazol-4-yl)-1,3-thiazole-4-carboxamide | 390 | 391 | Free Base | 1 |
| 177 | 2-(1-phenyl-1H-pyrazol-4-y1)-N-[(piperidin-4-yl)methyl] -1,3- thiazole-4-carboxamide | 367 | 368 | HCl Salt | 2 |
| 178 | N-[(morpholin-2-yl)methyl]-2-(1 phenyl-1H-pyrazol-4-yl)-1,3-thiazole-4-carboxamide | 369 | 370 | HCl Salt | 2 |
| 179 | 2-(1-phenyl-1H-pyrazol-4-yl)-N-[(pyrrolidin-3-yl)methyl]-1,3-thiazole-4-carboxamide | 353 | 354 | HCl Salt | 2 |
| 180 | N-(azetidin-3-yl)-2-(1-phenyl-1H-pyrazol-4-yl)-1,3-thiazole-4-carboxamide | 325 | 326 | HCl Salt | 2 |
| 181 | methyl 4-[2-(1-phenyl-1H- pyrazol-4-yl)-1,3-thiazole-4-amido]piperidine-4-carboxylate | 411 | 412 | HCl Salt | 2 |
| 182 | N-(1-benzylpiperidin-4-yl)-2-(1-phenyl-1H-pyrazol-4-yl)-1,3- thiazole-4-carboxamide | 444 | 444 | Free Base | 1 |
| 183 | 2-(1-phenyl-1H-pyrazol-4-y1)-N-[(3R)-pyrrolidin-3-yl]-1,3- thiazole-4-carboxamide | 339 | 340 | HCl Salt | 2 |
| 184 | 2-(1-phenyl-1H-pyrazol-4-y1)-N-(pyrrolidin-3 -yl)- 1, 3 -thiazole-4-carboxamide | 339 | 340 | HCl Salt | see full experim ental |
| 185 | N-(1-ethylpiperidin-4-yl)-2-(1- phenyl-1H-pyrazol-4-yl)-1,3-thiazole-4-carboxamide | 382 | 382 | Free Base | 1 |
| 186 | N-{1-azabicyclo[2.2.2]octan-3- yl}-N-methyl-2-(1-phenyl-1H- pyrazol-4-yl)-1,3 -thiazole-4-carboxamide | 394 | 394 | Free Base | 1 |
| 187 | N,N-dimethyl-1-[2-(1-phenyl- 1H-pyrazol-4-yl)-1,3-thiazole-4-carbonyl]pyrrolidin-3-amine | 367 | 368 | Free Base | 1 |
| 188 | N-(1-ethylpyrrolidin-3-yl)-N- methyl-2-(1-phenyl-1H-pyrazol- 4-yl)-1,3-thiazole-4-carboxamide | 382 | 382 | Free Base | 1 |
| 189 | N-methyl-N-[(1- methylpyrrolidin-3-yl)methyl]-2- (1-phenyl-1H-pyrazol-4-yl)-1,3-thiazole-4-carboxamide | 382 | 382 | Free Base | 1 |
| 190 | N-{2-[cyclohexyl(methyl)amino]ethyl }-N-methyl-2-(1-phenyl-1H- pyrazol-4-yl)-1,3-thiazole-4- carboxamide | 424 | 424 | Free Base | 1 |
| 191 | N-cyclopentyl-N-(2- methylpropyl)-2-(1-phenyl-1H- pyrazol-4-yl)-1,3-thiazole-4-carboxamide | 395 | 395 | Free Base | 1 |
| 192 | N-cyclopentyl-N-ethyl-2-(1- phenyl-1H-pyrazol-4-yl)-1,3-thiazole-4-carboxamide | 366 | 367 | Free Base | 1 |
| 193 | 1-ethyl-4-[2-(1-phenyl-1H- pyrazol-4-yl)-1,3-thiazole-4-carbonyl]-1,4-diazepane | 382 | 382 | Free Base | 1 |
| 194 | N-cyclopentyl-N-cyclopropyl-2- (1-phenyl-1H-pyrazol-4-yl)-1,3-thiazole-4-carboxamide | 378 | 379 | Free Base | 1 |
| 195 | N-methyl-2-(1-phenyl-1H- pyrazol-4-yl)-N-[1-(propan-2- yl)piperidin-4-yl]-1,3 -thiazole-4-carboxamide | 410 | 410 | Free Base | 1 |
| 196 | N-(1-ethylpiperidin-4-yl)-N- methyl-2-(1-phenyl-1H-pyrazol- 4-yl)-1,3-thiazole-4-carboxamide | 396 | 396 | Free Base | see full experim ental |
| 197 | N,N-dimethyl-1-[2-(1-phenyl- 1H-pyrazol-4-yl)-1,3-thiazole-4-carbonyl]piperidin-4-amine | 382 | 382 | Free Base | 1 |
| 198 | 1-{4-[2-(1-phenyl-1H-pyrazol-4- yl)-1,3-thiazole-4- carbonyl]piperazin-1-yl}ethan-1-one | 381 | 382 | Free Base | 1 |
| 199 | 1-ethyl-4-[2-(1-phenyl-1H-pyrazol-4-yl)- 1, 3 -thiazole-4-carbonyl]piperazine | 367 | 368 | Free Base | 1 |
| 200 | 4-[2-(1-phenyl-1H-pyrazol-4-yl)-1,3 -thiazole-4- carbonyl]piperazin-2-one | 353 | 354 | Free Base | 1 |
| 201 | N-cyclohexyl-N-methyl-2-(1- phenyl-1H-pyrazol-4-yl)-1,3-thiazole-4-carboxamide | 366 | 367 | Free Base | 1 |
| 202 | 2-(1-phenyl-1H-pyrazol-4-yl)-N-[2-(piperidin-1-yl)ethyl]-1,3- thiazole-4-carboxamide | 382 | 382 | Free Base | 1 |
| 203 | N-(cyclohexylmethyl)-2-(1-phenyl-1H-pyrazol-4-yl)-1,3- thiazole-4-carboxamide | 366 | 367 | Free Base | 1 |
| 204 | N-[2-(morpholin-4-yl)ethyl]-2-(1-phenyl-1H-pyrazol-4-yl)-1,3-thiazole-4-carboxamide | 383 | 384 | Free Base | 1 |
| 205 | N-(oxan-4-yl)-2-(1-phenyl-1H- pyrazol-4-yl)-1,3 -thiazole-4-carboxamide | 354 | 355 | Free Base | 1 |
| 206 | N-cyclohexyl-2-(1-phenyl-1H- pyrazol-4-yl)-1,3 -thiazole-4-carboxamide | 352 | 353 | Free Base | 1 |
| 207 | N-(2-hydroxyethyl)-2-(1-phenyl- 1H-pyrazol-4-yl)-1,3-thiazole-4-carboxamide | 314 | 315 | Free Base | 1 |
| 208 | N-[(3S)-1-methylpiperidin-3-yl]-2-(1-phenyl-1H-pyrazol-4-yl)- 1,3-thiazole-4-carboxamide | 367 | 368 | Free Base | 1 |
| 209 | 1-[2-(1-phenyl-1H-pyrazol-4-yl)- 1,3-thiazole-4-carbonyl]-4-(propan-2-yl)piperazine | 382 | 382 | Free Base | 1 |
| 210 | 2-{4-[2-(1-phenyl-1H-pyrazol-4- yl)-1,3-thiazole-4-carbonyl]piperazin-1-yl}-N-(propan-2-yl)acetamide | 439 | 439 | Free Base | 1 |

**Table 2: Compounds according to the present invention, chemical structures, and names**

| **Compound/ Formula Number** | **Structure** | **Chemical Name** |
|---|---|---|
| 1 | 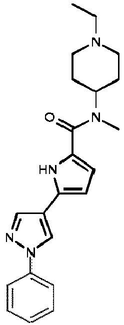 | N-(1-ethylpiperidin-4-yl)-N-methyl-5-(1-phenyl-1H- pyrazol-4-yl)-1H-pyrrole-2-carboxamide |
| 2 | 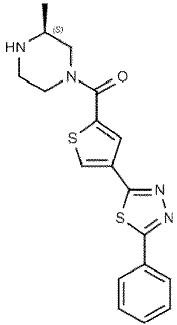 | (3S)-3-methyl-1-[4-(5-phenyl-1,3,4-thiadiazol-2-yl)thiophene-2- carbonyl]piperazine |
| 3 | 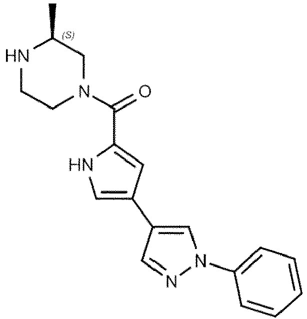 | (3S)-3-methyl-1-[4-(1-phenyl-1H-pyrazol-4-yl)-1H-pyrrole-2- carbonyl]piperazine |
| 4 | 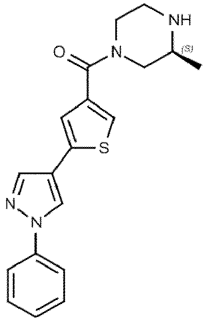 | (3S)-3-methyl-1-[5-(1-phenyl-1H-pyrazol-4-yl)thiophene-3-carbonyl]piperazine |
| 5 | 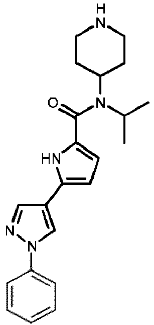 | 5-(1-phenyl-1H-pyrazol-4- yl)-N-(piperidin-4-yl)-N- (propan-2-yl)-1H-pyrrole-2-carboxamide |
| 6 | 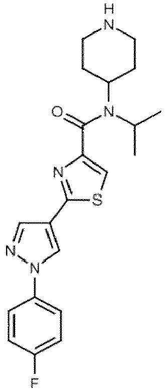 | 2-[1-(4-fluorophenyl)-1H- pyrazol-4-yl]-N-(piperidin-4-yl)-N-(propan-2-yl)-1,3-thiazole-4-carboxamide |
| 7 | 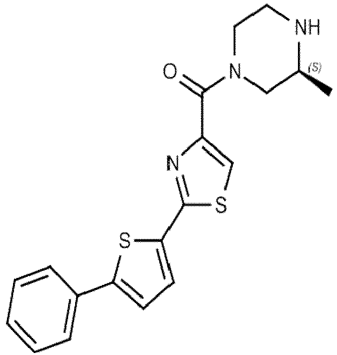 | (3S)-3-methyl-1-[2-(5-phenylthiophen-2-yl)- 1, 3 -thiazole-4-carbonyl]piperazine |
| 8 | 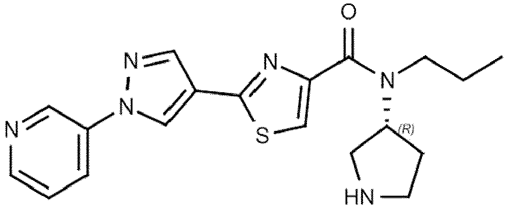 | N-propyl-2-[1-(pyridin-3-yl)-1H-pyrazol-4-yl]-N-[(3R)-pyrrolidin-3-yl]-1,3-thiazole-4-carboxamide |
| 9 | 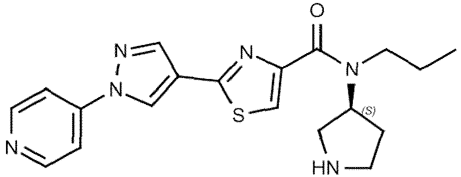 | N-propyl-2-[1 -(pyridin-4-yl)-1H-pyrazol-4-yl]-N-[(3S)-pyrrolidin-3-yl]-1,3-thiazole-4-carboxamide |
| 10 | 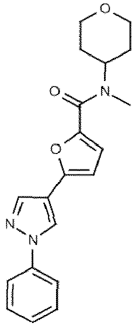 | N-methyl-N-(oxan-4-yl)-5-(1-phenyl-1H-pyrazol-4- yl)furan-2-carboxamide |
| 11 | 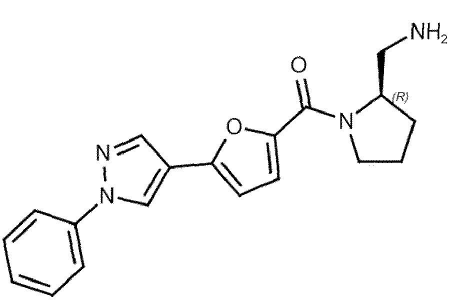 | 1-[(2R)-1-[5-(1-phenyl-1H-pyrazol-4-yl)furan-2-carbonyl]pyrrolidin-2-yl]methanamine |
| 12 | 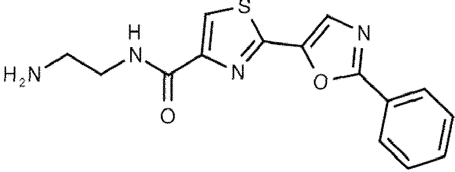 | N-(2-aminoethyl)-2-(2-phenyl-1,3-oxazol-5-yl)-1,3-thiazole-4-carboxamide |
| 13 | 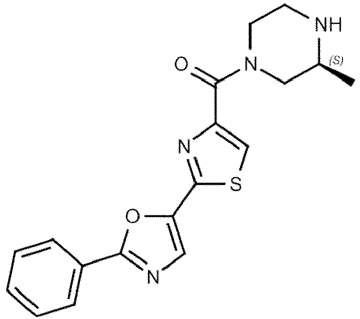 | (3S)-3-methyl-1-[2-(2-phenyl-1,3-oxazol-5-yl)-1,3-thiazole-4-carbonyl]piperazine |
| 14 | 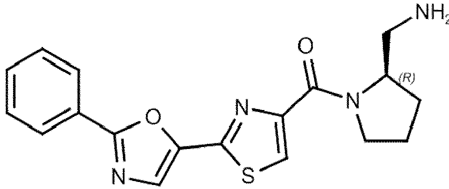 | 1-[(2R)-1-[2-(2-phenyl-1,3-oxazol-5-yl)-1,3-thiazole-4-carbonyl]pyrrolidin-2- yl]methanamine |
| 15 | 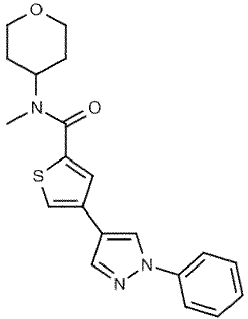 | N-methyl-N-(oxan-4-yl)-4-(1-phenyl-1H-pyrazol-4- yl)thiophene-2- carboxamide |
| 16 | 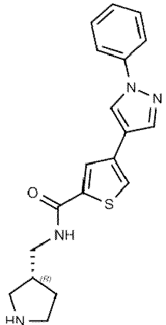 | 4-(1-phenyl-1H-pyrazol-4-yl)-N-{[(3R)-pyrrolidin-3-yl]methyl}thiophene-2-carboxamide |
| 17 | 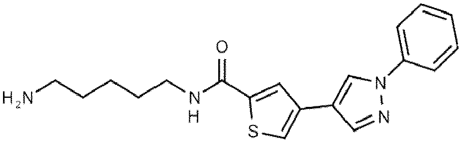 | N-(5-aminopentyl)-4-(1-phenyl-1H-pyrazol-4- yl)thiophene-2-carboxamide |
| 18 | 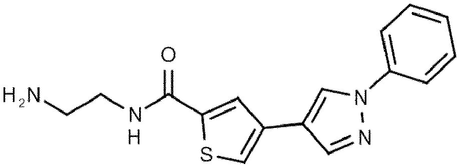 | N-(2-aminoethyl)-4-(1-phenyl-1H-pyrazol-4- yl)thiophene-2-carboxamide |
| 19 | 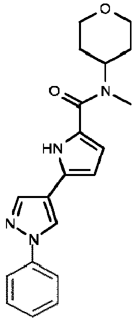 | N-methyl-N-(oxan-4-yl)-5- (1-phenyl-1H-pyrazol-4-yl)-1H-pyrrole-2-carboxamide |
| 20 | | N-(2-aminoethyl)-5-(1- phenyl-1H-pyrazol-4-yl)- 1H-pyrrole-2-carboxamide |
| 21 | 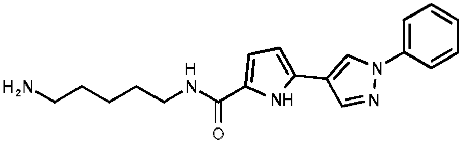 | N-(5-aminopentyl)-5-(1-phenyl-1H-pyrazol-4-yl)- 1H-pyrrole-2-carboxamide |
| 22 | 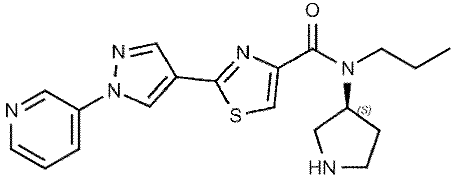 | N-propyl-2-[1-(pyridin-3-yl)-1H-pyrazol-4-yl]-N-[(3 S)-pyrrolidin-3-yl]-1,3-thiazole-4-carboxamide |
| 23 | 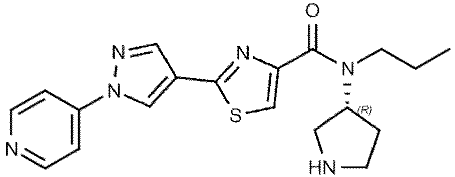 | N-propyl-2-[1 -(pyridin-4-yl)-1H-pyrazol-4-yl]-N-[(3R)-pyrrolidin-3-yl]-1,3-thiazole-4-carboxamide |
| 24 | 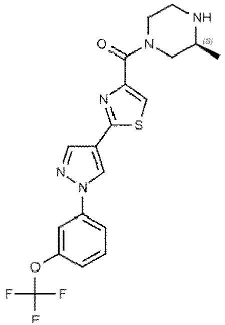 | (3S)-3-methyl-1-(2-{1-[3-(trifluoromethoxy)phenyl]-1H-pyrazol-4-yl}-1,3- thiazole-4- carbonyl)piperazine |
| 25 | 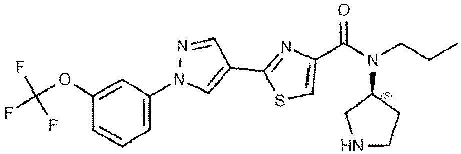 | N-propyl-N-[(3S)-pyrrolidin-3-yl]-2-{1-[3-(trifluoromethoxy)penyl]-1H-pyrazol-4-yl}-1,3- thiazole-4-carboxamide |
| 26 | 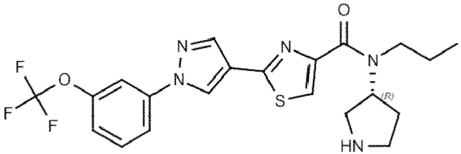 | N-propyl-N-[(3R)-pyrrolidin-3-yl]-2-{ 1-[3-(trifluoromethoxy)phenyl]-1H-pyrazol-4-yl}-1,3- thiazole-4-carboxamide |
| 27 | 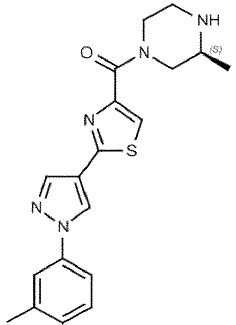 | (3S)-3-methyl-1-{2-[1-(3-methylphenyl)-1H-pyrazol-4-yl]-1,3-thiazole-4- carbonyl}piperazine |
| 28 | 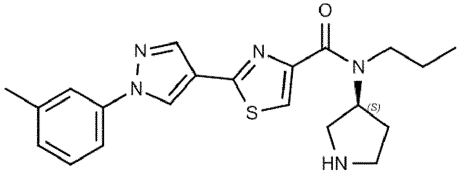 | 2-[1-(3-methylphenyl)-1H-pyrazol-4-yl]-N-propyl-N-[(3S)-pyrrolidin-3-yl]-1,3-thiazole-4-carboxamide |
| 29 | 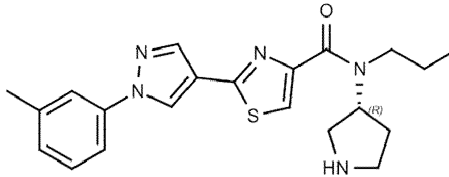 | 2-[1-(3-methylphenyl)-1H-pyrazol-4-yl]-N-propyl-N- [(3R)-pyrrolidin-3-yl]-1,3-thiazole-4-carboxamide |
| 30 | 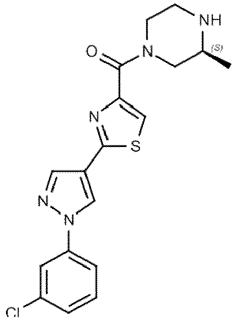 | (3 S)-1-{2-[1-(3-chlorophenyl)-1H-pyrazol-4-yl]-1,3-thiazole-4- carbonyl}-3-methylpiperazine |
| 31 | 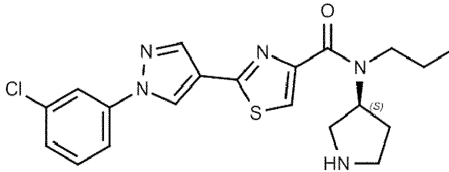 | 2-[1-(3-chlorophenyl)-1H-pyrazol-4-yl]-N-propyl-N- [(3S)-pyrrolidin-3-yl]-1,3-thiazole-4-carboxamide |
| 32 | 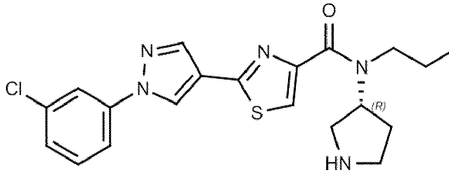 | 2-[1-(3-chlorophenyl)-1H-pyrazol-4-yl]-N-propyl-N- [(3R)-pyrrolidin-3-yl]-1,3-thiazole-4-carboxamide |
| 33 | 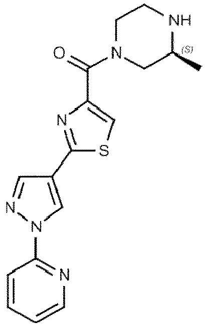 | (3S)-3-methyl-1-{2-[1-(pyridin-2-yl)-1H-pyrazol-4-yl]-1,3-thiazole-4- carbonyl}piperazine |
| 34 | 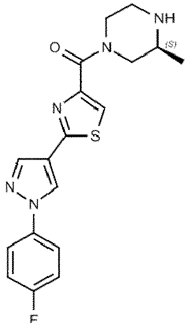 | (3S)-1-{2-[1-(4-fluorophenyl)-1H-pyrazol-4yl]-1,3-thiazole-4- carbonyl}-3-methylpiperazine |
| 35 | 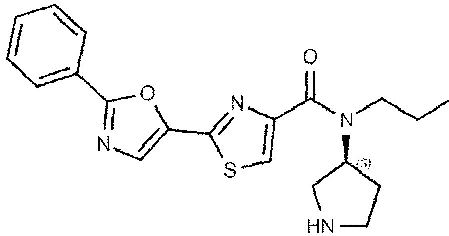 | 2-(2-phenyl-1,3-oxazol-5-yl)-N-propyl-N-[(3 S)- pyrrolidin-3-yl]-1,3- thiazole-4-carboxamide |
| 36 | 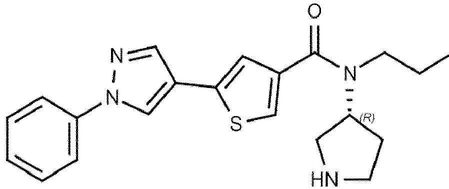 | 5-(1-phenyl-1H-pyrazol-4- yl)-N-propyl-N-[(3R)- pyrrolidin-3-yl]thiophene-3-carboxamide |
| 37 | 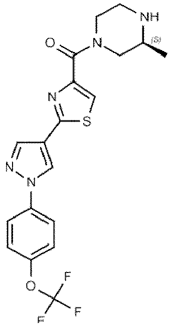 | (3S)-3-methyl-1-(2-{1-[4-(trifluoromethoxy)phenyl]-1H-pyrazol-4-yl}-1,3- thiazole-4-carbonyl)piperazine |
| 38 | 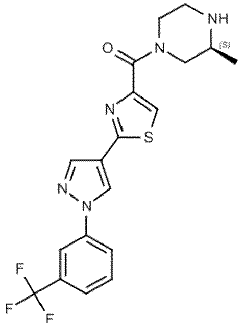 | (3S)-3-methyl-1-(2-{1-[3-(trifluoromethyl)phenyl]-1H-pyrazol-4-yl}-1,3- thiazole-4-carbonyl)piperazine |
| 39 | 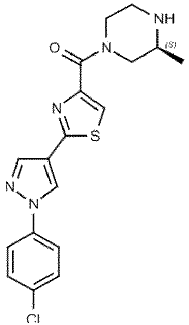 | (3S)-1-{2-[1-(4-chlorophenyl)-1H-pyrazol-4-yl]-1,3-thiazole-4- carbonyl}-3-methylpiperazine |
| 40 | 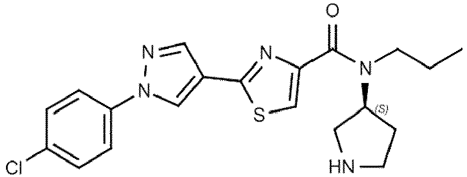 | 2-[1-(4-chlorophenyl)-1H- pyrazol-4-yl]-N-propyl-N- [(3 S)-pyrrolidin-3-yl]-1,3-thiazole-4-carboxamide |
| 41 | 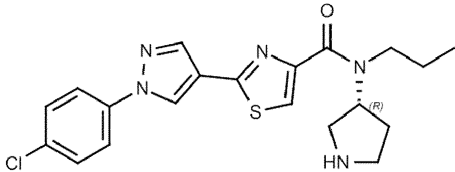 | 2-[1-(4-chlorophenyl)-1H- pyrazol-4-yl]-N-propyl-N- [(3R)-pyrrolidin-3-yl]-1,3-thiazole-4-carboxamide |
| 42 | 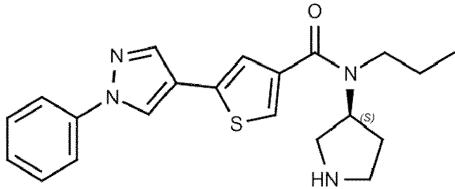 | 5-(1-phenyl-1H-pyrazol-4- yl)-N-propyl-N-[(3 S)- pyrrolidin-3-yl]thiophene-3-carboxamide |
| 43 | 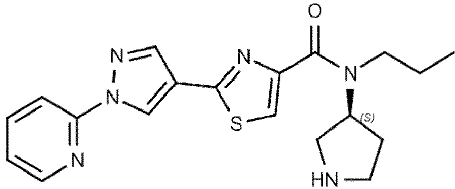 | N-propyl-2-[1-(pyridin-2-yl)-1H-pyrazol-4-yl]-N-[(3S)-pyrrolidin-3-yl]-1,3-thiazole-4-carboxamide |
| 44 | 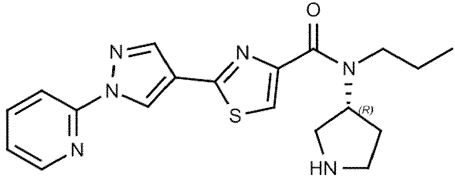 | N-propyl-2-[1 -(pyridin-2-yl)-1H-pyrazol-4-yl]-N-[(3R)-pyrrolidin-3-yl]-1,3-thiazole-4-carboxamide |
| 45 | 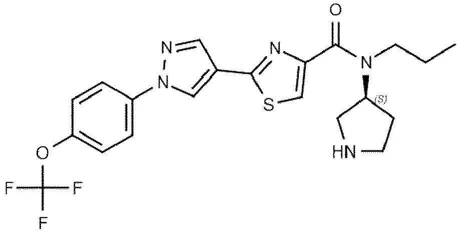 | N-propyl-N-[(3S)-pyrrolidin-3-yl]-2-{1-[4-(trifluoromethoxy)phenyl]-1H-pyrazol-4-yl}-1,3- thiazole-4-carboxamide |
| 46 | 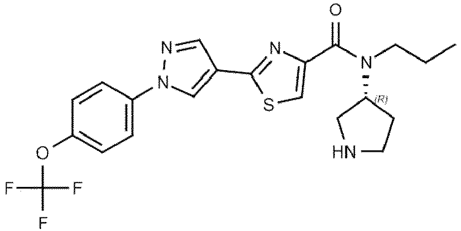 | N-propyl-N-[(3R)-pyrrolidin-3-yl]-2-{ 1-[4-(trifluoromethoxy)phenyl]-1H-pyrazol-4-yl}-1,3- thiazole-4-carboxamide |
| 47 | 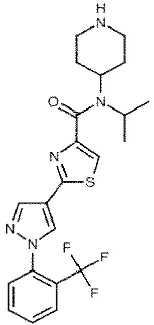 | N-(piperidin-4-yl)-N- (propan-2-yl)-2-{1-[2- (trifluoromethyl)phenyl]-1H-pyrazol-4-yl} -1,3 - thiazole-4-carboxamide |
| 48 | 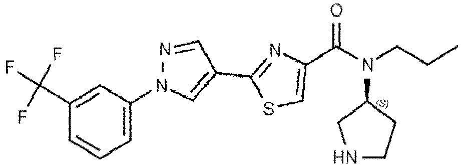 | N-propyl-N-[(3 S)-pyrrolidin-3-yl]-2-{ 1-[3-(trifluoromethyl)phenyl]-1H-pyrazol-4-yl}-1,3- thiazole-4-carboxamide |
| 49 | 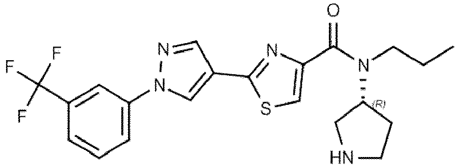 | N-propyl-N-[(3R)-pyrrolidin-3-yl]-2-{ 1-[3-(trifluoromethyl)phenyl]-1H-pyrazol-4-yl}-1,3- thiazole-4-carboxamide |
| 50 | 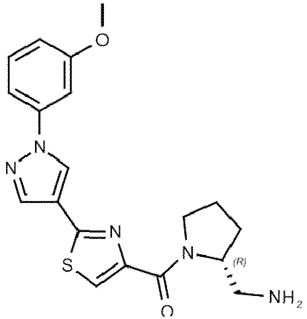 | 1-[(2R)-1-{2-[1-(3-methoxyphenyl)-1H-pyrazol-4-yl]-1,3-thiazole-4 carbonyl}pyrrolidin-2- yl]methanamine |
| 51 | 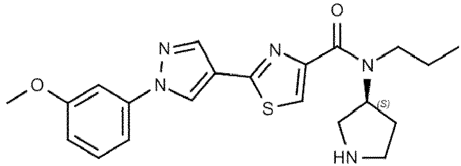 | 2-[1-(3-methoxyphenyl)- 1H-pyrazol-4-yl]-N-propyl-N-[(3S)-pyrrolidin-3-yl]-1,3-thiazole-4-carboxamide |
| 52 | 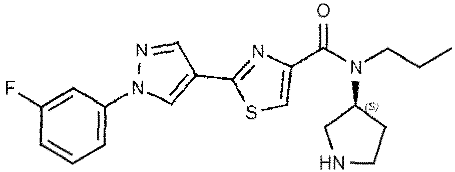 | 2-[1-(3-fluorophenyl)-1H- pyrazol-4-yl]-N-propyl-N- [(3 S)-pyrrolidin-3 -yl] -1,3-thiazole-4-carboxamide |
| 53 | 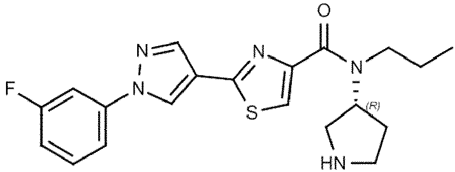 | 2-[1-(3-fluorophenyl)-1H- pyrazol-4-yl]-N-propyl-N- [(3R)-pyrrolidin-3-yl]-1,3-thiazole-4-carboxamide |
| 54 | 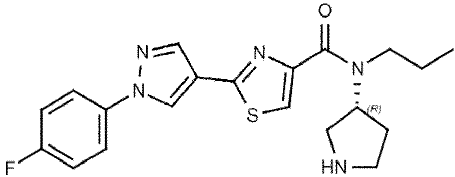 | 2-[1-(4-fluorophenyl)-1H- pyrazol-4-yl]-N-propyl-N- [(3R)-pyrrolidin-3-yl]-1,3-thiazole-4-carboxamide |
| 55 | 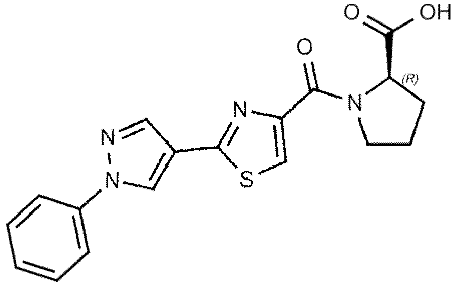 | (2R)-1-[2-(1-phenyl-1H-pyrazol-4-yl)- 1, 3 -thiazole-4 carbonyl]pyrrolidine-2- carboxylic acid |
| 56 | 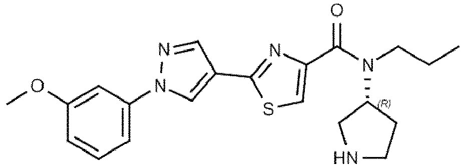 | 2-[1-(3-methoxyphenyl)- 1H-pyrazol-4-yl]-N-propyl-N-[(3R)-pyrrolidin-3-yl]-1,3-thiazole-4-carboxamide |
| 57 | 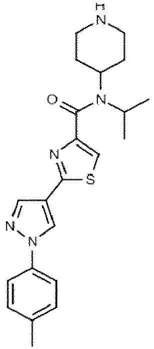 | 2-[1-(4-methylphenyl)-1H-pyrazol-4-yl]-N-(piperidin-4-yl)-N-(propan-2-yl)-1,3-thiazole-4-carboxamide |
| 58 | 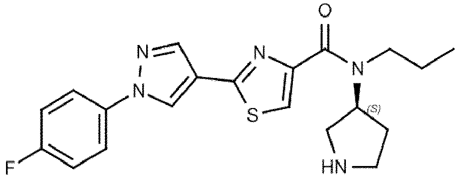 | 2-[1-(4-fluorophenyl)-1H- pyrazol-4-yl]-N-propyl-N- [(3 S)-pyrrolidin-3 -yl] -1,3-thiazole-4-carboxamide |
| 59 | 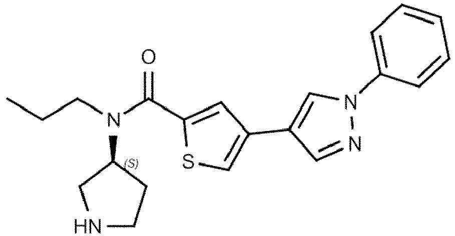 | 4-(1-phenyl-1H-pyrazol-4- yl)-N-propyl-N-[(3 S)- pyrrolidin-3-yl]thiophene-2-carboxamide |
| 60 | 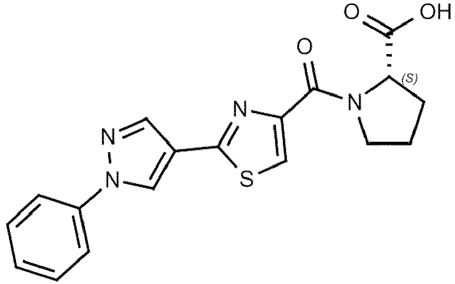 | (2S)-1-[2-(1-phenyl-1H-pyrazol-4-yl)- 1, 3 -thiazole-4 carbonyl]pyrrolidine-2- carboxylic acid |
| 61 | 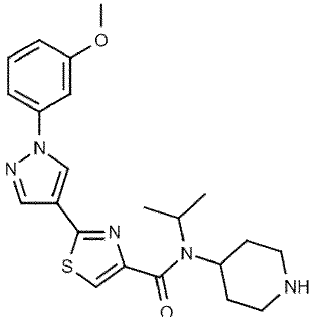 | 2-[1-(3-methoxyphenyl)-1H-pyrazol-4-yl]-N- (piperidin-4-yl)-N-(propan-2-yl)-1,3-thiazole-4- carboxamide |
| 62 | 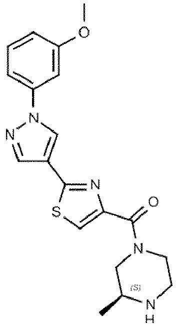 | (3S)-1-{2-[1-(3-methoxyphenyl)-1H- pyrazol-4-yl]-1,3-thiazole-4carbonyl}-3- methylpiperazine |
| 63 | 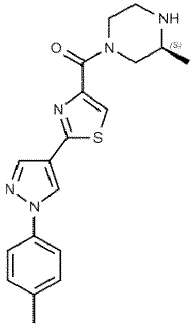 | (3S)-3-methyl-1-{2-[1-(4-methylphenyl)-1H-pyrazol-4-yl]-1,3-thiazole-4- carbonyl}piperazine |
| 64 | 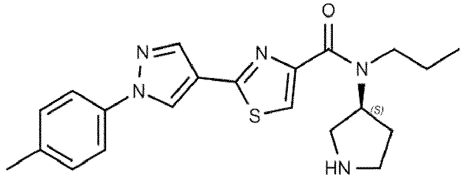 | 2-[1-(4-methylphenyl)-1H-pyrazol-4-yl]-N-propyl-N-[(3S)-pyrrolidin-3-yl]-1,3-thiazole-4-carboxamide |
| 65 | 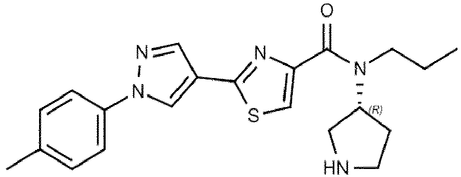 | 2-[1-(4-methylphenyl)-1H- pyrazol-4-yl]-N-propyl-N- [(3R)-pyrrolidin-3-yl]-1,3-thiazole-4-carboxamide |
| 66 | 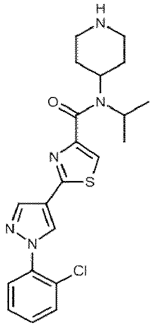 | 2-[1-(2-chlorophenyl)-1H-pyrazol-4-yl]-N-(piperidin-4-yl)-N-(propan-2-yl)-1,3-thiazole-4-carboxamide |
| 67 | 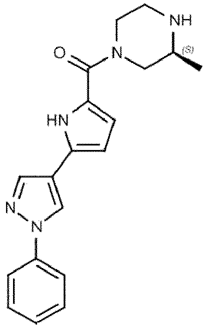 | (3S)-3-methyl-1-[5-(1-phenyl-1H-pyrazol-4-yl)-1H-pyrrole-2-carbonyl]piperazine |
| 68 | 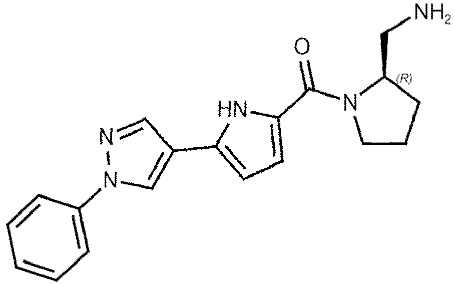 | 1-[(2R)-1-[5-(1-phenyl-1H-pyrazol-4-yl)-1H-pyrrole-2-carbonyl]pyrrolidin-2- yl]methanamine |
| 69 | 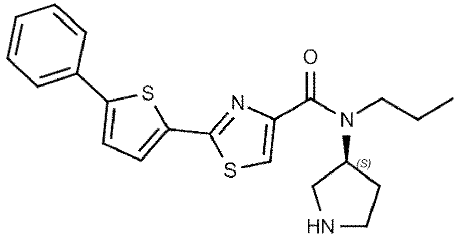 | 2-(5-phenylthiophen-2-yl)-N-propyl-N-[(3S)- pyrrolidin-3-yl]-1,3- thiazole-4-carboxamide |
| 70 | 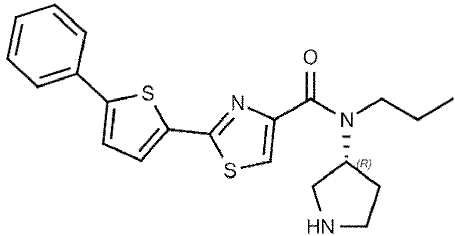 | 2-(5-phenylthiophen-2-yl)-N-propyl-N-[(3R)- pyrrolidin-3-yl]-1,3- thiazole-4-carboxamide |
| 71 | 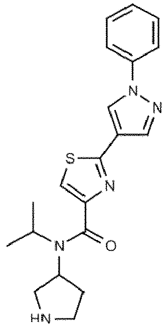 | 2-(1-phenyl-1H-pyrazol-4-yl)-N-(propan-2-yl)-N-(pyrrolidin-3-yl)-1,3- thiazole-4-carboxamide |
| 72 | 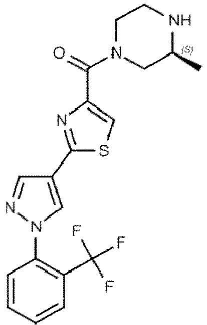 | (3S)-3-methyl-1-(2-{1-[2-(trifluoromethyl)phenyl]-1H-pyrazol-4-yl}-1,3- thiazole-4-carbonyl)piperazine |
| 73 | 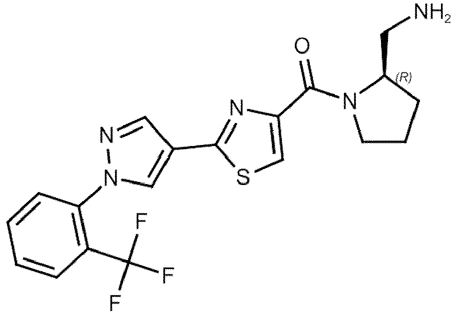 | 1-[(2R)-1-(2-{1-[2-(trifluoromethyl)phenyl]- 1H-pyrazol-4-yl}-1,3- thiazole-4-carbonyl)pyrrolidin-2-yl]methanamine |
| 74 | 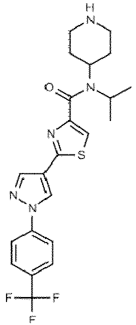 | N-(piperidin-4-yl)-N- (propan-2-yl)-2-{1-[4- (trifluoromethyl)phenyl]-1H-pyrazol-4-yl}-1,3- thiazole-4-carboxamide |
| 75 | 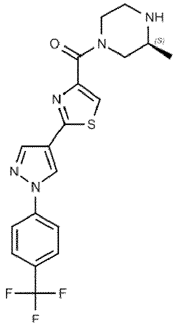 | (3S)-3-methyl-1-(2-{1-[4-(trifluoromethyl)phenyl]- 1H-pyrazol-4-yl}-1,3- thiazole-4-carbonyl)piperazine |
| 76 | 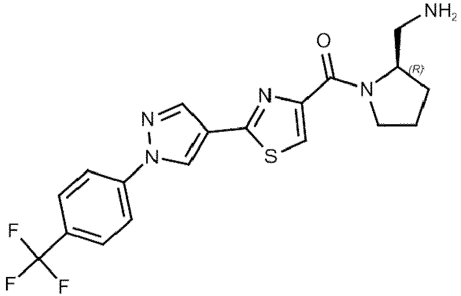 | 1-[(2R)-1-(2-{1-[4-(trifluoromethyl)phenyl]- 1H-pyrazol-4-yl}-1,3- thiazole-4-carbonyl)pyrrolidin-2-yl]methanamine |
| 77 | 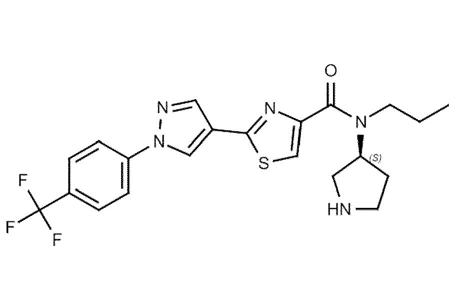 | N-propyl-N-[(3S)-pyrrolidin-3-yl]-2-{1-[4-(trifluoromethyl)phenyl]-1H-pyrazol-4-yl}-1,3- thiazole-4-carboxamide |
| 78 | 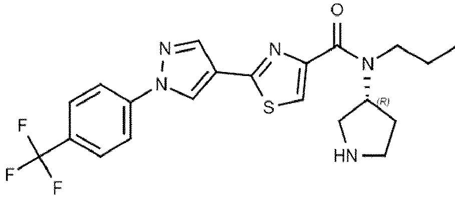 | N-propyl-N-[(3R)-pyrrolidin-3-yl]-2-{1-[4-(trifluoromethyl)phenyl]-1H-pyrazol-4-yl}-1,3- thiazole-4-carboxamide |
| 79 | 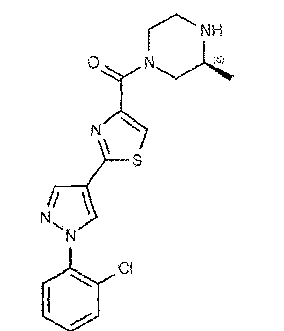 | (3S)-1-{2-[1-(2-chlorophenyl)-1H-pyrazol-4-yl]-1,3-thiazole-4- carbonyl}-3-methylpiperazine |
| 80 | 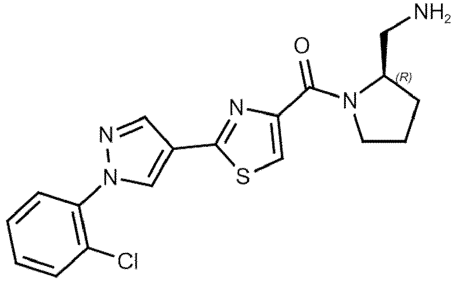 | 1-[(2R)-1-{2-[1-(2-chlorophenyl)-1H-pyrazol-4-yl]-1,3-thiazole-4- carbonyl}pyrrolidin-2-yl]methanamine |
| 81 | 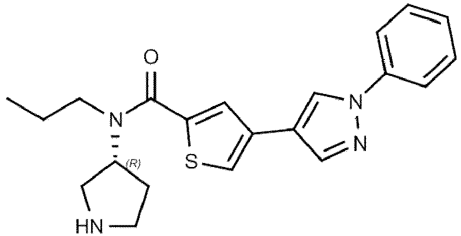 | 4-(1-phenyl-1H-pyrazol-4- yl)-N-propyl-N-[(3R)- pyrrolidin-3-yl]thiophene-2-carboxamide |
| 82 | 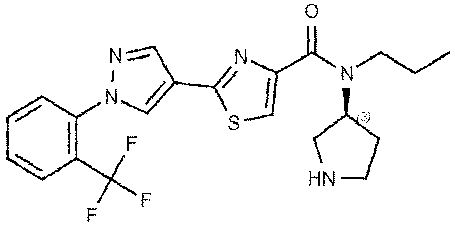 | N-propyl-N-[(3 S)-pyrrolidin-3-yl]-2-{1-[2-(trifluoromethyl)phenyl]-1H-pyrazol-4-yl}-1,3- thiazole-4-carboxamide |
| 83 | 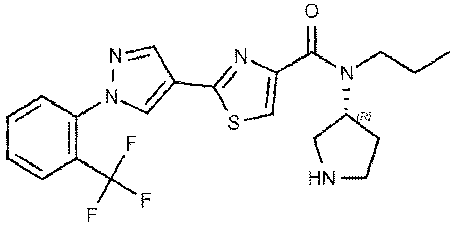 | N-propyl-N-[(3R)-pyrrolidin-3-yl]-2-{ 1-[2-(trifluoromethyl)phenyl]-1H-pyrazol-4-yl}-1,3- thiazole-4-carboxamide |
| 84 | 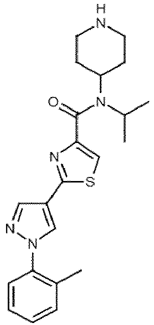 | 2-[1-(2-methylphenyl)-1H-pyrazol-4-yl]-N-(piperidin-4-yl)-N-(propan-2-yl)-1,3-thiazole-4-carboxamide |
| 85 | 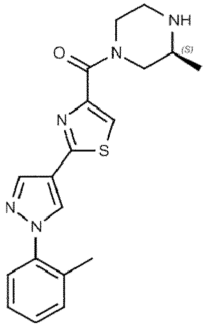 | (3S)-3-methyl-1-{2-[1-(2-methylphenyl)-1H-pyrazol-4-yl]-1,3-thiazole-4- carbonyl}piperazine |
| 86 | 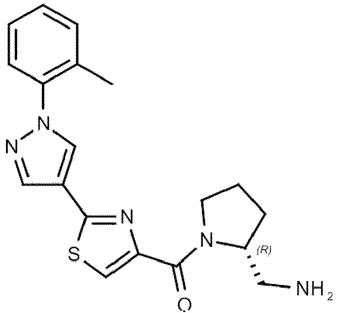 | 1-[(2R)-1-{2-[1-(2-methylphenyl)-1H-pyrazol-4-yl]-1,3-thiazole-4- carbonyl}pyrrolidin-2-yl]methanamine |
| 87 | 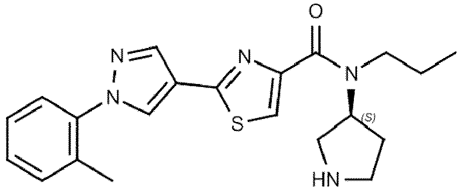 | 2-[1-(2-methylphenyl)-1H-pyrazol-4-yl]-N-propyl-N-[(3S)-pyrrolidin-3-yl]-1,3-thiazole-4-carboxamide |
| 88 | 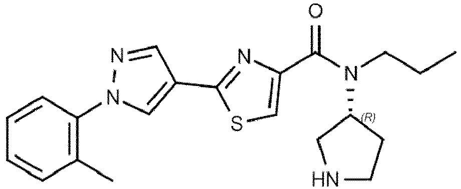 | 2-[1-(2-methylphenyl)-1H- pyrazol-4-yl]-N-propyl-N- [(3R)-pyrrolidin-3-yl]-1,3-thiazole-4-carboxamide |
| 89 | 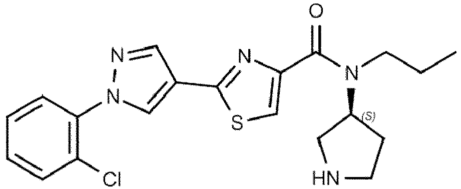 | 2-[1-(2-chlorophenyl)-1H- pyrazol-4-yl]-N-propyl-N- [(3S)-pyrrolidin-3-yl]-1,3-thiazole-4-carboxamide |
| 90 | 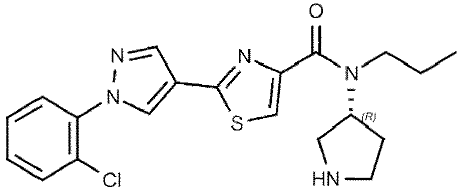 | 2-[1-(2-chlorophenyl)-1H- pyrazol-4-yl]-N-propyl-N- [(3R)-pyrrolidin-3-yl]-1,3-thiazole-4-carboxamide |
| 91 | 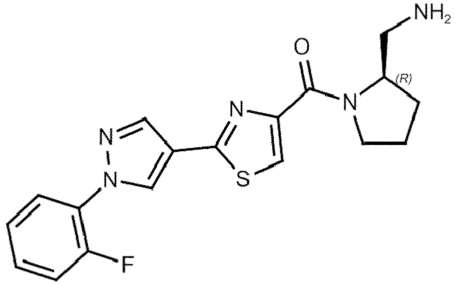 | 1-[(2R)-1-{2-[1-(2-fluorophenyl)-1H-pyrazol-4yl]-1,3-thiazole-4- carbonyl}pyrrolidin-2-yl]methanamine |
| 92 | 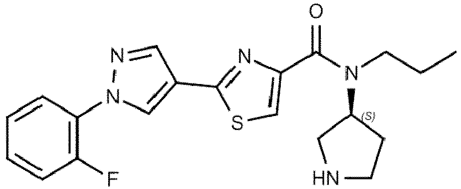 | 2-[1-(2-fluorophenyl)-1H- pyrazol-4-yl]-N-propyl-N- [(3 S)-pyrrolidin-3 -yl] -1,3-thiazole-4-carboxamide |
| 93 | 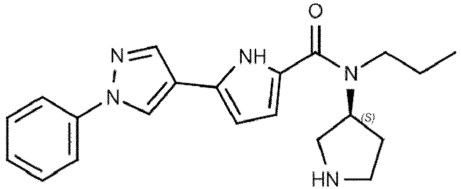 | 5-(1-phenyl-1H-pyrazol-4- yl)-N-propyl-N-[(3S)- pyrrolidin-3-yl]-1H-pyrrole-2-carboxamide |
| 94 | 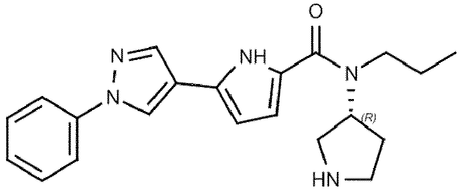 | 5-(1-phenyl-1H-pyrazol-4- yl)-N-propyl-N-[(3R)- pyrrolidin-3-yl]-1H-pyrrole-2-carboxamide |
| 95 | 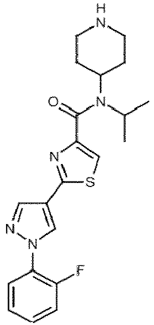 | 2-[1-(2-fluorophenyl)-1H- pyrazol-4-yl]-N-(piperidin-4-yl)-N-(propan-2-yl)-1,3-thiazole-4-carboxamide |
| 96 | 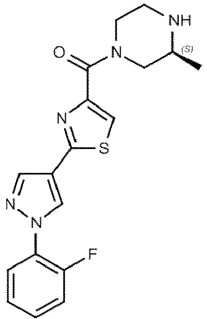 | (3S)-1-{2-[1-(2-fluorophenyl)-1H-pyrazol-4yl]-1,3-thiazole-4- carbonyl}-3-methylpiperazine |
| 97 | 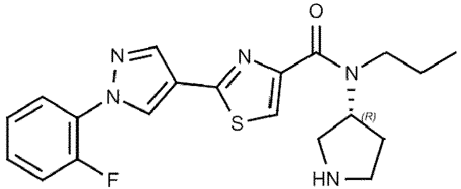 | 2-[1-(2-fluorophenyl)-1H- pyrazol-4-yl]-N-propyl-N- [(3R)-pyrrolidin-3-yl]-1,3-thiazole-4-carboxamide |
| 98 | 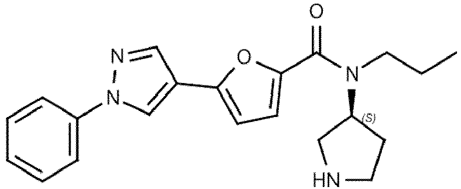 | 5-(1-phenyl-1H-pyrazol-4-yl)-N-propyl-N-[(3S)- pyrrolidin-3-yl]furan-2- carboxamide |
| 99 | 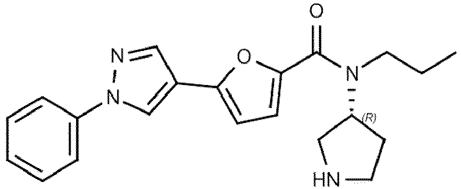 | 5-(1-phenyl-1H-pyrazol-4-yl)-N-propyl-N-[(3R)- pyrrolidin-3-yl]furan-2-carboxamide |
| 100 | 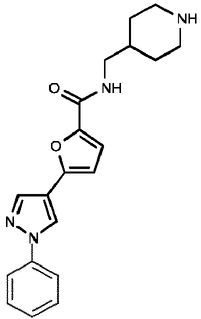 | 5-(1-phenyl-1H-pyrazol-4-yl)-N-[(piperidin-4- yl)methyl]furan-2-carboxamide |
| 101 | 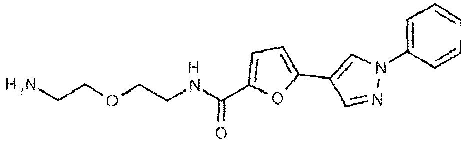 | N-[2-(2-aminoethoxy)ethyl]-5-(1-phenyl-1H-pyrazol-4- yl)furan-2-carboxamide |
| 102 | 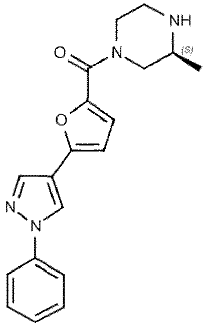 | (3S)-3-methyl-1-[5-(1-phenyl-1H-pyrazol-4-yl)furan-2-carbonyl]piperazine |
| 103 | 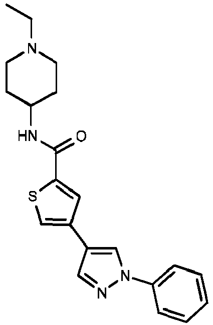 | N-(1-ethylpiperidin-4-yl)-4-(1-phenyl-1H-pyrazol-4- yl)thiophene-2- carboxamide |
| 104 | 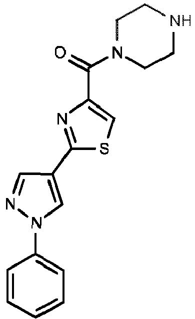 | 1-[2-(1-phenyl-1H-pyrazol-4-yl)-1,3-thiazole-4-carbonyl]piperazine |
| 105 | 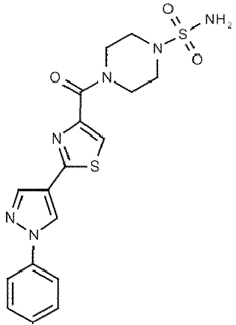 | 4-[2-(1-phenyl-1H-pyrazol-4-yl)-1,3-thiazole-4- carbonyl]piperazine-1-sulfonamide |
| 106 | 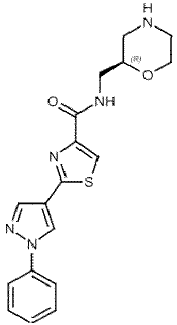 | N-{[(2R)-morpholin-2-yl]methyl}-2-(1-phenyl-1H- pyrazol-4-yl)-1,3-thiazole-4carboxamide |
| 107 | 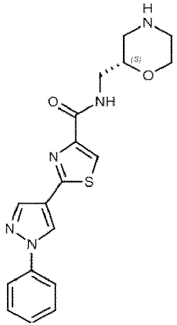 | N-{[(2S)-morpholin-2-yl]methyl}-2-(1-phenyl-1H- pyrazol-4-yl)-1,3-thiazole-4carboxamide |
| 108 | 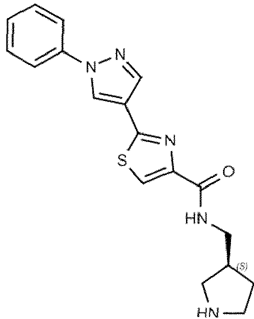 | 2-(1-phenyl-1H-pyrazol-4- yl)-N-{[(3S)-pyrrolidin-3-yl]methyl}-1,3-thiazole-4-carboxamide |
| 109 | 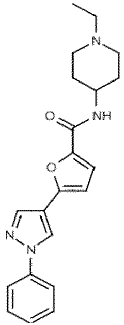 | N-(1-ethylpiperidin-4-yl)-5-(1-phenyl-1H-pyrazol-4- yl)furan-2-carboxamide |
| 110 | 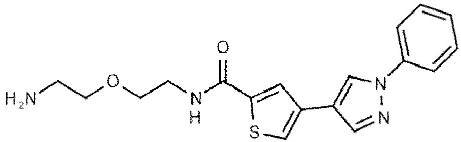 | N-[2-(2-aminoethoxy)ethyl]-4-(1-phenyl-1H-pyrazol-4- yl)thiophene-2- carboxamide |
| 111 | 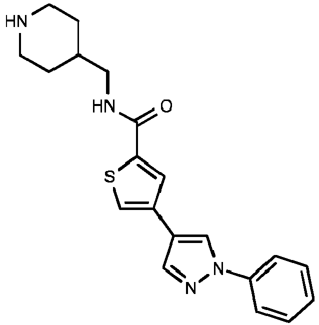 | 4-(1-phenyl-1H-pyrazol-4-yl)-N-[(piperidin-4- yl)methyl]thiophene-2-carboxamide |
| 112 | 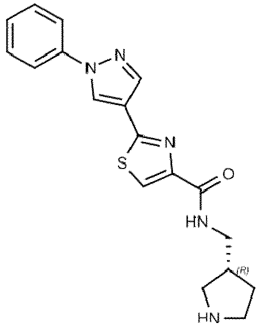 | 2-(1-phenyl-1H-pyrazol-4- yl)-N-{[(3R)-pyrrolidin-3 - yl]methyl} -1,3-thiazole-4-carboxamide |
| 113 | 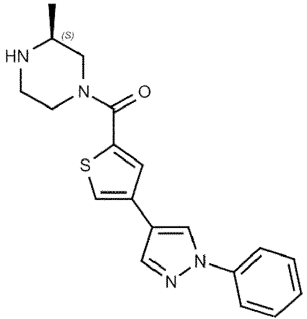 | (3S)-3-methyl-1-[4-(1-phenyl-1H-pyrazol-4-yl)thiophene-2-carbonyl]piperazine |
| 114 | 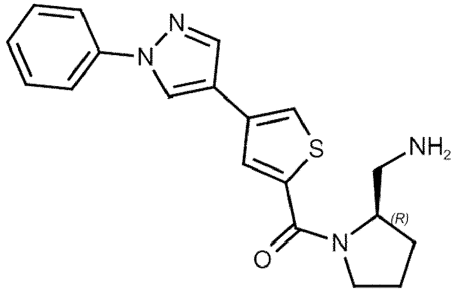 | 1-[(2R)-1-[4-(1-phenyl-1H-pyrazol-4-yl)thiophene-2-carbonyl]pyrrolidin-2- yl]methanamine |
| 115 | 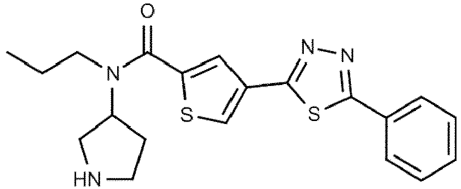 | 4-(5-phenyl-1,3,4-thiadiazol-2-yl)-N-propyl-N-(pyrrolidin-3- yl)thiophene-2-carboxamide |
| 116 | 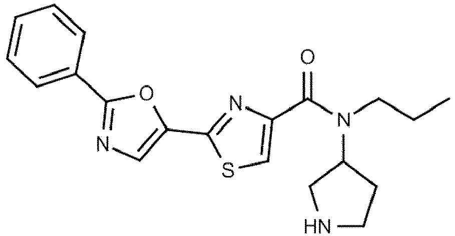 | 2-(2-phenyl-1,3-oxazol-5- yl)-N-propyl-N-(pyrrolidin-3-yl)-1,3-thiazole-4-carboxamide |
| 117 | 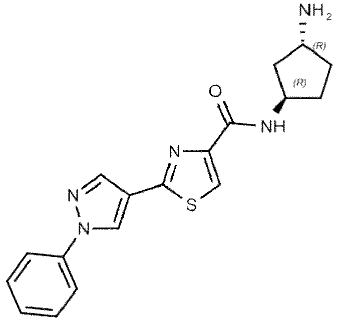 | N-[(1R,3R)-3-aminocyclopentyl]-2-(1- phenyl-1H-pyrazol-4-yl)- 1,3-thiazole-4-carboxamide |
| 118 | 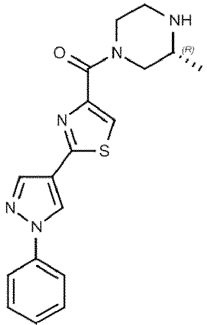 | (3R)-3-methyl-1-[2-(1-phenyl-1H-pyrazol-4-yl)-1,3-thiazole-4-carbonyl]piperazine |
| 119 | 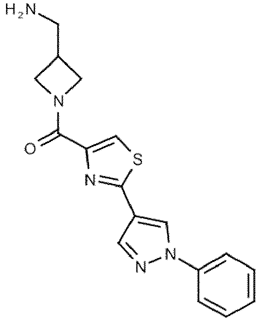 | 1-{1-[2-(1-phenyl-1H-pyrazol-4-yl)-1,3-thiazole-4 carbonyl]azetidin-3- yl}methanamine |
| 120 | 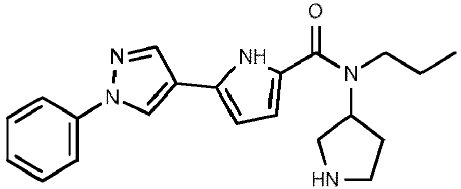 | 5-(1-phenyl-1H-pyrazol-4- yl)-N-propyl-N-(pyrrolidin-3-yl)-1H-pyrrole-2- carboxamide |
| 121 | 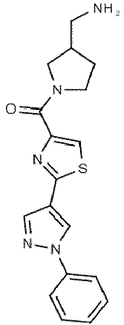 | 1-{1-[2-(1-phenyl-1H-pyrazol-4-yl)-1,3-thiazole-4 carbonyl]pyrrolidin-3- yl}methanamine |
| 122 | 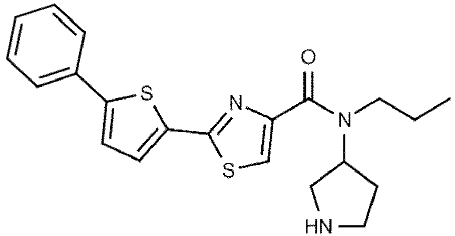 | 2-(5-phenylthiophen-2-yl)-N-propyl-N-(pyrrolidin-3-yl)-1,3-thiazole-4-carboxamide |
| 123 | 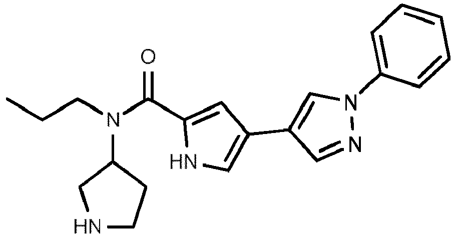 | 4-(1-phenyl-1H-pyrazol-4-yl)-N-propyl-N-(pyrrolidin-3-yl)-1H-pyrrole-2- carboxamide |
| 124 | 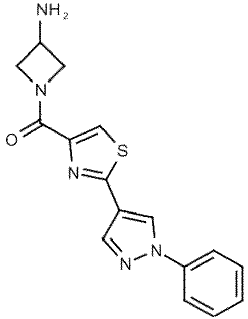 | 1-[2-(1-phenyl-1H-pyrazol-4-yl)-1,3-thiazole-4- carbonyl]azetidin-3-amine |
| 125 | 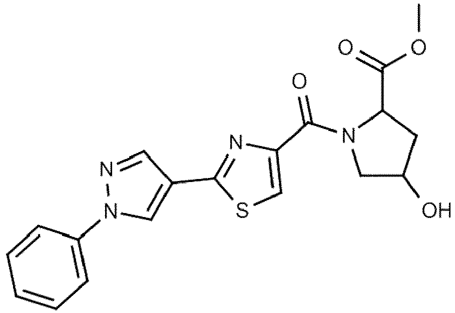 | methyl 4-hydroxy-1-[2-(1-phenyl-1H-pyrazol-4-yl)- 1,3-thiazole-4-carbonyl]pyrrolidine-2- carboxylate |
| 126 | 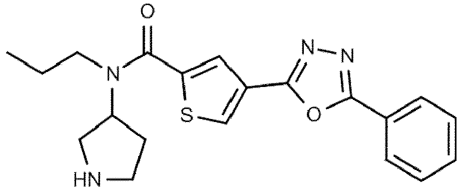 | 4-(5-phenyl-1,3,4- oxadiazol-2-yl)-N-propyl-N(pyrrolidin-3-yl)thiophene- 2-carboxamide |
| 127 | 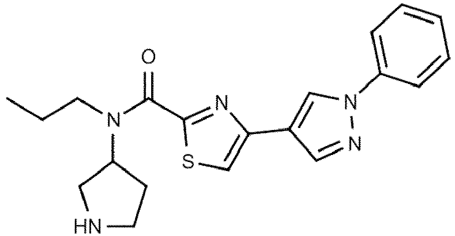 | 4-(1-phenyl-1H-pyrazol-4- yl)-N-propyl-N-(pyrrolidin-3-yl)-1,3-thiazole-2-carboxamide |
| 128 | 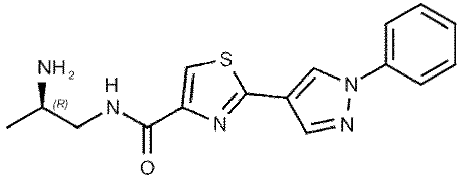 | N-[(2R)-2-aminopropyl]-2- (1-phenyl-1H-pyrazol-4-yl)-1,3-thiazole-4-carboxamide |
| 129 | 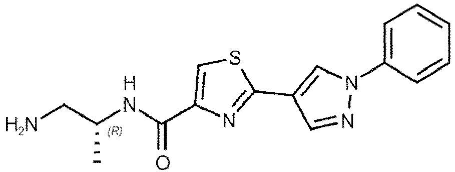 | N-[(2R)-1-aminopropan-2- yl]-2-(1-phenyl-1H-pyrazol-4-yl)-1,3-thiazole-4-carboxamide |
| 130 | 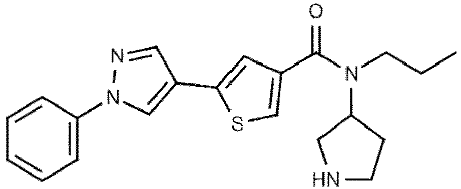 | 5-(1-phenyl-1H-pyrazol-4- yl)-N-propyl-N-(pyrrolidin-3-yl)thiophene-3- carboxamide |
| 131 | 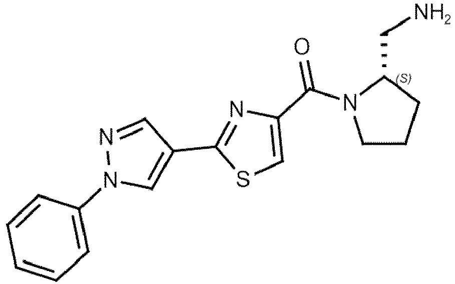 | 1-[(2S)-1-[2-(1-phenyl-1H-pyrazol-4-yl)- 1, 3 -thiazole-4 carbonyl]pyrrolidin-2- yl]methanamine |
| 132 | 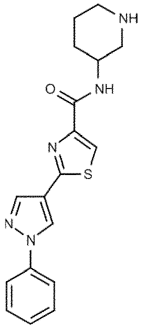 | 2-(1-phenyl-1H-pyrazol-4- yl)-N-(piperidin-3-yl)-1,3-thiazole-4-carboxamide |
| 133 | 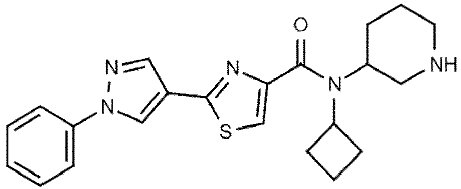 | N-cyclobutyl-2-(1-phenyl-1H-pyrazol-4-yl)-N-(piperidin-3 -yl)- 1, 3 - thiazole-4-carboxamide |
| 134 | 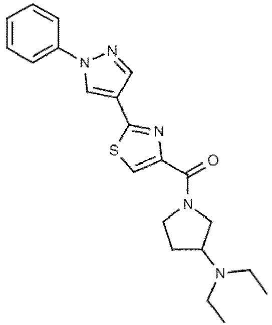 | N,N-diethyl-1-[2-(1-phenyl-1H-pyrazol-4-yl)-1,3-thiazole-4-carbonyl]pyrrolidin-3 -amine |
| 135 | 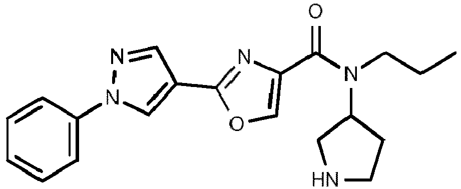 | 2-(1-phenyl-1H-pyrazol-4- yl)-N-propyl-N-(pyrrolidin-3-yl)-1,3-oxazole-4-carboxamide |
| 136 | 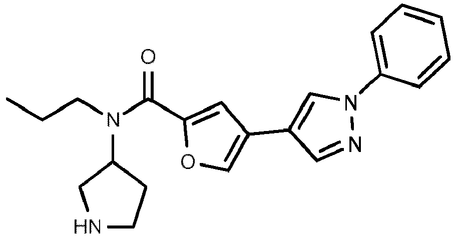 | 4-(1-phenyl-1H-pyrazol-4- yl)-N-propyl-N-(pyrrolidin-3-yl)furan-2-carboxamide |
| 137 | 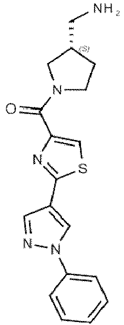 | 1-[(3S)-1-[2-(1-phenyl-1H-pyrazol-4-yl)-1,3-thiazole-4 carbonyl]pyrrolidin-3- yl]methanamine |
| 138 | 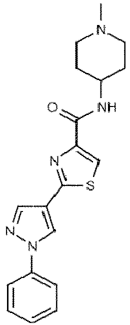 | N-(1-methylpiperidin-4-yl)-2-(1-phenyl-1H - pyrazol-4- yl)-1,3-thiazole-4- carboxamide |
| 139 | 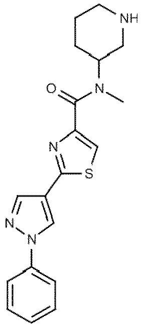 | N-methyl-2-(1-phenyl-1H-pyrazol-4-yl)-N-(piperidin-3-yl)-1,3-thiazole-4- carboxamide |
| 140 | 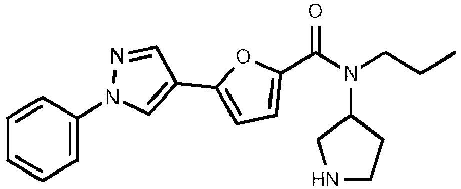 | 5-(1-phenyl-1H-pyrazol-4- yl)-N-propyl-N-(pyrrolidin-3-yl)furan-2-carboxamide |
| 141 | 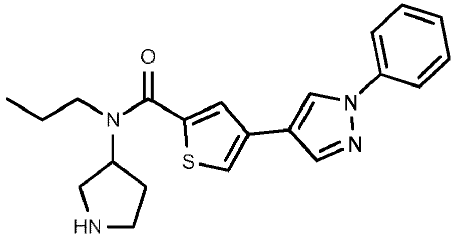 | 4-(1-phenyl-1H-pyrazol-4- yl)-N-propyl-N-(pyrrolidin-3-yl)thiophene-2- carboxamide |
| 142 | 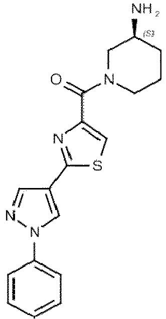 | (3S)-1-[2-(1-phenyl-1H-pyrazol-4-yl)-1,3-thiazole-4 carbonyl]piperidin-3-amine |
| 143 | 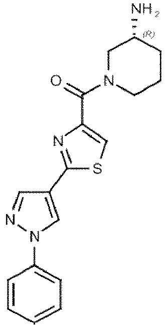 | (3R)-1-[2-(1-phenyl-1H-pyrazol-4-yl)-1,3-thiazole-4 carbonyl]piperidin-3-amine |
| 144 | 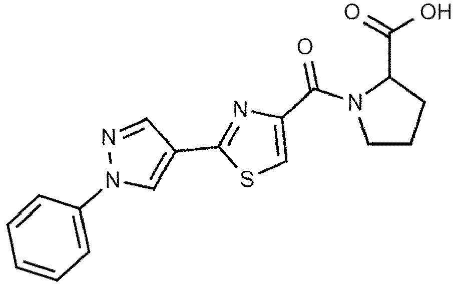 | 1-[2-(1-phenyl-1H-pyrazol-4-yl)-1,3-thiazole-4-carbonyl]pyrrolidine-2-carboxylicacid |
| 145 | 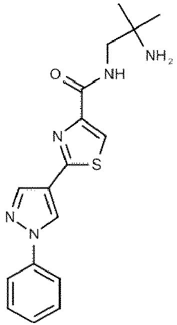 | N-(2-amino-2- methylpropyl)-2-(1-phenyl-1H-pyrazol-4-yl)-1,3- thiazole-4-carboxamide |
| 146 | 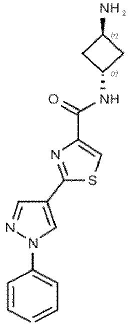 | 2-(1-phenyl-1H-pyrazol-4-yl)-N-[(1r,3r)-3-aminocyclobutyl]-1,3- thiazole-4-carboxamide |
| 147 | 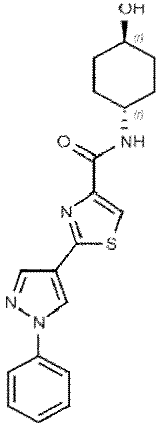 | 2-(1-phenyl-1H-pyrazol-4-yl)-N-[(1r,4r)-4-hydroxycyclohexyl]-1,3-thiazole-4-carboxamide |
| 148 | 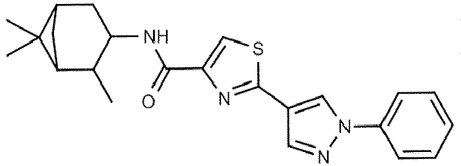 | 2-(1-phenyl-1H-pyrazol-4-yl)-N-{2,6,6-trimethylbicyclo[3.1.1]heptan-3-yl}-1,3-thiazole-4- carboxamide |
| 149 | 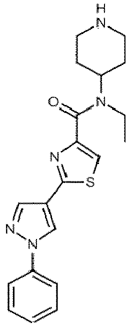 | N-ethyl-2-(1-phenyl-1H- pyrazol-4-yl)-N-(piperidin-4-yl)-1,3-thiazole-4-carboxamide |
| 150 | 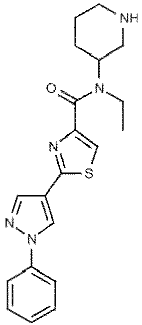 | N-ethyl-2-(1-phenyl-1H- pyrazol-4-yl)-N-(piperidin-3-yl)-1,3-thiazole-4-carboxamide |
| 151 | 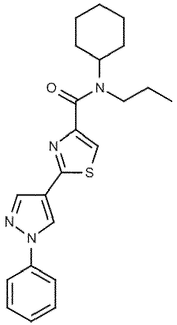 | N-cyclohexyl-2-(1-phenyl- 1H-pyrazol-4-yl)-N-propyl-1,3-thiazole-4-carboxamide |
| 152 | | methyl({1-[2-(1-phenyl-1H-pyrazol-4-yl)-1,3-thiazole-4carbonyl]pyrrolidin-2-yl}methyl)amine |
| 153 | 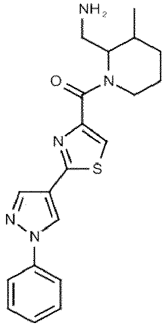 | 1-{3-methyl-1-[2-(1-phenyl-1H-pyrazol-4-yl)-1,3- thiazole-4- carbonyl]piperidin-2-yl}methanamine |
| 154 | 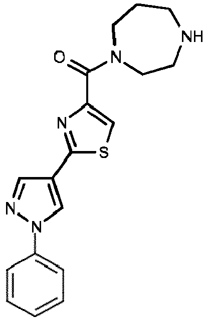 | 1-[2-(1-phenyl-1H-pyrazol-4-yl)-1,3-thiazole-4- carbonyl]-1,4-diazepane |
| 155 | 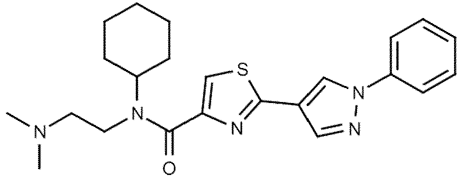 | N-cyclohexyl-N-[2-(dimethylamino-phenyl-1H-pyrazol-4-yl)- 1,3-thiazole-4-carboxamide |
| 156 | 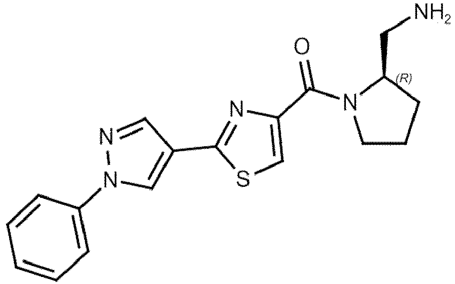 | 1-[(2R)-1-[2-(1-phenyl-1H-pyrazol-4-yl)-1,3-thiazole-4 carbonyl]pyrrolidin-2- yl]methanamine |
| 157 | 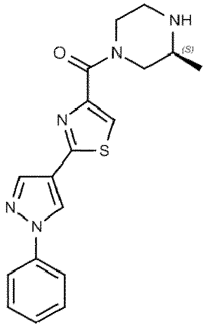 | (3S)-3-methyl-1-[2-(1-phenyl-1H-pyrazol-4-yl)-1,3-thiazole-4-carbonyl]piperazine |
| 158 | 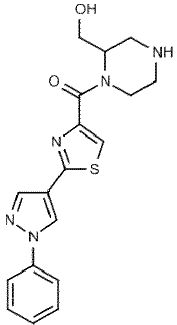 | {1-[2-(1-phenyl-1H-pyrazol 4-yl)-1,3-thiazole-4-carbonyl]piperazin-2-yl}methanol |
| 159 | 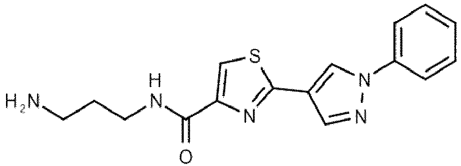 | N-(3-aminopropyl)-2-(1- phenyl-1H-pyrazol-4-yl)- 1,3-thiazole-4-carboxamide |
| 160 | 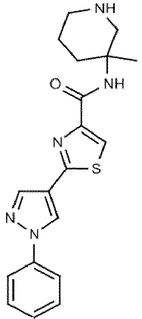 | N-(3-methylpiperidin-3-yl)-2-(1-phenyl-1H-pyrazol-4- yl)-1,3-thiazole-4- carboxamide |
| 161 | 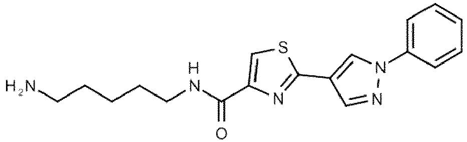 | N-(5-aminopentyl)-2-(1- phenyl-1H-pyrazol-4-yl)- 1,3-thiazole-4-carboxamide |
| 162 | | N-(2-aminoethyl)-2-(1- phenyl-1H-pyrazol-4-yl)- 1,3-thiazole-4-carboxamide |
| 163 | 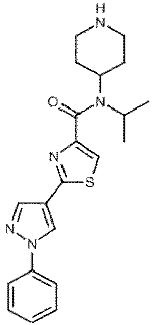 | 2-(1-phenyl-1H-pyrazol-4-yl)-N-(piperidin-4-yl)-N- (propan-2-yl)-1,3-thiazole-4-carboxamide |
| 164 | 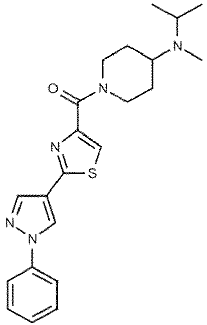 | N-methyl-1-[2-(1-phenyl-1H-pyrazol-4-yl)-1,3- thiazole-4-carbonyl]-N- (propan-2-yl)piperidin-4-amine |
| 165 | 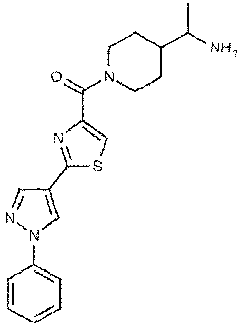 | 1-{1-[2-(1-phenyl-1H-pyrazol-4-yl)-1,3-thiazole-4 carbonyl]piperidin-4- yl}ethan-1-amine |
| 166 | 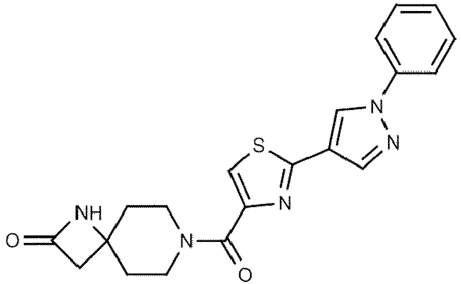 | 7-[2-(1-phenyl-1H-pyrazol-4-yl)-1,3-thiazole-4- carbonyl]-1,7-diazaspiro[3.5]nonan-2-one |
| 167 | 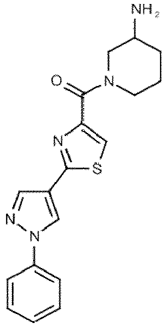 | 1-[2-(1-phenyl-1H-pyrazol-4-yl)-1,3-thiazole-4- carbonyl]piperidin-3-amine |
| 168 | 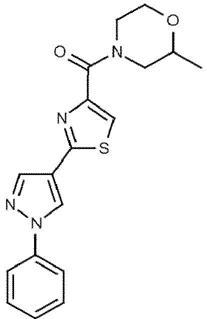 | 2-methyl-4-[2-(1-phenyl-1H-pyrazol-4-yl)-1,3-thiazole-4-carbonyl]morpholine |
| 169 | 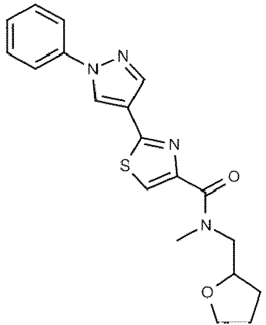 | N-methyl-N-[(oxolan-2-yl)methyl]-2-(1-phenyl- 1H- pyrazol-4-yl)-1,3-thiazole-4carboxamide |
| 170 | 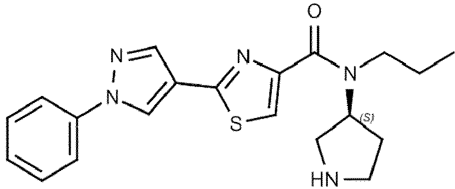 | 2-(1-phenyl-1H-pyrazol-4-yl)-N-propyl-N-[(3S)- pyrrolidin-3-yl]-1,3- thiazole-4-carboxamide |
| 171 | 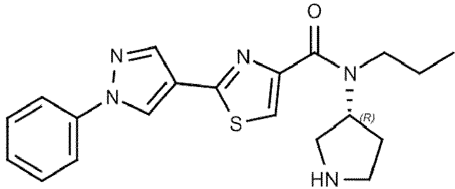 | 2-(1-phenyl-1H-pyrazol-4-yl)-N-propyl-N-[(3R)- pyrrolidin-3-yl]-1,3- thiazole-4-carboxamide |
| 172 | 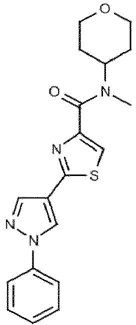 | N-methyl-N-(oxan-4-yl)-2- (1-phenyl-1H-pyrazol-4-yl)-1,3-thiazole-4-carboxamide |
| 173 | 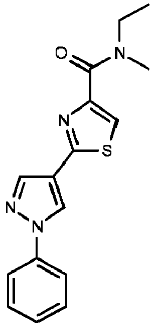 | N-ethyl-N-methyl-2-(1- phenyl-1H-pyrazol-4-yl)- 1,3-thiazole-4-carboxamide |
| 174 | 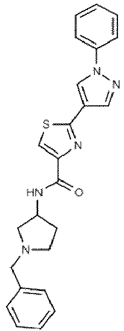 | N-(1-benzylpyrrolidin-3-yl)-2-(1-phenyl-1H-pyrazol-4- yl)-1,3-thiazole-4- carboxamide |
| 175 | 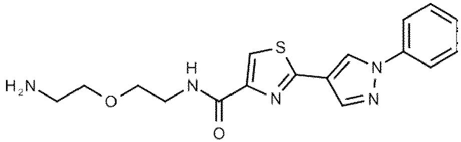 | N-[2-(2-aminoethoxy)ethyl]-2-(1- phenyl-1H-pyrazol-4-yl)- 1,3-thiazole-4-carboxamide |
| 176 | 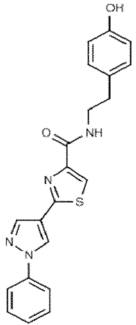 | N-[2-(4-hydroxyphenyl)ethyl]-2-(1-phenyl-1H-pyrazol-4-yl)- 1,3-thiazole-4-carboxamide |
| 177 | 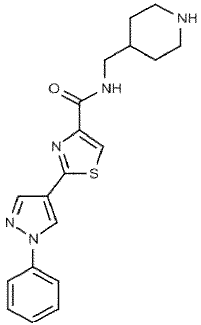 | 2-(1-phenyl-1H-pyrazol-4-yl)-N- [(piperidin-4-yl)methyl] -1,3 -thiazole-4-carboxamide |
| 178 | 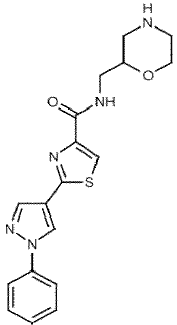 | N-[(morpholin-2- yl)methyl]-2-(1-phenyl-1H-pyrazol-4-yl)-1,3-thiazole-4carboxamide |
| 179 | 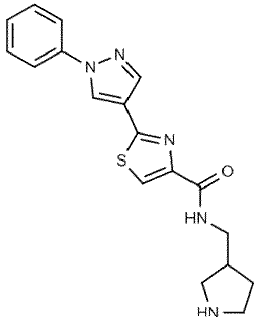 | 2-(1-phenyl-1H-pyrazol-4-yl)-N-[(pyrrolidin-3-yl)methyl] -1,3 -thiazole-4-carboxamide |
| 180 | 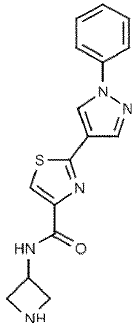 | N-(azetidin-3-yl)-2-(1- phenyl-1H-pyrazol-4-yl)- 1,3-thiazole-4-carboxamide |
| 181 | 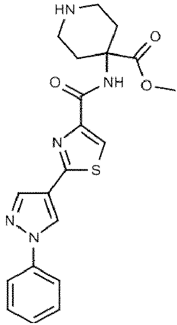 | methyl 4-[2-(1-phenyl-1H- pyrazol-4-yl)-1,3-thiazole-4 amido]piperidine-4- carboxylate |
| 182 | 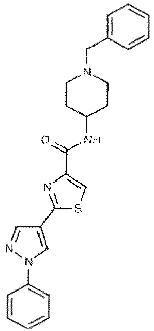 | N-(1-benzylpiperidin-4-yl)-2-(1-phenyl-1H-pyrazol-4- yl)-1,3-thiazole-4- carboxamide |
| 183 | 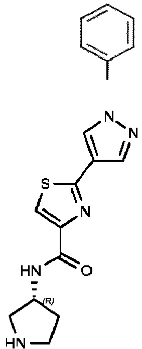 | 2-(1-phenyl-1H-pyrazol-4- yl)-N-[(3R)-pyrrolidin-3-yl] 1,3-thiazole-4-carboxamide |
| 184 | 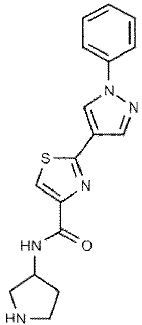 | 2-(1-phenyl-1H-pyrazol-4- yl)-N-(pyrrolidin-3 -yl)- 1, 3 - thiazole-4-carboxamide |
| 185 | 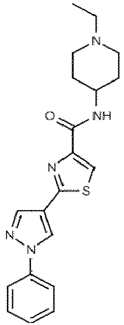 | N-(1-ethylpiperidin-4-yl)-2- (1-phenyl-1H-pyrazol-4-yl)-1,3-thiazole-4-carboxamide |
| 186 | 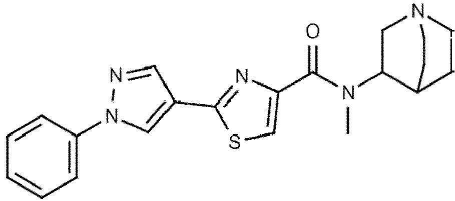 | N-{1-azabicyclo[2.2.2]octan-3- yl}-N-methyl-2-(1-phenyl-1H-pyrazol-4-yl)-1,3- thiazole-4-carboxamide |
| 187 | 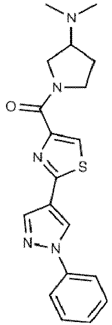 | N,N-dimethyl-1-[2-(1- phenyl-1H-pyrazol-4-yl)-1,3-thiazole-4- carbonyl]pyrrolidin-3- amine |
| 188 | 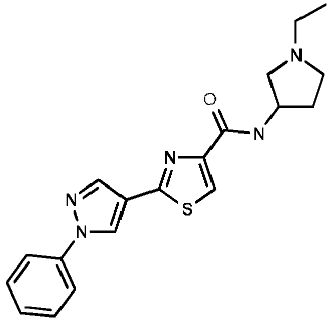 | N-(1-ethylpyrrolidin-3-yl)- N-methyl-2-(1-phenyl-1H- pyrazol-4-yl)-1,3-thiazole-4 carboxamide |
| 189 | 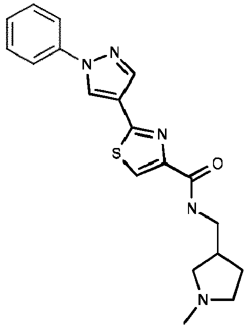 | N-methyl-N-[(1- methylpyrrolidin-3-yl)methyl]-2-(1-phenyl-1H-pyrazol-4-yl)-1,3-thiazole-4carboxamide |
| 190 | 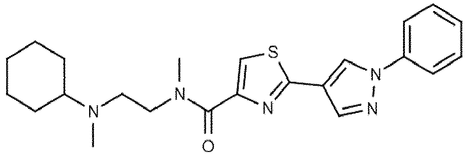 | N-{2-[cyclohexyl(methyl)amino]ethyl}-N-methyl-2-(1- phenyl-1H-pyrazol-4-yl)- 1,3-thiazole-4-carboxamide |
| 191 | 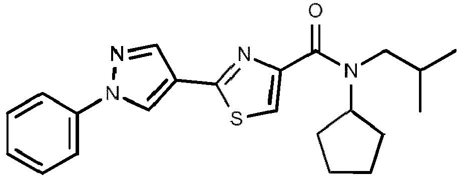 | N-cyclopentyl-N-(2- methylpropyl)-2-(1-phenyl-1H-pyrazol-4-yl)-1,3- thiazole-4-carboxamide |
| 192 | | N-cyclopentyl-N-ethyl-2-(1-phenyl- 1H-pyrazol-4-yl)- 1,3-thiazole-4-carboxamide |
| 193 | 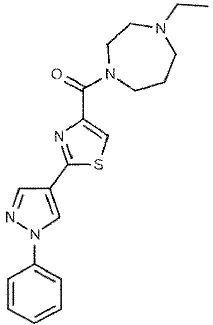 | 1-ethyl-4-[2-(1-phenyl-1H- pyrazol-4-yl)-1,3 -thiazole-4carbonyl]-1,4-diazepane |
| 194 | | N-cyclopentyl-N- cyclopropyl-2-(1-phenyl-1H-pyrazol-4-yl)-1,3- thiazole-4-carboxamide |
| 195 | 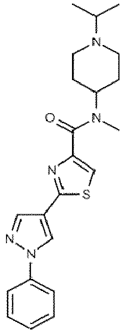 | N-methyl-2-(1-phenyl-1H- pyrazol-4-yl)-N-[1-(propan-2-yl)piperidin-4-yl]-1,3 -thiazole-4-carboxamide |
| 196 | 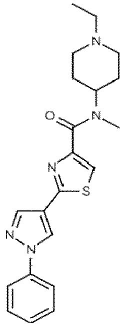 | N-(1-ethylpiperidin-4-yl)-N-methyl-2-(1-phenyl-1H- pyrazol-4-yl)-1,3-thiazole-4 carboxamide |
| 197 | 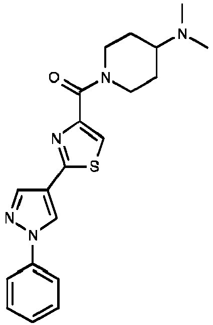 | N,N-dimethyl-1-[2-(1- phenyl-1H-pyrazol-4-yl)- 1,3-thiazole-4- carbonyl]piperidin-4-amine |
| 198 | 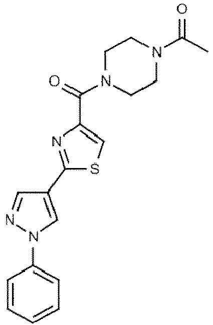 | 1-{4-[2-(1-phenyl-1H-pyrazol-4-yl)- 1, 3 -thiazole-4 carbonyl]piperazin-1- yl}ethan-1-one |
| 199 | 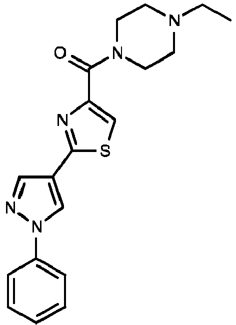 | 1-ethyl-4-[2-(1-phenyl-1H- pyrazol-4-yl)-1,3-thiazole-4carbonyl]piperazine |
| 200 | 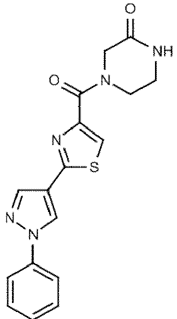 | 4-[2-(1-phenyl-1H-pyrazol-4-yl)-1,3-thiazole-4- carbonyl]piperazin-2-one |
| 201 | 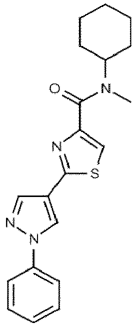 | N-cyclohexyl-N-methyl-2- (1-phenyl-1H-pyrazol-4-yl)-1,3-thiazole-4-carboxamide |
| 202 | 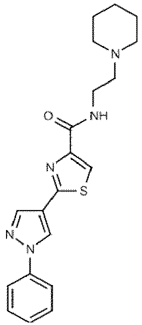 | 2-(1-phenyl-1H-pyrazol-4-yl)-N-[2-(piperidin-1 - yl)ethyl]-1,3-thiazole-4- carboxamide |
| 203 | 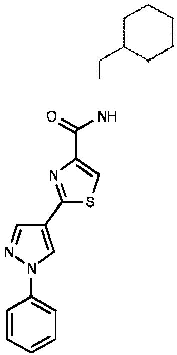 | N-(cyclohexylmethyl)-2-(1-phenyl-1H-pyrazol-4-yl)- 1,3-thiazole-4-carboxamide |
| 204 | 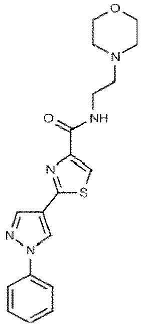 | N-[2-(morpholin-4-yl)ethyl]-2-(1-phenyl-1H- pyrazol-4-yl)-1,3-thiazole-4 carboxamide |
| 205 | 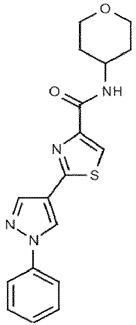 | N-(oxan-4-yl)-2-(1-phenyl-1H-pyrazol-4-yl)-1,3- thiazole-4-carboxamide |
| 206 | 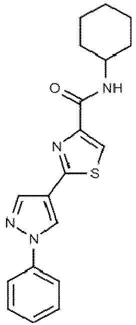 | N-cyclohexyl-2-(1-phenyl-1H-pyrazol-4-yl)-1,3- thiazole-4-carboxamide |
| 207 | 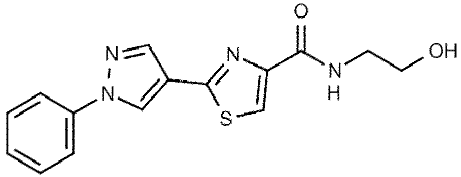 | N-(2-hydroxyethyl)-2-(1- phenyl-1H-pyrazol-4-yl)- 1,3-thiazole-4-carboxamide |
| 208 | 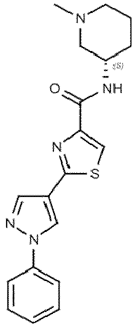 | N-[(3S)-1-methylpiperidin-3-yl]-2-(1-phenyl-1H- pyrazol-4-yl)-1,3-thiazole-4 carboxamide |
| 209 | 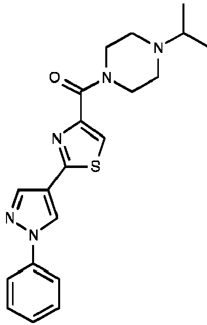 | 1-[2-(1-phenyl-1H-pyrazol-4-yl)-1,3-thiazole-4- carbonyl]-4-(propan-2-yl)piperazine |
| 210 | 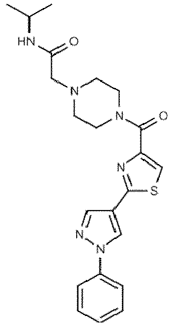 | 2-{4-[2-(1-phenyl-1H-pyrazol-4-yl)-1,3-thiazole-4 carbonyl]piperazin-1-yl}-N-(propan-2-yl)acetamide |

### Biological assays and data

As stated above, the compounds of the present invention induce and/or stimulate autophagy and are useful in treating autophagy-related diseases. The biological activity of the compounds of the present invention can be determined by any appropriate test to determine the ability to induce and/or stimulate autophagy.

### Assessment of stimulation of autophagy

Lysosomes play a fundamental role in the autophagic pathway by fusing with autophagosomes and creating 'autolysosomes' in order to digest their contents. Stimulation of lysosome and autolysosome formation by a compound is indicative of a stimulation of autophagy. The ability of compounds to stimulate lysosome and autolysosome formation, and thus autophagy, in live cells was assessed via fluorescent microscopy using various fluorescent stains for labelling and tracking acidic organelles (including lysosomes and autolysosomes) such as: LysoView^{™} 650 (70059 and 70059-T, Biotium), LysoViewTM633 (70058 and 70058-T, Biotium) and LysoTracker^{™} Deep Red (L12492, ThermoFisher Scientific). The cellular phenotype was quantitatively assessed for the induction of acidic vesicle formation and compared to a non-treated control, thus providing a measure of the ability of the compound under investigation to stimulate autophagy.

Representative procedure using LysoView^{™}633 dye or LysoTracker^{™} Deep Red dye: Human osteosarcoma U2OS cells (40,000 cells/well) were seeded in a 24 well glass bottom plate (Sensoplate, Greiner Bio-One) and were incubated overnight in a humidified atmosphere at 37°C and 5% CO2. Cells were grown in DMEM (Gibco) supplemented with 10% Fetal Bovine Serum (FBS) and 100 units/ml penicillin and 100 µg/ml streptomycin (Invitrogen). After the attachment period, cells were treated with different compounds of interest (at various concentrations in DMSO) or DMSO (non-treated control) in cell culture medium and were incubated for 24 hours. Compound-containing medium was removed and cells were incubated with pre-warmed cell culture medium containing 1x LysoView^{™} 633 (70058 and 70058-T, Biotium) or 50 nM LysoTrackerT^{M} Deep Red (L12492, ThermoFisher Scientific) for 45 minutes at 37°C. Finally, cell nuclei were stained for 10 minutes using Hoechst 33342 (1 µg/mL) and then the medium was replaced with fresh medium. The 24 well plate was then fitted into a heated stage on the microscope and cells maintained at 37°C. Images were captured using appropriate filter set for Cy5 and DAPI detection with the EVOS M7000 Microscope (ThermoFisher Scientific). The cellular phenotype was visually assessed for the induction of acidic vesicle formation relative to the non-treated control; multiple images were acquired and analysed using ImageJ or Cell Profiler.

All of compounds 1-210 according to the invention, as well as the preferred racemic mixture of compounds 170 and 171 showed increased acidic vesicle formation in human osteosarcoma U2OS cells relative to a DMSO-treated control (non-treated control) at a concentration of 10 µM. In this assay, acidic vesicle formation was increased by at least 25 % for compounds 1-210 and for the racemic mixture of compounds 170 and 171. This is evidence for a significant stimulation of autophagy by these compounds under the conditions as tested.

Selected compounds of this invention were also tested and showed increased acidic vesicle formation in human osteosarcoma U2OS cells relative to a DMSO-treated control (non-treated control) at a concentration of 0.8 µM. This is evidence for significant stimulation of autophagy by the compounds of this invention, and particularly by these compounds under the conditions as tested. Table 2 shows this data for the selected compounds. Compounds having an activity designated as "+" provided a percentage increase in acidic vesicle formation of between 10% and 30% over the DMSO-treated control. Compounds having an activity designated as "++" provided a percentage increase in acidic vesicle formation of between 30% and 50% over the DMSO-treated control. Compounds having an activity designated as "+++" provided a percentage increase in acidic vesicle formation of between 50% and 100% over the DMSO-treated control. Compounds having an activity designated as "++++" provided a percentage increase in acidic vesicle of greater than 100% over the DMSO-treated control. Preferred compounds are shown in Table 2a, more preferred compounds are shown in Table 2b.

**Table 2**

| | | | | | |
|---|---|---|---|---|---|
| **Compound No.** | **Activity** | **Compound No.** | **Activity** | **Compound No.** | **Activity** |
| 5 | ++ | 46 | ++ | 76 | + |
| 11 | + | 47 | ++ | 82 | ++ |
| 16 | + | 48 | +++ | 83 | ++ |
| 17 | + | 49 | ++ | 84 | + |
| 20 | + | 50 | +++ | 86 | ++ |
| 23 | + | 51 | ++ | 87 | + |
| 24 | + | 52 | + | 89 | + |
| 25 | + | 53 | + | 90 | + |
| 26 | ++ | 54 | + | 92 | +++ |
| 27 | + | 55 | + | 93 | ++++ |
| 28 | + | 56 | + | 94 | ++++ |
| 29 | ++ | 57 | ++ | 95 | +++ |
| 31 | + | 58 | + | 96 | ++ |
| 35 | + | 59 | + | 97 | ++ |
| 36 | + | 60 | + | 98 | +++ |
| 37 | + | 63 | + | 99 | +++ |
| 40 | + | 65 | ++ | 100 | ++ |
| 41 | + | 66 | + | 101 | ++ |
| 42 | + | 67 | + | 102 | ++ |
| 43 | + | 69 | ++ | 103 | ++ |
| 44 | + | 70 | ++ | 104 | + |
| 45 | ++ | 72 | ++ | 107 | + |

**Table 2 (continued)**

| **Compound No.** | **Activity** | **Compound No.** | **Activity** |
|---|---|---|---|
| 109 | + | 170 | + |
| 110 | +++ | 171 | ++ |
| 111 | ++ | 177 | + |
| 116 | +++ | 179 | ++ |
| 120 | +++ | 180 | ++ |
| 122 | ++++ | 183 | + |
| 123 | ++++ | 185 | +++ |
| 124 | +++ | 190 | + |
| 125 | ++ | 196 | ++ |
| 130 | + | 198 | + |
| 140 | + | 202 | ++ |
| 141 | +++ | 204 | + |
| 142 | + | 208 | + |
| 143 | + | | |
| 144 | ++ | | |
| 148 | + | | |
| 153 | + | | |
| 155 | + | | |
| 159 | ++ | | |
| 162 | + | | |
| 163 | + | | |
| 167 | + | | |

**Table 2a Preferred compounds**

| **Compound No.** | **Activity** |
|---|---|
| 110 | +++ |
| 116 | +++ |
| 120 | +++ |
| 122 | ++++ |
| 123 | ++++ |
| 124 | +++ |
| 141 | +++ |
| 185 | +++ |
| 48 | +++ |
| 50 | +++ |
| 92 | +++ |
| 93 | ++++ |
| 94 | ++++ |
| 95 | +++ |
| 98 | +++ |
| 99 | +++ |

**Table 2b More preferred compounds**

| **Compound No.** | **Activity** |
|---|---|
| 122 | ++++ |
| 123 | ++++ |
| 93 | ++++ |
| 94 | ++++ |

Certain compounds of this invention also showed increased acidic vesicle formation in other cell types, relative to a DMSO-treated control (non-treated control). For example, the racemic mixture of compounds 170 and 171 showed significantly greater acidic vesicle formation in adult human dermal fibroblast cells (HDFa cells) relative to a DMSO-treated control at a concentration of 10 µM. This is evidence for significant stimulation of autophagy by this compound under these conditions.

Based on these results this is evidence that these compounds induce autophagy. Additional evidence that this activity will be therapeutic in various autophagy-related diseases are disclosed in the following examples.

### Assessment of activity in models of various diseases and conditions

### Example 1

### Demonstration of activity in an induced pluripotent stem cell (iPSC) model of Glycogen Storage Disease type 1A (GSD1A) derived from human patient cells.

The racemic mixture of compounds 170 and 171 was selected as an example compound of this invention to be evaluated in this model.

***Timetable for treatment of cells with the compounds:***
**Day 1.** Differentiated hepatocyte GSD1A disease model cells (DefiniGEN Ltd) were cultured in a collagen I coated 48-well plate. Empty wells were filled with PBS.
**Day 9.** The racemic mixture of compounds 170 and 171 (as a solution in DMSO) was added to the disease model cells
**Day 11.** Media for disease model cells was replaced with fresh media with compound.
**Day 12.** Cells were washed once in PBS and once in HBSS and 30 µL of NP40 Lysis buffer supplied with Phosphatase and Protease inhibitor cocktail was added to each well. Plate was immediately placed on dry ice.

***Lysate preparation:*** Cell lysates were thawed and 30 µL of RIPA buffer supplied with Phosphatase and Protease inhibitor cocktail was added to each.

***Protein quantification:*** An aliquot of the lysates was used for protein quantification using BCA.

***Glycogen quantification:*** An aliquot of the lysates was boiled at 96 °C for 5 minutes. Boiled lysates were then centrifuged at 1000 xg for 5 minutes to remove precipitates. Supernatant was used for Glycogen quantification using MAK016 kit (Merck), following manufacturer's instructions. Briefly, in 2 aliquots 2 µL of each supernatant was transferred to a half-area 96-well plate. 8 µL of Hydrolysis buffer from the kit was added to one aliquot from each sample (-HE; to quantify free glucose content of the lysate). The other sample was supplied with 8 µL of Hydrolysis buffer supplied with Hydrolysis Enzyme (+HE; to quantify free glucose + glycogen content of the lysate). After 30 minutes of incubation at room temperature, 10 µL of probe mix was added to each well and the absorbance at 570 nm was measured after 30 minutes incubation at room temperature. A570 readings were first adjusted based on the blank readings. Glycogen content of the cells was estimated by subtracting -HE readings from +HE readings, and the values were normalised to DMSO only control. The quantification experiment was performed twice.

***Triglyceride quantification:*** Boiled supernatants from the previous experiment were also used for Triglyceride quantification using MAK266 kit (Merck), following manufacturer's instructions. Briefly, 2 µL of each supernatant was transferred to a half-area 96-well plate. 8 µl of Lipase reaction mix from the kit was added each sample. After 20 minutes of incubation at room temperature, 10 µL of probe mix was added to each well and the absorbance at 570 nm was measured after 30 minutes incubation at room temperature. A570 readings were first adjusted based on the blank readings and the values were normalised to DMSO only control. Note that the quantification experiment was performed twice.

***Results:*** The racemic mixture of compounds 170 and 171 at a concentration of 10 µM successfully reduced cellular glycogen and triglycerides in hepatocytes differentiated from GSD1A patient derived iPSCs (by over 75% and 20%, respectively) when compared with the DMSO-only control.

***Conclusion:*** The demonstrated breakdown of glycogen in patient-derived differentiated hepatocytes strongly supports efficacy of compounds in this invention in the treatment of GSD1A. The breakdown of glycogen is a specific type of autophagy known as glycophagy.

### Example 2

### Demonstration of antiviral activity against Human Cytomegalovirus (HCMV) and Zika Virus (ZIKV)

The racemic mixture of compounds 170 and 171 was selected as an example compound of this invention to be evaluated in this model.

### Infective assay:

Cells were seeded in 96 well plates and the following day pre-treated with a 3-fold serial dilution of compound starting from 20 µM, in 100 µL of assay media. 24 hours later, cells were infected (without removing the media) with ZIKV or HCMV at a multiplicity of infection (MOI) compatible with multiple rounds of virus replication. DMSO-treated infected and uninfected cells were included as positive and negative controls. The experimental conditions for each virus are summarized in Table 3.

| | | | | | |
|---|---|---|---|---|---|
| Virus | Cell line | Assay media | MOI | Assay duration | Stained antigen |
| ZIKV | antigenSH-SY5Y | DMEM/F 12 2% FBS | 5 | 48 h | E |
| HCMV | HFF | DMEM 5% FBS | 1 | 15 days | gB |

**Table 3**. Experimental conditions used in the infectivity assays. FBS: foetal bovine serum, E: envelope, gB: glycoprotein B. MOI: multiplicity of infection (number of infectious viruses per cell). At the indicated time points, selected to allow for more than one round of virus replication, the cells were fixed and immuno-stained for virus specific antigens. Images were acquired on a Perkin Elmer Opera LTX and the percentage of infected cells was calculated using Columbus software.

### Cytotoxicity assay:

In parallel to the infectivity assays, in order to determine the cytotoxic properties of the compound, cells were seeded in 96 well plates and the following day pre-treated with the same serial dilutions of compound in 100 µl of assay media. The compound was left on the cells for the duration of the infectivity assays, after which a metabolic assay (MTT assay) was used to determine cell viability compared to a DMSO-treated control.

Results: The IC50 and TC50 values of compound 170/171 for ZIKV and HCMV are summarized in Table 4.

| | | | | |
|---|---|---|---|---|
| | HCMV | | ZIKV | |
| | IC50 | TC50 | IC50 | TC50 |
| The racemic mixture of compounds 170 and 171 | <10 | >75 | <15 | - |

**Table 4**. IC50 and TC50 values for the compound according to formula 170/171 against HCMV and ZIKV. IC50 and TC50 are reported in micromolar. A "-" symbol indicates no inhibition.

***Conclusion:*** The racemic mixture of compounds 170 and 171 was found to demonstrate antiviral activity against both HCMV and ZIKV over a range of concentrations less than 20 µM (IC50 <10 µM and <15 µM respectively) with a limited toxicity profile. This is evidence that compounds of this invention are efficacious in the treatment of certain autophagy-related viral infections

### Example 3

The racemic mixture of compounds 170 and 171 was selected as an example compound of this invention to be evaluated in this model.

### Activity in a cellular model of NAFLD: gel assay for mutant PNPLA3-expressing HepG2 cells

***Method:*** HepG2 cells were transfected with GFP-PNPLA3 (I148>M) overexpression plasmid using Lipofectamine LTX in DMEM media supplied with 1% Oleic Acid-BSA and were left overnight. The next day, cells were trypsinated and were seeded in a 96-well plate in DMEM media supplied with 1% Oleic Acid-BSA. After 7 hours, media was changed to standard DMEM supplied with the compound of interest. After 24 hours, cells were washed once with PBS and were lysed in RIPA buffer. Lysates were collected, centrifuged and the supernatants were mixed with Laemmli buffer (without DTT or boiling) and were loaded on a 4-20% SDS-PAGE gel. After electrophoresis, gels were directly imaged in the FITC channel to visualise the GFP signal.

***Results:*** The racemic mixture of compounds 170 and 171 showed a 3.7 fold increase in GFP breakdown Bafilomycin-sensitive-product signal versus an inactive control. Therefore, the racemic mixture of compounds 170 and 171 is responsible for lysosomal breakdown of mutated PNPLA3 in this model.

The I148>M mutated form of PNPLA3 is associated with increased risk of Non-Alcohol Fatty Liver Disease [NAFLD]. Therefore, this model functions as an in vitro model of NAFLD, and it is an example of conditions and diseases relating to degradation of misfolded proteins. Activity in this model is also evidence that compounds of this invention are active versus various diseases involving misfolded proteins.

### Example 4

### Activity in a cellular model of Parkinson's disease

Compounds 144, 156, 157, 171, 185 and 196 were selected as example compounds of this invention to be evaluated in this model.

This model (run by Neuro-Sys SAS, France) is based on a primary culture of dopaminergic Tyrosine hydroxylase (TH)-positive neurons injured with the dopaminergic neurons-specific toxin (DA-toxin) 1-methyl-4-phenyl-1,2,3,6 tetrahydropyridine (MPTP, a prodrug of MPP⁺) (Dauer and Przedborski, 2003). Any substances reducing DA-toxin neurotoxicity may be useful as a new therapeutic agent for the treatment or prevention of PD.

### Methods

**Primary culture of mesencephalic neurons:** Rat dopaminergic neurons were cultured as described by Visanji et al., 2008 and Callizot et al., 2019. Briefly, pregnant female rat (Wistar) of 15 days of gestation were killed using a deep anesthesia with CO2 chamber and a cervical dislocation. The midbrains obtained from 15-day-old rat embryos (Janvier, France) were dissected under a microscope. The embryonic midbrains will be removed and placed in ice-cold medium of Leibovitz (L15) containing 2% of Penicillin-Streptomycin (PS) and 1% of bovine serum albumin (BSA). The ventral portion of the mesencephalic flexure, a region of the developing brain rich in dopaminergic neurons, was used for the cell preparations.

The midbrains were dissociated by trypsinisation for 20 min at 37°C (solution at a final concentration of 0.05% trypsin and 0.02% EDTA). The reaction was stopped by the addition of Dulbecco's modified Eagle's medium (DMEM) containing DNAase I grade II (0.5 mg/mL) and 10% of foetal calf serum (FCS). Cells were then mechanically dissociated by 3 passages through a 10 ml pipette. Cells were then centrifuged at 180 x g for 10 min at +4°C on a layer of BSA (3.5%) in L15 medium. The supernatant was discarded and the cell pellets re-suspended in a defined culture medium consisting of Neurobasal supplemented with B27 (2%), L-glutamine (2 mM) and 2% of PS solution and 10 ng/mL of Brain-derived neurotrophic factor (BDNF) and 1 ng/mL of Glial-Derived Neurotrophic Factor (GDNF). Viable cells were counted in a Neubauer cytometer using the trypan blue exclusion test. The cells were seeded at a density of 40, 000 cells/well in 96 well-plates (pre-coated with poly-L-lysine) and maintained in a humidified incubator at 37°C in 5% CO2/95% air atmosphere. Half of the medium was changed every 2 days with fresh medium. The wells of first and last lines and columns were used (to avoid any edge effect) and were be filled with sterile water.

### Test compounds and MPTP:

**i) Pre-incubation**. On day 6 of culture, the test compounds were dissolved in PBS or DMSO and incubated for 1 hour before the MPTP exposure.
**ii) Injury**. One hour after the application of test compound, MPTP was added to a final concentration of 4µM, diluted in control medium still in presence of compounds/inhibitors for 48h. Test compounds were tested on one culture in 96-well plate (n = 6 culture wells per condition).

### Endpoint evaluation:

**Immunostaining: TH and α-Synuclein**. 48 Hours after intoxication, the cell culture supernatant was removed, and the cells were fixed by a solution of 4% paraformaldehyde in PBS, pH =7.3 for 20 min at room temperature. The cells were washed twice in PBS, and then permeabilized. Non-specific sites were blocked with a solution of PBS containing 0.1% of saponin and 1% FCS for 15 min at room temperature. The cultures were incubated with: (a) monoclonal anti-Tyrosine Hydroxylase (TH) antibody produced in mouse at dilution of 1/10000 in PBS containing 1% FCS, 0.1 % saponin, for 2 hours at room temperature, and (b) polyclonal anti-alpha synuclein (a-synuclein) antibody produced in rabbit at dilution of 1/200 in PBS containing 1% FCS, 0.1 % saponin, for 2 h at room temperature. These antibodies were revealed with Alexa Fluor 488 goat anti-mouse IgG at the dilution 1/800 and with Alexa Fluor 568 goat anti-rabbit IgG at the dilution 1/400 in PBS containing 1% FCS, 0.1 % saponin, for 1 h at room temperature.

**Automatic computer analysis.** For each condition, 20 pictures (representing the whole well area) were automatically taken using ImageXpress^{®} (Molecular Devices) at 10x magnification (20 pictures, for TH and α-synuclein into TH neurons) using the same acquisition parameters. From images, analyses was directly and automatically performed by MetaXpress^{®} (Molecular Devices).

### The following read-outs were measured:

i) Analysis of total number of TH neurons (dopaminergic TH positive neurons)
ii) Total neurite network of dopaminergic TH positive neurons (in µm)
iii) α-Synuclein aggregation (overlapping between TH and α-syn staining)

Compounds 144, 156, 157, 171, 185 and 196 showed positive effects in this in vitro model of PD based on MPP+ injury.

Compound 144 significantly protected the dopaminergic neurons at a concentration of 100 nM (a 25% increase in the number of dopaminergic TH positive neurons compared to MPTP injury alone). This compound lowered α-Synuclein aggregation at concentrations of 1 µM, 500 nM and 100 nM (a >15% decrease in α-syn area per dopaminergic TH positive neuron compared to MPP+ injury alone).

Compound 156 significantly protected the dopaminergic neurons at concentrations of 10 µM, 1 µM and 500 nM (>18% increase in the number of dopaminergic TH positive neurons compared to MPP+ injury alone) and protected the neurite network at concentrations of 10 µM, 1 µM and 500 nM (>39% increase in the length of the neurite network of dopaminergic TH positive neurons compared to the MPP+ injury alone). This compound lowered α-Synuclein aggregation at concentrations of 10 µM, 1 µM, 500 nM and 100 nM (>13% decrease in α-syn area per dopaminergic TH positive neuron compared to MPP+ injury alone).

Compound 157 showed significant positive effects at a concentration of 1 µM on all the three read-outs: (i) a 27% increase in the number of dopaminergic TH postive neurons compared to MPP+ injury alone, (ii) a 35% increase in the length of the neurite network of dopaminergic TH positive neurons compared to the MPP+ injury alone, and (iii) a 21% decrease in α-syn area per dopaminergic TH positive neuron compared to MPP+ injury alone.

Compound 171 showed significant positive effects at concentrations of 500 µM and 1 µM on all three read-outs: (i) a >25% increase in the number of dopaminergic TH postive neurons compared to MPP+ injury alone, (ii) a >31% increase in the length of the neurite network of dopaminergic TH positive neurons compared to the MPP+ injury alone, and (iii) a >18% decrease in α-syn area per dopaminergic TH positive neuron compared to MPP+ injury alone.

Compound 185 displayed a large and significant neuroprotective effect at concentrations of 500 nM, 1 µM and 10 µM: (i) a >22% in increase in the number of dopaminergic TH positive neurons compared to MPP+ injury alone and (ii) a > 27% increase in the length of the neurite network of dopaminergic TH positive neurons compared to the MPP+ injury alone. This effect was correlated with a decrease of α-Synuclein aggregation (a >14% decrease in α-syn area per dopaminergic TH positive neuron compared to MPP+ injury alone was observed).

For Compound 196, a significant neuroprotective effect was observed at a concentration of 500 nM (a 26% increase in the number of dopaminergic TH postive neurons compared to MPP+ injury alone). The compound lowered α-Synuclein aggregation at concentrations of 1 µM, 500 nM and 100 nM (decreases in α-syn area per dopaminergic TH positive neuron compared to MPP+ injury alone of 19%, 35% and 26% respectively).

This assay is an *in vitro* model of Parkinson's disease. Activity in this model (positive effects) strongly support that compounds of this invention are efficacious in the treatment of Parkinson's disease and other autophagy-related neurodegenerative diseases or conditions.

### References:

Callizot N, Combes M, Henriques A, Poindron P. Necrosis, apoptosis, necroptosis, three modes of action of dopaminergic neuron neurotoxins. PLoS ONE 14(4): e0215277 Dauer W., Przedborski S. Parkinson's disease: mechanisms and models. Neuron 2003, 39(6):889-909
Visanji NP, Orsi A, Johnston TH, Howson PA, Dixon K, Callizot N, Brotchie JM and Rees DD. PYM50028, a novel, orally active, nonpeptide neurotrophic factor inducer, prevents and reverses neuronal damage induced by MPP+ in mesencephalic neurons and by MPTP in a mouse model of Parkinson's disease. FASEB J. 2008; 22(7):2488-97

### Example 5

### Demonstration of activity in a cellular model of Amyotrophic Lateral Sclerosis (ALS)

Compounds 157, 185 and 196 were selected example compounds if this invention to evaluate in this model. This model (run by Neuro-Sys SAS, France) evaluates the protective effects of test compounds on primary spinal cord lower motor neurons (MNs) from SOD1 G93A Tg rats, after a glutamate injury. The MNs are pre-treated with the test compounds for 1 hour prior to exposure to glutamate, and readouts are measured 24 hours later. This model functions as an in vitro model of Amyotrophic Lateral Sclerosis. In this model, the glutamate injury leads to: (i) a decrease in the number of MNs, (ii) a reduction in the length of the motor neuron network, and (iii) an increase in the cytoplasmic area of Transactivating response element DNA binding protein 43 kDa (TDP43) = relative to a non-injured control (TDP43 has been shown to accumulate in the cytpoplasm of motor neurons in most cases of ALS). Any substances which can attenuate one or more of these effects can be considered to have protective effects in the model and may be useful as a new therapeutic agent for the treatment or prevention of ALS.

### Methods

**Genotyping of SOD1 Tg embryos:** Pregnant female rat SOD1G93A (Sprague Dawley, Taconic), of 14 days of gestation were obtained fromTaconic Bioscience. On the day of the dissection (from pregnant females at 14 days of gestation), a piece of each embryo brain (~3 mm3) was placed in a 2 mL tube free DNase with a new scalpel. The DNA was extracted with the SYBR Green Extract-N-Amp tissue PCR kit (Sigma Aldrich). Briefly, 120 µL of extraction solution was put on each piece of embryo heads. Then, they were incubated for 10 min at room temperature. At the end of this incubation period, the heads were incubated for 5 min at 95°C. Immediately after this last incubation, 100 µL of neutralizing solution was added; each DNA extract was diluted at 1/40 and stored at +4°C until use. SOD1G93A gene was determined using genomic fragment with human SOD1 primers (5'-CATCAGCCCTAATCCATCTGA-3' (SEQ ID NO: 1); 5'- CGCGACTAACAATCAAAGTGA-3' (SEQ ID NO: 2)). The SOD1 primers were diluted at 3µM in sterile ultrapure water. Briefly, a mix for PCR was prepared with ultrapure water (4 µL per sample), primer at 3µM (2 µL per sample) and Master Mix (10 µL per sample). In a PCR 96 wells plate, 16 µL of PCR mix was added in each well. 4 µL of each diluted DNA was added according to a plan deposit. The RT-PCR was ran using the CFX96 Biorad RT-PCR system, using the following program: Initial denaturation (95°C, 20 sec)/ 45 cycles (95°C, 10 sec; 65°C, 10 sec; 72°C, 30 sec) / Melt curve (95°C, 15 sec; 64°C, 1min; 90°C, 30 sec; 60°C 15 sec). The amplification plots and melt curves were analyzed with the Biorad software. The results for each sample were compared to negative control (ultrapure water) and to the positive control (DNA from Tg embryos).

### Primary culture of spinal cord motor neurons

Rat spinal cord motor neurons (MNs) were be cultured as described by Wang et al. 2013 and Boussicault et al., 2020. Briefly, pregnant female rats of 14 days gestation (Sprague Dawley; Taconic) were killed using a deep anesthesia with CO2 chamber and a cervical dislocation. Then, fetuses were removed from the uterus and immediately placed in ice-cold L15 Leibovitz medium with a 2% penicillin (10,000 U/ml) and streptomycin (10 mg/ml) solution (PS) and 1% bovine serum albumin (BSA).

Spinal cords were treated for 20 min at 37°C with a trypsin- EDTA solution at a final concentration of 0.05% trypsin and 0.02% EDTA. The dissociation was stopped by addition of Dulbecco's modified Eagle's medium (DMEM) with 4.5 g/liter of glucose, containing DNAse I grade II (final concentration 0.5 mg/mL) and 10% fetal calf serum (FCS). Cells were mechanically dissociated by three forced passages through the tip of a 10-ml pipette. Cells were then centrifuged at 515 x g for 10 min at 4°C. The supernatant was discarded, and the pellet was resuspended in a defined culture medium consisting of Neurobasal medium with a 2% solution of B27 supplement, 2 mmol/liter of L-glutamine, 2% of PS solution, and 10 ng/mL of brain-derived neurotrophic factor (BDNF). Viable cells were counted in a Neubauer cytometer, using the trypan blue exclusion test. The cells were seeded at a density of 20,000 per well in 96-well plates precoated with poly-L-lysine and will be cultured at 37°C in an air (95%)-CO2 (5%) incubator. The medium was changed every 2 days. The wells of the first lines and columns were not used for culture (to avoid any edge effect) and were filled with sterile water. The spinal cord motor neurons were injured with glutamate after 13 days of culture.

### Test compounds and glutamate exposure:

### Vehicle: Culture medium (0.1% DMSO)

**i) Pre-incubation.** On day 13 of culture, primary motor neurons were pre-treated with the test compounds for 1 hour.
**ii) Injury.** On day 13 of culture, and after the 1 hour of pre-incubation, glutamate was added to a final concentration of 5 µM diluted in control medium still in presence of the compounds for 20 min. After 20 min, glutamate was washed out and fresh culture medium was added for an additional 24 hours.

### Test compounds were tested on cultures in 96-well plates (6 wells per condition)

### Endpoint evaluation:

**Immunostaining: MAP-2 and TDP43.** 24 hours after intoxication, the supernatants were discarded, and cells were fixed by a cold solution of ethanol (95%) and acetic acid (5%) for 5 min at -20 °C. After permeabilization with 0.1% of saponin, cells were incubated for 2 hours with: (a) a mouse monoclonal antibody anti microtubule-associated-protein 2 (MAP-2) at dilution of 1/400 in PBS containing 1% fetal calf serum and 0.1% of saponin. This antibody was revealed with Alexa Fluor 488 goat anti-mouse IgG at the dilution 1/400 in PBS containing 1% FCS, 0.1% saponin, for 1 hour at room temperature, and (b) a rabbit polyclonal antibody anti-nuclear TAR DNA-binding protein 43 (TDP-43) at dilution of 1/100 in PBS containing 1% fetal calf serum and 0.1% of saponin. The antibody TDP-43 was revealed with Alexa Fluor 568 goat anti-rabbit at a dilution of 1/400 in PBS containing 1% FCS, 0.1% saponin, for 1 hour at room temperature.

### Automatic computer analysis

For each condition, 30 pictures (representative of the all well area) per well were automatically taken using ImageXpress^{®} (Molecular Devices) with 20x magnification, using the same acquisition parameters. From images, analyses were directly and automatically performed by MetaXpress^{®} (Molecular Devices).

The following endpoints were automatically assessed:
i) Analysis of motor neuron survival (number of motor neurons),
ii) Analysis of motor neuron neurite network (neurite length in µm)
iii) Cytoplasmic area of TDP-43 in motor neurons (overlapping between MAP-2 and cytoplasmic TDP-43 in µm²).

Compounds 157, 185 and 196 showed positive effects in this in vitro model of ALS.

Compound 157 had beneficial effects upon the neurite network (a >20% increase in neurite network length compared to the glutamate injury alone case, at compound concentrations of 0.1 µM, 0.5 µM and 1 µM) and fully abolished the abnormal TDP43 accumulation in the cytoplasm normally resulting from glutamate injury (at compound concentrations of 0.1 µM, 0.5 µM,1 µM and 10 µM).

Compound 185 fully abolished the abnormal cytoplasmic TDP-43 accumulation normally resulting from glutamate injury (at compound concentrations of 0.1 µM, 0.5 µM and 1 µM).

Compound 196 improved the neuronal survival (a >10% increase in number of motor neurons compared to the glutamate injury alone case, at compound concentrations of 1 and 10 µM), protected the neurite network (a 16% increase in neurite network length compared to the glutamate injury alone case at a compound concentration of 10 µM) and fully abolished the abnormal TDP43 accumulation in the cytoplasm normally resulting from glutamate injury (at compound concentrations of 0.5 µM, 1 µM and10 µM).

This model functions as an *in vitro* model of Amyotrophic lateral sclerosis (ALS). Activity (positive effects) in this model strongly support that compounds of this invention are efficacious in the treatment of Amyotrophic lateral sclerosis and other autophagy-related neurodegenerative diseases or conditions

### References

Boussicault L, Laffaire J, Schmitt P, Rinaudo P, Callizot N, Nabirotchkin S, Hajj R, Cohen D. Combination of acamprosate and baclofen (PXT864) as a potential new therapy for amyotrophic lateral sclerosis. J Neurosci Res. 2020 Dec;98(12):2435-2450. Sakka L, Delétage N, Lalloué F, Duval A, Chazal J, Lemaire JJ, Meiniel A, Monnerie H, Gobron S. SCO-spondin derived peptide NX210 induces neuroprotection in vitro and promotes fiber regrowth and functional recovery after spinal cord injury. PLoS One. 2014 Mar 25;9(3):e93179.
Vera A, Stanic K, Montecinos H, Torrejón M, Marcellini S, Caprile T. SCO-spondin from embryonic cerebrospinal fluid is required for neurogenesis during early brain development. Front Cell Neurosci. 2013 Jun 3;7:80.
Wang W, Li L, Lin WL, Dickson DW, Petrucelli L, Zhang T, Wang X. The ALS disease-associated mutant TDP-43 impairs mitochondrial dynamics and function in motor neurons. Hum Mol Genet. 2013 Dec 1;22(23):4706-19

### Example 6

### Activity in a mouse model of Charcot-Marie-Tooth 1A (CMT1A)

Compound 163 was selected as an example compound of this invention to be evaluated in this model. This model (run by In vivex SAS, France) uses C3-PMP22 transgenic mice *(B6.Cg-Tg(PMP22)C3Fbas*/*J)* which express three copies of a wild-type human peripheral myelin protein 22 (PMP22) gene. These mice present an age-dependent demyelinating neuropathy characterized by predominantly distal loss of strength and sensation. C3-PMP mice show no overt clinical signs at 3 weeks and develop mild neuromuscular impairment in an age-dependent manner. They have stable, low nerve conduction velocities similar to adults with human CMT1A. Myelination is delayed in these mice, and they contain reduced numbers of myelinated fibres at 3 weeks of age.

### Overview of the study

### Animal characteristics:

**Sex/Species/Strain:** *B6.Cg-Tg(PMP22)C3Fbas*/*J* Male
**Age:** 3 weeks old
**Supplier:** Jackson Laboratory (USA)
**Approximate weight at initiation of treatment:** 19.0 g ± 2.5 g
**Animal number:** 6 mice per group
**Animal identification:** At the beginning of the study, animals were identified with a number on the tail. Each parameter was noted in the lab book.
**Acclimation and clinical signs:** Animals arrived on site 7 days (one week) before the experiment to allow optimal acclimation. Clinical signs and mortality were recorded daily. Body weight was determined twice a week.
**Study outline:**
**Dose:** 30 mg/kg
**Administration:** Oral gavage
**Readouts:**
   i) Rotarod (neuromuscular performance)
   ii) Sciatic nerve electrophysiology (EMG)
   iii) PMP22 analysis by western blot
   iv) Histology of sciatic nerve
**Experimental time duration:** Readouts measures at 1 and 2 months old.
   - Baseline rotarod and EMG readouts at 1 month old
   - Rotarod and EMG readouts at 2 months old
   - Western blot for PMP22 and histopathology of sciatic nerve at 2 months old
**Vehicle used for** test Compound 163: 10% DMSO/5% Cremophor RH 40 and 85% of 10% HP-β-CD in water (v/v)
**Vehicle control group:** vehicle only (non-treated control)
**Statistics:** Statistical analyses were performed using Prism software. A probability less than 5% (p<0.05) was considered as significant.

### Operating procedures for study readouts

**Sciatic nerve electrophysiology.** Electromyography was performed on mice anesthetized with ketamine/xylazine mixture. A pair of steel needle electrodes (AD Instruments, MLA1302) were placed subcutaneously along the nerve at the sciatic notch (proximal stimulation). A second pair of electrodes were placed along the tibial nerve above the ankle (distal stimulation). Supramaximal square-wave pulses, lasting 10 ms at 1 mA were delivered using a PowerLab 26T (AD Instruments). Compound muscle action potential (CMAP) was recorded from the intrinsic foot muscles using steel electrodes. Both amplitudes and latencies of CMAP were determined. The distance between the 2 sites of stimulation was measured alongside the skin surface with fully extended legs, and nerve conduction velocities (NCVs) were calculated automatically from sciatic nerve latency measurements using Excel.

**Rotarod.** A rotating rod apparatus (Bioseb, France) was used to measure neuromuscular coordination and balance. Mice were first given a pretraining trial to familiarize them with the rotating rod. Latency to fall was measured at a successively increased speed from 4 to 40 rpm over a 300-second max. time period. Each animal underwent 3 trials a day. For each day, values from the 3 trials were averaged for each animal, and then averaged for each group.

### PMP22 Western blot analysis

After electrophysiology analysis and animal sacrifice, the left sciatic nerve of all animals were sampled, solubilized in 4°C RIPA lysis buffer completed with protease inhibitors (Fisher Scientific, France), sonicated three times during 10 s on ice (Microson ultrasonic cell disruptorXL, Microsonic) and centrifuged for 30 min at 10,000 rpm at 4°C. 50 µL of the supernatant was sampled and total protein was quantified by NanoDrop using bovine serum albumin dilutions as standard. Then, ten micrograms of protein samples were denatured using Laemmli/β-mercaptoethanol (Merck, S3401) incubation at 95°C during 10 minutes, separated on a denaturing 10% SDS-polyacrylamide gel and transferred onto nitrocellulose membranes. Membranes were blocked for 60 min with 5 mL of Licor Blocking Buffer. The following primary antibodies were incubated overnight at 4°C in the same blocking buffer: rabbit anti-PMP22 (1:750, Sigma-Aldrich, SAB4502217) and mouse anti-tubulin (1:1000, Sigma Aldrich, T9026-100UL). The following day the membranes were washed 3 times for 10 minutes in TBS Tween-20 (0.1% V/V) and then incubated for 1 hour at room temperature with the secondary fluorescence antibodies: donkey anti-mouse IRDye 680 (1:10.000, LI-COR Biosciences) and donkey anti-rabbit IRDye 800 (1:10.000, LI-COR Biosciences). After secondary antibody incubation, the membranes were washed three times for 10 min with TBS Tween-20 (0.1% V/V). Samples were leaded randomly to avoid membrane variability. Visualization of the bands was performed using Odyssey CLX LI-COR Imaging System and band quantification was performed using Image J software (version 4.0).

### Sciatic nerve toluidine blue staining

After animal sacrifice, the right sciatic nerves of 3 mice / group were fixed for 20 minutes in situ with 4 % PFA and 2.5% glutaraldehyde in 0.1 M phosphate buffer (pH 7.3). Then, nerves were removed and post-fixed overnight in the same buffer. After washing for 30 minutes in 0.2 M PBS buffer, the samples were dehydrated using ethanol gradient solutions, and embedded in epoxy resin. Semithin cross sections were cut and stained with 0.5 % of toluidine blue + 1 % borax + 100 mL MilliQ water. The axonal diameter, number of myelinated axons and the myelin g-ratio were quantified using Image J g-ratio plugging.

### Summary of in vivo study phase:

### Day 1: From 8 AM to 11 AM

Progressive behavioral test learning (rotarod test learning) was carried out one day before to start the in vivo part of the study. In order to reduce animal stress and anxiety due to the new environment, animals remained for 2 hours in the behavioral test room.

### Day 2: From 8 AM to 11 AM - Rotarod:

Neuromuscular performances were analyzed using a rotarod test. The values from 3 trials were averaged for each animal, and then averaged for each animal group.

### Day 3: From 8 AM to 11 AM - Electrophysiology (EMG):

The animals were anesthetized using ketamine/xylazine mixture and sciatic nerve electrophysiology recording was performed. The compound muscle action potential (CMAP) was recorded from the intrinsic foot muscles using steel electrodes. Then, the nerve conduction velocities (NCVs) of each animal were calculated from sciatic nerve latency measurements.

### From Day 3 to day 31:At 7 AM and at 7 PM

Compound 163 was administrated by oral gavage twice a day to the members of one group of mice (compound treated group). Vehicle only was administrated by oral gavage twice a day to the members of another group of mice (vehicle control group).

### Day 29: From 8 AM to 11 AM

Progressive behavioral test learning (rotarod test learning) was carried out. In order to reduce animal stress and anxiety due to the new environment, animals remained for 2 hours in the behavioral test room.

### Day 30: From 8 AM to 11 AM - Rotarod:

Neuromuscular performances were analyzed using a rotarod test. The values from 3 trials were averaged for each animal, and then averaged for each animal group

### Day 31:

### From 8 AM to 11 AM - Electrophysiology (EMG):

The animals were anesthetized using ketamine/xylazine mixture and sciatic nerve electrophysiology recording was performed. The CMAP was recorded from the intrinsic foot muscles using steel electrodes. Then, the NCVs of each animal were calculated from sciatic nerve latency measurements as stated before.

### 12AM - Terminal procedure, sciatic nerve sampling :

After electrophysiology recording, mice were sacrificed by cervical dislocation. Left and right sciatic nerve were sampled for PMP22 western blot and histological analysis respectively.

### Results

### Mortality and body weight

No mortality was observed during the study.

No pathological clinical sign was observed during the study.

No significant differences on body weight were observed between groups suggesting the absence of a systemic toxic effect of Compound 163 at the analyzed time points.

**Rotarod test.** Similar rotarod latencies were observed in the vehicle and Compound 163 group at the baseline (1 month old). Neuromuscular impairment was observed at 2 months old in the vehicle treated group characterized by a significant decrease of the rotarod latency. In CMT1A mice at 2 months old, the Compound 163 treated group presented a significant increase of the rotarod latency compared to the vehicle group (a 133% increase in mean rotarod latency was observed in the Compound 163 treated group compared to the vehicle group). These data suggest a protective effect of Compound 163 on the neuromuscular impairment induced by the CMT1A disorder when administrated twice a day during one month at 30 mg/kg.

### Sciatic nerve electrophysiology.

### Compound Muscle Action Potential (CMAP) Amplitude

Similar CMAP amplitudes were observed in the vehicle and Compound 163 group at the baseline (1 month old). Sciatic nerve conduction impairment was observed at 2 months old in the vehicle treated group characterized by a significant decrease of the CMAP amplitude. In CMT1A mice at 2 months old, the Compound 163 treated group presented a significant increase of the CMAP amplitude compared to the vehicle group (an 86% increase in CMAP amplitude was observed in the Compound 163 treated group compared to the vehicle group). These data suggest a protective effect of Compound 163 compound on the CMAP impairment induced by the CMT1A disorder when administrated twice a day during one month at 30 mg/kg. Moreover, because the CMAP amplitude is directly linked to the axonal activity, these results suggest a direct or indirect efficacy of Compound 163 on the axon functionality.

### Nerve conduction velocity (NCV)

Concerning the nerve conduction velocities, similar nerve conduction velocities were observed in vehicle and Compound 163 groups at the baseline (1 month old). Sciatic nerve conduction impairment was observed at 2 months old in the vehicle treated group characterized by a significant decrease of the nerve conduction velocity. In CMT1A mice at 2 months old, an increase of the nerve conduction velocity was observed the Compound 163 treated group (a 94% increase in NCV was observed in the Compound 163 treated group compared to the vehicle group).

### PMP22 western blot analysis

Compound 163 treated group presented a significant decrease of the overexpression of PMP22 in the sciatic nerve of CMT1a mice at 2 months old compared to the vehicle group (a 33% decrease in the level of PMP22 was observed in the Compound 163 treated group compared to the vehicle group), confirming the positive efficacy of Compound 163 on the CMT1A disorder, and corroborating the behavioural test and electrophysiology data.

### Histology analysis of sciatic nerve

Sciatic nerve toluidine blue staining was performed to determine the efficacy of Compound 163 from a histological point of view. In CMT1A mice at 2 months old, a significant increase of the number of myelinating axons and decrease of g-ratio was observed after Compound 163 treatment compared to the vehicle group (a 64% increase in the number of myelinating axons and a 22% decrease in the g-ratio). In CMT1A mice at 2 months old, an increase in the axonal diameter was also observed in the Compound 163 treatment group compared to the vehicle group (a 45% increase). These data confirm the functional efficacy data observed using the behavioral test and electrophysiological analysis and suggest that Compound 163 targets axonal and myeling Schwann cell pathology induced by CMT disorder.

### Summary and conclusions

Neuromuscular impairment and decrease of nerve conduction amplitude and velocity were observed in the preclinical CMT1A mouse model treated with vehicle. The Compound 163 treated group presented a significant increase of the rotarod latency and CMAP amplitude, decrease of PMP22 overexpression and an increase in the number of axons and myelin diameter compared to the vehicle group. Also, an increase of the nerve conduction velocity and axonal diameter were also observed in the Compound 163 treated group but these increases were not statistically significant compared to the vehicle group.

Taken together, this study confirms that Compound 163 presents significant protective effect on CMT1A neuropathy targeting myelin sheath and axons. The compound increases neuromuscular and electrophysiological performances and decreases the molecular pathomechanism when administrated twice a day during 30 days at 30 mg/kg.

This model functions as an *in vivo* model of Charcot-Marie-Tooth 1A (CMT1A). Activity (positive effects) in this model strongly supports that compounds of this invention are efficacious in the treatment of Charcot-Marie-Tooth 1A (CMT1A and other autophagy-related neurodegenerative diseases or conditions.

### Example 7

### Demonstration of activity in a mouse model of Parkinson's disease (PD)

Compound 196 was selected as example compound of this invention to be evaluated in this model.

This model (run by Neuro-Sys SAS, France) uses C57BL/6JRj mice injured by intra-nigral injections of a solution containing protofibrils of alpha-synuclein (a-syn, precisely quantified by automatic Western Blot) and combined with pharmacological inhibition of GBA by conduritol B epoxide, in aged mice. The model reproduces essential neuropathological features of PD (e.g. loss of dopaminergic neurons, α -syn aggregation, endoplasmic reticulum [ER] stress) and the neuroinflammatory response (microglia activation and release of proinflammatory cytokines) in the nigra striata area. Any substances with positive effects in this model may be useful as a new therapeutic agent for the treatment or prevention of PD.

### Overview of the study

### Animal characteristics:

**Type of animal:** C57BL/6JRj (Male)
**Age:** 18 months old
**Supplier:** Janvier Labs
**Animal number:** 7 mice per group
**Acclimation and clinical signs:** Animals arrived on site 7 days (one week) before the experiment to allow optimal acclimation.

### Dosing outline:

**Test compound dose and administration:** 30 mg/kg, per os (gavage or drinking water), twice a day
**Test compound treatment:** The test compound was solubilized (vehicle = water), aliquoted and stored at -20°C. Aliquots were thawed overnight at 4 °C and given twice a day (morning and afternoon). Treatment started on the day of the stereotaxic injections of α-syn preparation (Day 0). Treatment was administrated until the last day of the experiment (Day 28; = day of dissection).
**CBE dose and administration:** 50 mg/kg, Intraperitoneal (i.p.), twice a day.
**CBE treatment:** Administration of CBE (vehicle = saline [NaCl 0.9%] started on the day of the stereotaxic injections of α-syn preparation (Day 0). Treatment was administrated until the last day of the experiment (Day 28; = day of dissection), **Untreated control:** vehicle (saline; i.p.) administered twice a day,

### Readouts:

i) Body mass.
ii) Dopaminergic Tyrosine hydroxylase (TH)-positive neuron survival in the substantia nigra pars compacta (SNpc), i.e. a measure of the number of TH neurons in the SNpc.
iii) Aggregation of α -syn in TH neurons, in the SNpc.
iv) Activation of Ionized calcium-binding adaptor protein-1 (Iba-1)-positive microglial cells in the SNpc.

### Methods

### Stereotaxic injections of α-syn oligomers

Human α-syn peptide (stock at 69 µM in water at -20°C) was reconstituted at 50µM in NaCl, 0.9% (final concentration). All mice in compound treated groups were subjected to surgery and received 2.5 µL of α-syn solution. Mice were anesthetized by isoflurane (4%, for induction), in an induction chamber coupled with a vaporizer and to an oxygen concentrator. Mice were placed on the stereotaxic frame. Anesthesia was maintained by isoflurane (2%) with a face mask coupled to the isoflurane vaporizer and oxygen concentrator machine. The skull was exposed and holes were drilled. The α-syn preparation was bilaterally injected into the SNpc, at the following coordinates: A-P, -0.3 mm; M-L, ±0.12 mm; D-V, -0.45 mm. Depth of anesthesia and rectal temperature were verified every 5 minutes. After surgery, mice were allowed to recover before being placed back in the cage.

### Endpoint evaluation

For overall evaluation and body mass, mice were observed daily, and body mass was monitored prior the morning drug administration.

### Plasma sampling, tissue collection and immunostaining.

At the end of the experiment (on day 28 post-surgery), mice (n=5/group) were deeply anesthetized, whole blood was collected on heparin tubes by heart puncture, centrifugated at 1500 g, 10 minutes, 4°C. Plasma was collected and snap frozen. Directly after heart puncture, mice were perfused with cold PBS (3 minutes), and cold paraformaldehyde (PFA) 4% in PBS (3 minutes). Immunostaining was performed with the n=5/group. Brains were dissected and further fixed in PFA 4%, overnight at 4°C. After, brains were placed in 30% sucrose in Tris-phosphate saline (TBS) solution at 4°C. Coronal sections, including the SNpc, of 40 µm-thickness were cut using a freezing microtome (4 sections per mouse, each 100 mm apart). For immunostaining, free-floating sections were incubated in TBS with 0.25% bovine serum albumin, 0.3% Triton X-100 and 1% goat serum, for 1 hour at room temperature. This incubation blocked unspecific binding sites and permeabilized the tissues. Four (n=4) brain sections per animal were processed and incubated for 24 hours at 4°C or 2 hours at room temperature with selected antibodies:
- TH: Chicken polyclonal antibody anti-tyrosine hydroxylase (1/1000).
- α-syn: Rabbit polyclonal antibody anti-alpha synuclein (1/200).
- IBA1: Goat polyclonal antibody anti-IBA1 (1/200)

These antibodies were revealed with Alexa Fluor 488 anti-rabbit IgG, Alexa Fluor 568 anti-chicken IgG, at the dilution 1/500, incubated in TBS with 0.25% donkey serum albumin, 0.3% Triton X-100 and 1% goat serum.

Images were acquired with a confocal laser-scanning microscopy. The following readouts were investigated:
- Number of TH positive cells in the SNpc.
- Activation of Iba1-positive microglial cells in the SNpc.
- Aggregation of α-syn in TH neurons, in the SNpc.

### Results

Compound 196 did not significantly modify body mass of the animals after 4 weeks of treatment, suggesting the absence of a systemic toxic effect of Compound 196 at the analyzed time points. A >60% increase in the number of dopaminergic TH positive neurons in the SNpc was observed in the compound treated group compared to the α-syn/CBE injured group. In addition, a >15% decrease in the number of Iba1-positive microglial cells in the SNpc was observed in the compound treated group compared to the the α-syn/CBE injured group. Taken together, this data indicates that Compound 196 was able to significantly protect dopaminergic TH positive neurons from neurodegeneration in this study.

This model functions as an in vivo model of Parkinson's disease. Activity in this model (positive effects) strongly support that compounds of this invention are efficacious in the treatment of Parkinson's disease and other autophagy-related neurodegenerative diseases or conditions.

## Claims

1. A compound according to Formula (I), 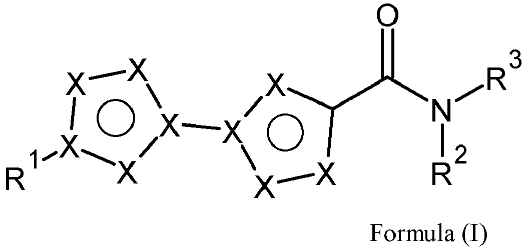 wherein
X is independently selected from chemically possible combinations of C, N, O, and S, and is optionally substituted with -CH₃, -CH₂-CH₃ or COOH,
R¹ is selected from
cyclopentyl, cyclohexyl, optionally including chemically possible N, O, and/or S, and optionally mono- or bi-substituted with -CH3, -NH₂, -COOH, C₁ to C₄ alkoxy, halo, trifluoromethyl, trifluoromethoxy;
R² is selected from
H, CH₃, straight or branched C₂ to C₆ alkyl, optionally including chemically possible N, O, and/or S, and optionally substituted with -CH₃, -NH₂, -OH, -COOH, cyclopentyl, cyclohexyl, bicyclo[2.2.1]heptane, bicyclo[3.1.1]heptane, bicyclo[2.2.2]octane, optionally including chemically possible N, O, and/or S, and optionally substituted with -CH₃, -CH₂-CH₃, -OH, -isopropyl, -COOH, -COOCH₃, -CH₂-cyclohexyl, -NH₂,
R³ is selected from
H, straight or branched C₁ to C₆ alkyl, optionally substituted with -CH₃, -OH, - CH₂-CH₃, -NH₂, -CH₂-NH₂, -CH₂-NH-CH₃, -COOH, optionally including chemically possible one or more N, O, and/or S;
C₁ to C₄ alkoxy, optionally including chemically possible one or more N, O, and/or S, and optionally mono- or bi substituted with, -NH₂, -OH, -COOH;
cyclopropyl, cyclobutyl, optionally including chemically possible one or more N, O, and/or S, and optionally mono- or bi substituted with -CH₃, -CH₂-CH₃, -NH₂, - CH₂-NH₂, -CH₂-NH-CH₃, -OH, -COOH;
with the provisio that R² and R³ are not both H or CH₃;
R² and R³ may be connected to form a cyclobutyl, cyclopentyl, cyclohexyl, cycloheptyl ring, a bicyclic ring structure of fused cyclopentyl and/or cyclohexyl, bicyclo[3.1.1]heptane, optionally including chemically possible one or more N, O, and/or S, and optionally being mono- or bi-substituted with straight or branched C₁ to C₆ alkyl, optionally substituted with -CH₃, -OH, -CH₂-CH₃, -NH₂, -NH-CH₃, - CH₂-NH₂, -CH₂-NH-CH₃, -OH, -COOH, -COOCH₃, =O, isopropyl, sulfonamide, and optionally including chemically possible one or more N, O, and/or S;
a physiologically acceptable salt, a solvate, a hydrate, an enantiomer or a polymorph thereof.

2. A compound according to claim 1 according to the following formula II, 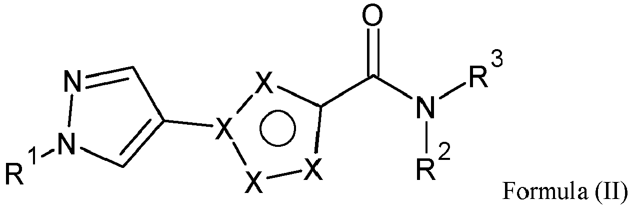 wherein
X, R¹, R² and R³ are as above,
a physiologically acceptable salt, a solvate, a hydrate, an enantiomer or a polymorph thereof.

3. A compound according to claim 1 or 2 according to the following formula III, 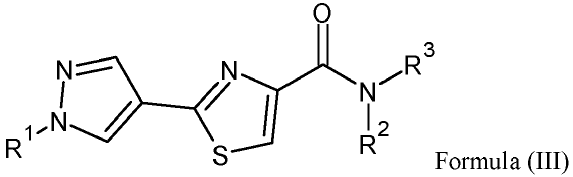 wherein
R¹, R² and R³ are as above,
a physiologically acceptable salt, a solvate, a hydrate, an enantiomer or a polymorph thereof.

4. A compound according to any one of claims 1 to 3, wherein
R¹ is selected from 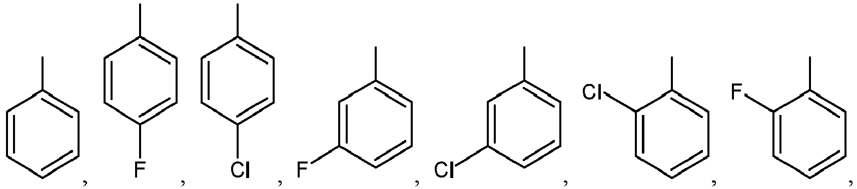 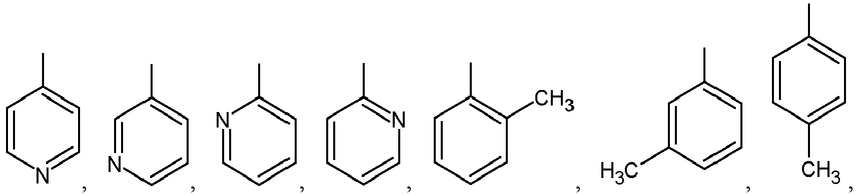 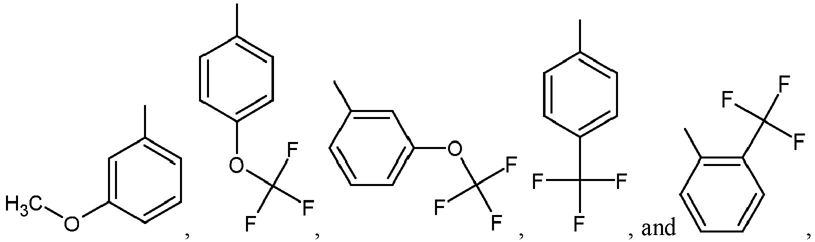 a physiologically acceptable salt, a solvate, a hydrate, an enantiomer or a polymorph thereof.

5. A compound according to any one of claims 1 to 4, wherein
R² is selected from H, -CH₃, cyclohexyl, 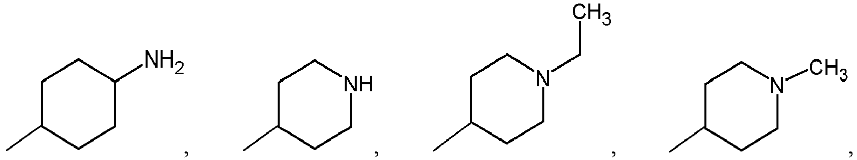 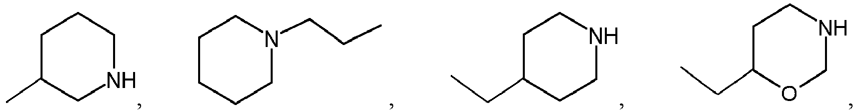 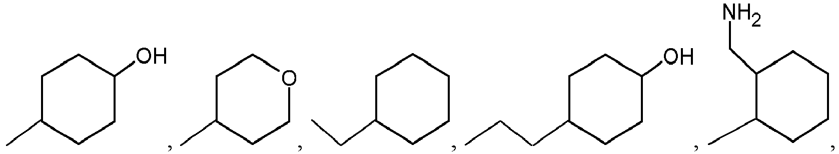 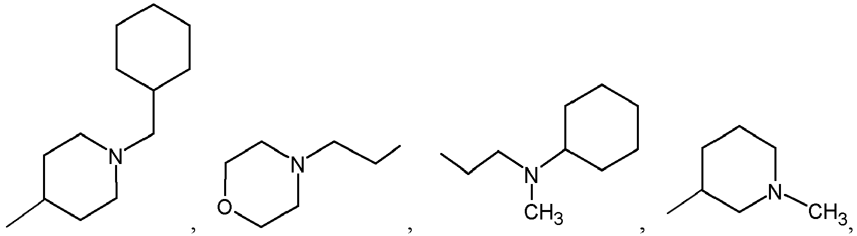 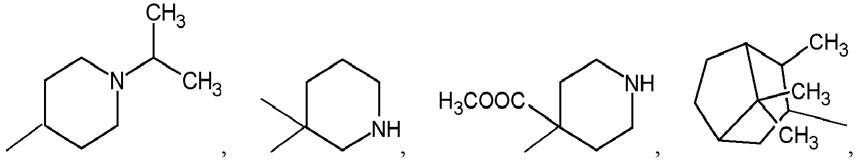 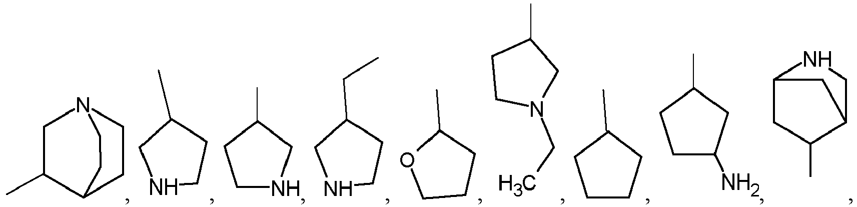 and 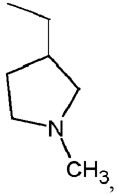
R³ is selected from H, -CH₃, -CH₂-CH₃, propyl, isopropyl, -CH₂-CH₂-OH, -CH₂-CH₂-NH₂, -CH₂-CH₂-CH₂-NH₂, -CH₂-CH₂-CH₂-CH₂-CH₂-NH₂, -CH₂-CH₂-O-CH₂-CH₂-NH₂, -CH₂-CH(CH₃)₂, -CH₂-CH(NH₂)-CH₃, -CH(-CH₃)-CH₂-NH₂, -CH₂-CH₂-N(CH₃)₂, 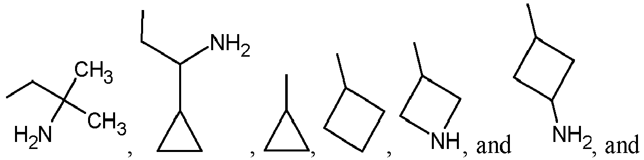
R² and R³ may be connected to form a ring selected from 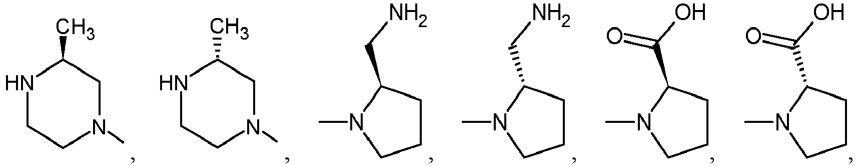 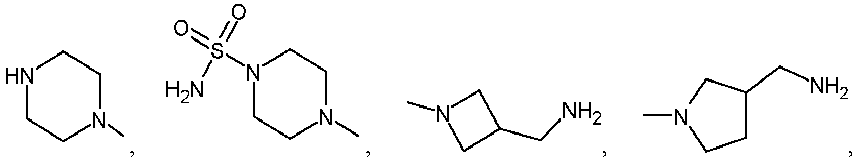 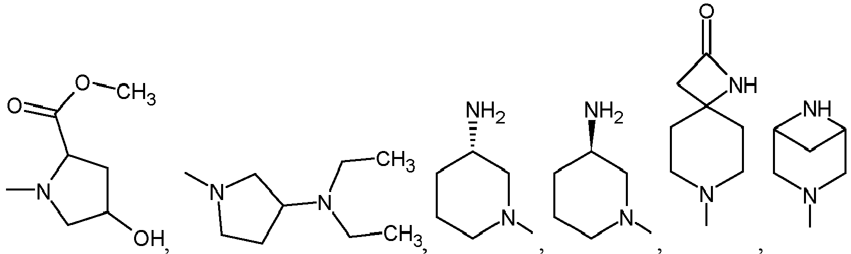 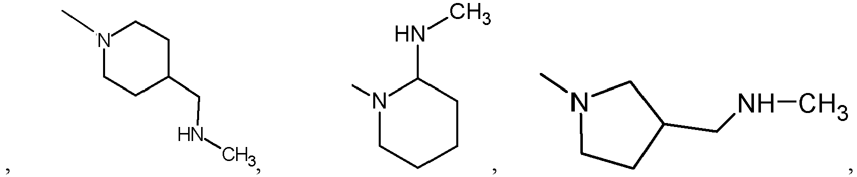 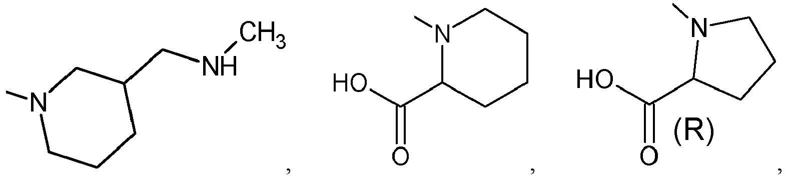 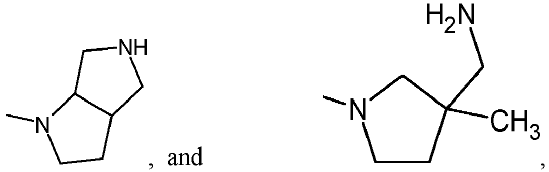 a physiologically acceptable salt, a solvate, a hydrate, an enantiomer or a polymorph thereof.

6. A compound according to any one of claims 1 to 3 according to the following formula IV, 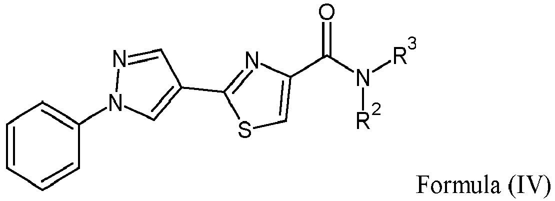
wherein R² is selected from 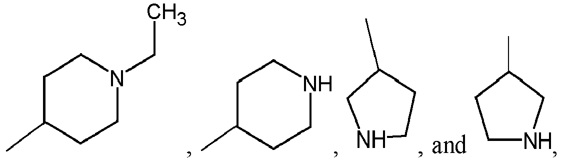
R³ is selected from H, CH₃, -CH₂-CH₂-CH₃, and isopropyl, or
R² and R³ form a ring selected from 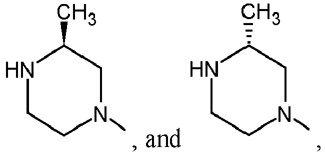
a physiologically acceptable salt, a solvate, a hydrate, an enantiomer or a polymorph thereof.

7. A compound selected from the following:
N-(piperidin-4-yl)-N- (propan-2-yl)-2-{1-[2- (trifluoromethyl)phenyl]- 1H-pyrazol-4-yl}-1,3- thiazole-4-carboxamide (Formula 47);
N-propyl-N-[(3S)-pyrrolidin-3-yl]-2-{1-[3- (trifluoromethyl)phenyl]- 1H-pyrazol-4-yl}-1,3- thiazole-4-carboxamide (Formula 48);
1-[(2R)-1-{2-[1-(3-methoxyphenyl)-1H- pyrazol-4-yl]-1,3-thiazole-4 carbonyl}pyrrolidin-2- yl]methanamine (Formula 50);
(2S)-1-[2-(1-phenyl-1H-pyrazol-4-yl)-1,3-thiazole-4 carbonyl]pyrrolidine-2-carboxylic acid (Formula 60);
2-[1-(2-fluorophenyl)-1H- pyrazol-4-yl] -N-propyl-N- [(3 S)-pyrrolidin-3 -yl] -1,3 - thiazole-4-carboxamide (Formula 92);
5-(1-phenyl-1H-pyrazol-4-yl)-N-propyl-N-[(3S)-pyrrolidin-3-yl]-1H-pyrrole- 2-carboxamide (Formula 93);
5-(1-phenyl-1H-pyrazol-4-yl)-N-propyl-N-[(3R)-pyrrolidin-3-yl]-1H-pyrrole- 2-carboxamide (Formula 94);
5-(1-phenyl-1H-pyrazol-4- yl)-N-propyl-N-[(3 S)- pyrrolidin-3-yl]furan-2-carboxamide (Formula 98);
5-(1-phenyl-1H-pyrazol-4- yl)-N-propyl-N-[(3R)- pyrrolidin-3-yl]furan-2-carboxamide (Formula 99);
4-[2-(1-phenyl-1H-pyrazol-4-yl)-1,3-thiazole-4- carbonyl]piperazine-1-sulfonamide (Formula 105);
N-[2-(2-aminoethoxy)ethyl]-4-(1- phenyl-1H-pyrazol-4- yl)thiophene-2-carboxamide (Formula 110);
2-(2-phenyl-1,3-oxazol-5- yl)-N-propyl-N-(pyrrolidin- 3-yl)-1,3-thiazole-4-carboxamide (Formula 116);
5-(1-phenyl-1H-pyrazol-4- yl)-N-propyl-N-(pyrrolidin- 3-yl)-1H-pyrrole-2-carboxamide (Formula 120);
2-(5 -phenylthiophen-2-yl)-N-propyl-N-(pyrrolidin-3 -yl)-1, 3 -thiazole-4-carboxamide (Formula 122);
4-(1-phenyl-1H-pyrazol-4-yl)-N-propyl-N-(pyrrolidin-3-yl)-1H-pyrrole-2-carboxamide (Formula 123);
1-[2-(1-phenyl-1H-pyrazol-4-yl)-1,3-thiazole-4- carbonyl]azetidin-3-amine (Formula 124);
4-(1-phenyl-1H-pyrazol-4- yl)-N-propyl-N-(pyrrolidin- 3-yl)thiophene-2-carboxamide (Formula 141);
1-[2-(1-phenyl-1H-pyrazol-4-yl)-1,3-thiazole-4-carbonyl]pyrrolidine-2- carboxylic acid (Formula 144);
1-[(2R)-1-[2-(1-phenyl-1H-pyrazol-4-yl)-1,3-thiazole-4 carbonyl]pyrrolidin-2-yl]methanamine (Formula 156);
(3S)-3-methyl-1-[2-(1-phenyl-1H-pyrazol-4-yl)- 1,3-thiazole-4- carbonyl]piperazine (Formula 157);
2-(1-phenyl-1H-pyrazol-4- yl)-N-(piperidin-4-yl)-N-(propan-2-yl)-1,3-thiazole- 4-carboxamide (Formula 163);
2-(1-phenyl-1H-pyrazol-4-yl)-N-propyl-N-[(3S)-pyrrolidin-3-yl]-1,3 - thiazole-4-carboxamide (Formula 170);
2-(1-phenyl-1H-pyrazol-4- yl)-N-propyl-N-[(3R)-pyrrolidin-3-yl]-1,3- thiazole-4-carboxamide (Formula 171);
N-(1-ethylpiperidin-4-yl)-2-(1-phenyl-1H-pyrazol-4-yl)-1,3-thiazole-4-carboxamide (Formula 185);
N-(1-ethylpiperidin-4-yl)-N-methyl-2-(1-phenyl-1H-pyrazol-4-yl)-1,3 -thiazole-4 carboxamide (Formula 196); and
a physiologically acceptable salt, a solvate, a hydrate, an enantiomer or a polymorph thereof.

8. A compound selected from the following:
5-(1-phenyl-1H-pyrazol-4-yl)-N-propyl-N-[(3S)-pyrrolidin-3-yl]-1H-pyrrole- 2-carboxamide (Formula 93);
5-(1-phenyl-1H-pyrazol-4-yl)-N-propyl-N-[(3R)-pyrrolidin-3-yl]-1H-pyrrole- 2-carboxamide (Formula 94);
2-(5 -phenylthiophen-2-yl)-N-propyl-N-(pyrrolidin-3 -yl)-1, 3 -thiazole-4-carboxamide (Formula 122);
4-(1-phenyl-1H-pyrazol-4-yl)-N-propyl-N-(pyrrolidin-3-yl)-1H-pyrrole-2-carboxamide (Formula 123);
(3S)-3-methyl-1-[2-(1-phenyl-1H-pyrazol-4-yl)-1,3-thiazole-4- carbonyl]piperazine (Formula 157);
2-(1-phenyl-1H-pyrazol-4- yl)-N-(piperidin-4-yl)-N-(propan-2-yl)-1,3-thiazole- 4-carboxamide (Formula 163);
2-(1-phenyl-1H-pyrazol-4-yl)-N-propyl-N-[(3S)-pyrrolidin-3-yl]-1,3 - thiazole-4-carboxamide (Formula 170);
2-(1-phenyl-1H-pyrazol-4- yl)-N-propyl-N-[(3R)-pyrrolidin-3-yl]-1,3- thiazole-4-carboxamide (Formula 171);
N-(1-ethylpiperidin-4-yl)-2-(1-phenyl-1H-pyrazol-4-yl)-1,3-thiazole-4-carboxamide (Formula 185); and
N-(1-ethylpiperidin-4-yl)-N-methyl-2-(1-phenyl-1H-pyrazol-4-yl)-1,3 -thiazole-4 carboxamide (Formula 196);
a physiologically acceptable salt, a solvate, a hydrate, an enantiomer or a polymorph thereof.

9. A method for producing a compound according to any one of claims 1 to 8, comprising steps according to any one of general experimental procedures 1 to 4 as disclosed above.

10. A pharmaceutical composition, comprising a pharmaceutically effective amount of the compound according to any one of claims 1 to 8, and a pharmaceutically acceptable carrier.

11. The compound according to any one of claims 1 to 8 or the pharmaceutical composition according to claim 9 for use in the prevention and/or treatment of diseases in a mammalian subject, such as a human.

12. The compound according to any one of claims 1 to 8 or the pharmaceutical composition according to claim 9 for use as an autophagy inducer, wherein preferably said use is cosmetic and/or in vitro.

13. The compound according to any one of claims 1 to 8 or the pharmaceutical composition according to claim 9 for use in the prevention and/or treatment of an autophagy-related disease or condition in a mammalian subject, such as a human.

14. The compound for use according to claim 13, wherein said autophagy-related disease or condition is selected from the group consisting of neurodegenerative diseases, Huntington's disease, Alzheimer's disease, Parkinson's disease, systemic lupus erythematosus, epilepsy, cancer, liver diseases, a1 antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKU1, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, stroke, coronary artery diseases, myocardial infarction, unstable angina pectoris, atherosclerosis or vasculitis, Behcet's syndrome, giant cell arteritis, polymyalgia rheumatica, Wegener's granulomatosis, Churg-Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, ALS, antigen induced recall response, immune response suppression, muscle degeneration and atrophy, frailty in aging, spinal cord injury, and diseases and conditions involving misfolded and/or nonfolded proteins.

15. The compound for use according to claim 13 or 14, wherein said prevention and/or treatment comprises a combination of at least two compounds for use according to claim 13 or 14, and/or a combination with at least one additional pharmaceutically active substance for said autophagy-related disease or condition.

16. The compound for use according to any one of claims 13 to 15, wherein said prevention and/or treatment further comprises detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.

17. A method for preventing and/or treating an autophagy-related disease or condition in a mammalian subject, such as a human, comprising administering to said mammal an effective amount of at least one compound according to any one of claims 1 to 8 or a pharmaceutical composition according to claim 9.

18. The method according to claim 17, wherein said autophagy-related disease or condition is selected from the group consisting of neurodegenerative diseases, Huntington's disease, Alzheimer's disease, Parkinson's disease, systemic lupus erythematosus, epilepsy, cancer, liver diseases, a1 antitrypsin deficiency, Charcot Marie Tooth syndrome, Rett Syndrome, Sickle Cell disease, Wilson Disease, amyloidosis, Gaucher's diseases, lysosomal and glycogen storage disorders, cystic fibrosis; viral infection and diseases, human cytomegalovirus (HCMV) infection, hepatitis B, human immunodeficiency virus infection, Zika virus infection, coronavirus infection, HCoV-229E, HCoV-NL63, betacoronavirus infection, such as HCoV-OC43, SARS-CoV-1, HCoV-HKU1, MERS-CoV or SARS-CoV-2, bacterial infections, metabolic disorders, diabetes, fibrosis, wound healing disorders, Niemann-Pick type C (NPC) disease, fibrinogen storage disease (FSB), inclusion body disease (IBD), muscular dystrophy, Duchenne muscular dystrophy, Limb-girdle muscular dystrophy, myopathy, myofibrillar myopathy, hereditary myopathy, diabetic cardiomyopathy, anti-inflammatory disorders, autoimmune diseases, multiple sclerosis, rheumatoid arthritis, irritable bowel syndrome, Crohn's disease, vascular disorders, stroke, coronary artery diseases, myocardial infarction, unstable angina pectoris, atherosclerosis or vasculitis, Behcet's syndrome, giant cell arteritis, polymyalgia rheumatica, Wegener's granulomatosis, Churg-Strauss syndrome, vasculitis, Henoch-Schonlein purpura, Kawasaki disease, viral infection or replication, pox virus infection, herpes virus infection, asthma, allergic rhinitis, COPD, osteoporosis, organ transplant rejection, psoriasis, hypertrophic scarring (keloid formation), adhesion formations following general or gynecological surgery, lung fibrosis, liver fibrosis, kidney fibrosis, disorders caused by intracellular parasites, malaria, tuberculosis, neuropathic pain, post-operative phantom limb pain or postherpetic neuralgia, allergies, ALS, antigen induced recall response, immune response suppression, muscle degeneration and atrophy, frailty in aging, spinal cord injury, and diseases and conditions involving misfolded and/or nonfolded proteins.

19. The method according to claim 17 or 18, further comprising detecting and/or monitoring in said subject a response of at least one autophagy-biomarker, preferably selected from the group of BECN1, and the ATG8/LC3 family, including LC3A, LC3B, LC3C), LC3-II, ULK1, p62, NBR1, ATG5 and ATG7.


SEQUENCE LISTING

<110>  Samsara Therapeutics Inc.
       Institut National de la Santé et de la Recherche Médicale(INSERM)
       Université de Paris
       Sorbonne Université

<120>  Autophagy inducing compounds and uses thereof

<130>  S34315EP

<160>  2

<170>  PatentIn version 3.5

<210>  1
<211>  21
<212>  DNA
<213>  Homo sapiens

<400>  1
catcagccct aatccatctg a                                                21


<210>  2
<211>  21
<212>  DNA
<213>  Homo sapiens

<400>  2
cgcgactaac aatcaaagtg a                                                21


25